# EUROPEAN PATENT SPECIFICATION

(11) **EP 2 970 333 B1**
(45) Date of publication and mention of the grant of the patent: **03.05.2017**
(21) Application number: 14709962.6
(22) Date of filing: 14.03.2014
(51) Int. Cl.: C07D 498/18, A61K 31/437, A61K 31/504, A61P 25/16

(54) **MACROCYCLIC LRRK2 KINASE INHIBITORS**
MAKROCYCLISCHE LRRK2-KINASEHEMMER
INHIBITEURS MACROCYCLIQUES DE LA KINASE LRRK2

(30) Priority: 15.03.2013 EP 13305311; 15.03.2013 EP 13305314
(43) Date of publication of application: 20.01.2016
(73) Proprietor: IPSEN PHARMA S.A.S., 92100 Boulogne Billancourt (FR); Oncodesign S.A., 21000 Dijon Cedex (FR)
(72) Inventor: HOFLACK, Jan, 2390 Malle (BE); BLOM, Petra, 9070 Destelbergen (BE); LAVERGNE, Olivier, 91120 Palaiseau (FR)
(74) Representative: Bounaga, Sakina
(86) International application number: PCT/EP2014/055049
(87) International publication number: WO 2014/140235

(56) References cited:
- WO-A1-2011/141756
- WO-A1-2012/038743
- WO-A1-2012/143143
- WO-A1-2013/001310
- WO-A1-2013/045653
- WO-A1-2013/046029

## Description

### Field of the invention

The present invention relates to novel macrocyclic compounds and compositions containing said compounds acting as kinase inhibitors, in particular as inhibitors of LRRK2 kinase (Leucine-Rich Repeat Kinase 2), for use in the diagnosis, prevention and/or treatment of LRRK2-kinase associated diseases. Moreover, disclosed are methods of using them, for instance as a medicine or diagnostic agent, in particular for the prevention, treatment and/or diagnosis of diseases characterized by LRRK2 kinase activity such as neurological disorders including Parkinson's disease and Alzheimer's disease. Finally, the present invention also relates to new macrocyclic compounds.

### Background of the invention

Parkinson's disease is a degenerative disorder of the central nervous system. It results from the death of dopaminergic neurones in the midbrain. In the early stages of the disease the most obvious symptoms are movement-related such as shaking, slowness of movement and difficulty with walking. Later on also cognitive and behavioural problems arise, with dementia commonly occurring in the advanced stages of the disease. Although Parkinson's disease is generally considered to be sporadic, within the last decade, a few mutations in the LRRK2 (leucine rich repeat kinase 2) gene have been linked to Parkinson's disease (WO2006068492 and WO2006045392). LRRK2, also known as dardarin, is a member of the leucine-rich repeat kinase family having mixed-lineage kinase activity, in particular in the brain, but also in other tissues throughout the body. Researchers have identified over 20 *LRRK2* mutations in families with late-onset Parkinson Disease. For example the G2019S mutation co-segregates with autosomal dominant Parkinsonism and accounts for about 6% of familial Parkinson's disease cases and 3% sporadic Parkinson's disease cases in Europe. The G2019S mutation occurs in the highly conserved kinase domain and it has therefore been postulated that the G2019S mutation may have an effect on kinase activity (WO2006068492). Furthermore, amino acid substitutions at a second residue R1441 are also associated with Parkinson's disease and have also been shown to elevate LRRK2 kinase activity. Over-expression of the mutant LRRK2 protein R1441G in transgenic mouse models (Li, Y et al. 2009, Nature Neuroscience 12:826-828) is associated with symptoms of Parkinson's disease as well as reduced dopamine release, suggesting that inhibitors of LRRK2 could also positively regulate dopamine release and have potential utility in treatment of conditions characterized by reduced dopamine levels, such as withdrawal sypmtoms/relapse associated with drug addiction; Tauopathy diseases such as Alzheimer's disease, argyrophilic grain disease, Pick's disease, corticobasal degeneration; inherited frontotemporal dementia; and Parkinson's disease. Two further mutations in LRRK2 have been clinically associated with the transition from mild cognitive impairment to Alzheimer's disease (WO200714979). These data further provide evidence that inhibitors of LRRK2 kinase activity could be useful for the treatment of dementias and related neurodegenerative disorders. Thus, pharmacological inhibition of LRRK2 kinase is an attractive strategy towards mechanism-based therapies in neurodegenerative disorders, such as Parkinson's disease and Alzheimer's disease. It was therefore an object of the present invention to provide compounds and compositions comprising said compounds, acting as inhibitors of LRRK2 kinases.

Untill today several (non-macrocyclic) pyrazolopyrimidines have been suggested for the treatment of neuronal disorders, in particular Alzheimer's disease and/or Parkinson's disease (see for example EP1908764, US6194410, EP1354884, EP0729758 and US6194410). However, none of the compounds disclosed in said references have been shown to have LRRK2 inhibitory activity.

Furthermore, the currently developed LRRK2 kinase inhibitors, in particular those for the treatment of neuronal disorders, do not comprise macrocyclic pyrazolopyrimidine moieties (see for example WO2009127652, WO2011038572).

Nonetheless, there is a continuing need to design and develop LRRK2 kinase inhibitors for the treatment of neuronal disorders. We have now found that the macrocyclic pyrazolopyrimidines, imidazopyridazines and pharmaceutically acceptable compositions according to this invention are useful for the treatment of several neuronal disorders associated with LRRK2 kinase activity.

### SUMMARY OF THE INVENTION

We have surprisingly found that the macrocyclic compounds described herein act as LRRK2 kinase inhibitors, and are thus very useful in the diagnosis, prevention and/or treatment of LRRK2-kinase associated diseases.

In a first objective the present invention provides a compound of Formula I or a stereoisomer, tautomer, racemic, salt, hydrate, N-oxide form, or solvate thereof, 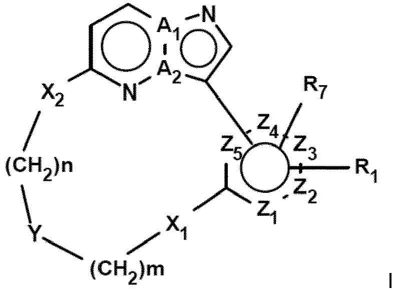 Wherein
A₁ and A₂ are selected from C and N; wherein when A₁ is C, then A₂ is N; and wherein when A₂ is C, then A₁ is N;
R₁ and R₇ are each independently selected from -H, -halo, -OH, -C₁₋₆alkyl, -O-C₁₋₆alkyl, -S-C₁₋₆alkyl, -NR₉R₁₀, -(C=O)-R₄, -SO₂-R₄, -CN, -NR₉-SO₂-R₄, -C₃₋₆cycloalkyl, and -Het₆; wherein each of said -C₁₋₆alkyl is optionally and independently substituted with from 1 to 3 substituents selected from -halo, -OH, -NR₁₁R₁₂, -O-C₁₋₆alkyl, and -S-C₁₋₆alkyl;
R₂ is selected from -H, -halo, -OH, -C₁₋₆alkyl, -O-C₁₋₆alkyl, -S-C₁₋₆alkyl, -(C=O)-C₁₋₆alkyl, -(C=O)-O-C₁₋₆alkyl, -(C=O)-NR₂₇R₂₈, -Het₃, -(C=O)-Het₃, -SO₂-C₁₋₆alkyl, and -C₃₋₆cycloalkyl; wherein each of said -C₁₋₆alkyl is optionally and independently substituted with from 1 to 3 substituents selected from -halo, -OH, -O-C₁₋₆alkyl, -S-C₁₋₆alkyl, -Het₃, -Ar₂, and -NR₁₃R₁₄;
R₃ is selected from -H, -halo, -OH, -C₁₋₆alkyl, -O-C₁₋₆alkyl, -S-C₁₋₆alkyl, -(C=O)-C₁₋₆alkyl, -(C=O)-O-C₁₋₆alkyl, -Het₂, -C₃₋₆cycloalkyl -(C=O)-Het₂, -(C=O)-NR₂₉R₃₀, and -SO₂-C₁₋₆alkyl; wherein each of said -C₁₋₆alkyl is optionally and independently substituted with from 1 to 3 substituents selected from -halo, -OH, -O-C₁₋₆alkyl, -S-C₁₋₆alkyl, -NR₁₅R₁₆, -Het₂, and -Ar₃;
R₄ is independently selected from -halo, -OH, -C₁₋₆alkyl, -O-C₁₋₆alkyl, -S-C₁₋₆alkyl, -NR₁₇R₁₈, and -Het₄;
R₅ is selected from -H, -C₁₋₆alkyl, -C₃₋₆cycloalkyl; wherein each of said C₁₋₆alkyl or -C₃₋₆cycloalkyl is optionally and independently substituted with from 1 to 3 substituents selected from -halo, -OH, -OC₁₋₆alkyl, -SC₁₋₆alkyl, -Het₅, and -NR₃₁R₃₂;
R₆ is selected from -H, -OH, -halo, -C₁₋₆alkyl, -O-C₁₋₆alkyl, -S-C₁₋₆alkyl, -NR₃₃R₃₄, and -Het₈;
R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉,R₃₀, R₃₁, R₃₂, R₃₃, R₃₄, R₃₇ and R₃₈ are each independently selected from -H, =O, -C₁₋₆alkyl, and -Het₁; wherein each of said -C₁₋₆alkyl is optionally and independently substituted with from 1 to 3 substituents selected from -halo, -OH, -O-C₁₋₆alkyl, -S-C₁₋₆alkyl, -NR₃₅R₃₆, -Het₇, and -Ar₄;
R₃₅ and R₃₆ are each independently selected from -H, =O and -C₁₋₆alkyl; wherein each of said-C₁₋₆alkyl is optionally and independently substituted with from 1 to 3 substituents selected from -halo, -OH, -O-C₁₋₆alkyl, and -S-C₁₋₆alkyl;
X₁ is selected from -C₁₋₆alkyl-, -O-C₁₋₆alkyl-, -S-C₁₋₆alkyl-, -(C=O)-, -NR₃-(C=O)-, -C₁₋₆alkyl-NR₃-, -NR₃-, -(C=O)-, -NR₃-(C=O)-NR₃₇-, -NR₃-C₁₋₆alkyl-, -NR₃-SO₂-, -NR₃(C=O)-C₁₋₆alkyl-, -(C=O)-NR₃-C₁₋₆alkyl-, -O-C₁₋₆alkyl-O-C₁₋₆alkyl- and -C₁₋₆alkyl-NR₃-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl, and -NR₂₃R₂₄
X₂ is selected from -C₁₋₆alkyl-, -O-C₁₋₆alkyl-, -S-C₁₋₆alkyl-, -(C=O)-, -NR₂-(C=O)-, -C₁₋₆alkyl-NR₂-, -NR₂-, -(C=O)-, -NR₂-(C=O)-NR₃₈-, -NR₂-C₁₋₆alkyl-, -NR₂-SO₂-, -NR₂(C=O)-C₁₋₆alkyl-, -(C=O)-NR₂-C₁₋₆alkyl-, -O-C₁₋₆alkyl-O-C₁₋₆alkyl- and -C₁₋₆alkyl-NR₂-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl and-NR₂₅R₂₆;
Y is selected from a direct bond, -CHR₆-, -O-, -S-, and -NR₅-;
Ar₂, Ar₃, and Ar₄ are each independently a 5- or 6-membered aromatic heterocycle optionally comprising 1 or 2 heteroatoms selected from O, N and S; wherein each of said Ar₂, Ar₃, and Ar₄ is optionally and independently substituted with from 1 to 3 substituents selected from -NR₁₉R₂₀, -C₁₋₆alkyl, -O-C₁₋₆alkyl, and -S-C₁₋₆alkyl;
Het₁, Het₂, Het₃, Het₄, Het₅, Het₆, Het₇ and Het₈ are each independently a 5-or 6-membered monocyclic heterocycle having from 1 to 3 heteroatoms selected from O, N and S, wherein each of said Het₁, Het₂, Het₃, Het₄, Het₅, Het₆, Het₇ and Het₈ is optionally substituted with from 1 to 3 substituents selected from -C₁₋₆alkyl, -O-C₁₋₆alkyl, -S-C₁₋₆alkyl, and -NR₂₁R₂₂; wherein each of said -C₁₋₆alkyl is optionally and independently substituted with from 1 to 3-halo;
Z₁, Z₂, Z₃, Z₄ and Z₅ are each independently selected from C and N;
m and n are each independently 1, 2, 3, or 4.
for use in the diagnosis, prevention and/or treatment of a LRRK2-kinase associated disease.

In a first embodiment, the present invention provides a compound of Formula I or a stereoisomer, tautomer, racemic, salt, hydrate, N-oxide form, or solvate thereof, wherein
A₁ is C and A₂ is N
R₁ and R₇ are each independently selected from -H, -halo, -OH, -C₁₋₆alkyl, -O-C₁₋₆alkyl, -S-C₁₋₆alkyl, -NR₉R₁₀, -(C=O)-R₄, -SO₂-R₄, -CN, -NR₉-SO₂-R₄, -C₃₋₆cycloalkyl, and -Het₆; wherein each of said -C₁₋₆alkyl is optionally and independently substituted with from 1 to 3 substituents selected from -halo, -OH, -NR₁₁R₁₂, -O-C₁₋₆alkyl, and -S-C₁₋₆alkyl;
R₂ is selected from -H, -halo, -OH, -C₁₋₆alkyl, -O-C₁₋₆alkyl, -S-C₁₋₆alkyl, -(C=O)-C₁₋₆alkyl, -(C=O)-O-C₁₋₆alkyl, -(C=O)-NR₂₇R₂₈, -Het₃, -(C=O)-Het₃, -SO₂-C₁₋₆alkyl, and -C₃₋₆cycloalkyl; wherein each of said -C₁₋₆alkyl is optionally and independently substituted with from 1 to 3 substituents selected from -halo, -OH, -O-C₁₋₆alkyl, -S-C₁₋₆alkyl, -Het₃, -Ar₂, and -NR₁₃R₁₄;
R₃ is selected from -H, -halo, -OH, -C₁₋₆alkyl, -O-C₁₋₆alkyl, -S-C₁₋₆alkyl, -(C=O)-C₁₋₆alkyl, -(C=O)-O-C₁₋₆alkyl, -Het₂, -C₃₋₆cycloalkyl -(C=O)-Het₂, -(C=O)-NR₂₉R₃₀, and -SO₂-C₁₋₆alkyl; wherein each of said -C₁₋₆alkyl is optionally and independently substituted with from 1 to 3 substituents selected from -halo, -OH, -O-C₁₋₆alkyl, -S-C₁₋₆alkyl, -NR₁₅R₁₆, -Het₂, and -Ar₃;
R₄ is independently selected from -halo, -OH, -C₁₋₆alkyl, -O-C₁₋₆alkyl, -S-C₁₋₆alkyl, -NR₁₇R₁₈, and -Het₄;
R₅ is selected from -H, -C₁₋₆alkyl, -C₃₋₆cycloalkyl; wherein each of said C₁₋₆alkyl or -C₃₋₆cycloalkyl is optionally and independently substituted with from 1 to 3 substituents selected from -halo, -OH, -OC₁₋₆alkyl, -SC₁₋₆alkyl, -Het₅, and -NR₃₁R₃₂;
R₆ is selected from -H, -OH, -halo, -C₁₋₆alkyl, -O-C₁₋₆alkyl, -S-C₁₋₆alkyl, -NR₃₃R₃₄, and -Het₈;
R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉,R₃₀, R₃₁, R₃₂, R₃₃, R₃₄, R₃₇ and R₃₈ are each independently selected from -H, =O, -C₁₋₆alkyl, and -Het₁; wherein each of said -C₁₋₆alkyl is optionally and independently substituted with from 1 to 3 substituents selected from -halo, -OH, -O-C₁₋₆alkyl, -S-C₁₋₆alkyl, -NR₃₅R₃₆,-Het₇, and -Ar₄;
R₃₅ and R₃₆ are each independently selected from -H, =O, and -C₁₋₆alkyl; wherein each of said-C₁₋₆alkyl is optionally and independently substituted with from 1 to 3 substituents selected from -halo, -OH, -O-C₁₋₆alkyl, and -S-C₁₋₆alkyl;
X₁ is selected from -C₁₋₆alkyl-, -O-C₁₋₆alkyl-, -S-C₁₋₆alkyl-, -(C=O)-, -NR₃-(C=O)-, -C₁₋₆alkyl-NR₃-, -NR₃-, -(C=O)-, -NR₃-(C=O)-NR₃₇-, -NR₃-C₁₋₆alkyl-, -NR₃-SO₂-, -NR₃-(C=O)-C₁₋₆alkyl-, -(C=O)-NR₃-C₁₋₆alkyl-, -O-C₁₋₆alkyl-O-C₁₋₆alkyl- and -C₁₋₆alkyl-NR₃-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl, and -NR₂₃R₂₄
X₂ is selected from -C₁₋₆alkyl-, -O-C₁₋₆alkyl-, -S-C₁₋₆alkyl-, -(C=O)-, -NR₂-(C=O)-, -C₁₋₆alkyl-NR₂-, -NR₂-, -(C=O)-, -NR₂-(C=O)-NR₃₈-, -NR₂-C₁₋₆alkyl-, -NR₂-SO₂-, -NR₂-(C=O)-C₁₋₆alkyl-, -(C=O)-NR₂-C₁₋₆alkyl-, -O-C₁₋₆alkyl-O-C₁₋₆alkyl- and -C₁₋₆alkyl-NR₂-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl and-NR₂₅R₂₆;
Y is selected from a direct bond, -CHR₆-, -O-, -S-, and -NR₅-;
Ar₂, Ar₃, and Ar₄ are each independently a 5- or 6-membered aromatic heterocycle optionally comprising 1 or 2 heteroatoms selected from O, N and S; wherein each of said Ar₂, Ar₃, and Ar₄ is optionally and independently substituted with from 1 to 3 substituents selected from -NR₁₉R₂₀, -C₁₋₆alkyl, -O-C₁₋₆alkyl, and -S-C₁₋₆alkyl;
Het₁, Het₂, Het₃, Het₄, Het₅, Het₆, Het₇ and Het₈ are each independently a 5-or 6-membered monocyclic heterocycle having from 1 to 3 heteroatoms selected from O, N and S, wherein each of said Het₁, Het₂, Het₃, Het₄, Het₅, Het₆, Het₇ and Het₈ is optionally substituted with from 1 to 3 substituents selected from -C₁₋₆alkyl, -O-C₁₋₆alkyl, -S-C₁₋₆alkyl, and -NR₂₁R₂₂; wherein each of said -C₁₋₆alkyl is optionally and independently substituted with from 1 to 3-halo;
Z₁, Z₂, Z₃, Z₄ and Z₅ are each independently selected from C and N;
m and n are each independently 1, 2, 3, or 4.
for use in the diagnosis, prevention and/or treatment of a LRRK2-kinase associated disease.

In yet a further embodiment, the present invention provides a compound of Formula I or a stereoisomer, tautomer, racemic, salt, hydrate, N-oxide form, or solvate thereof, wherein
A₁ is N and A₂ is C
R₁ and R₇ are each independently selected from -H, -halo, -OH, -C₁₋₆alkyl, -O-C₁₋₆alkyl, -S-C₁₋₆alkyl, -NR₉R₁₀, -(C=O)-R₄, -SO₂-R₄, -CN, -NR₉-SO₂-R₄, -C₃₋₆cycloalkyl, and -Het₆; wherein each of said -C₁₋₆alkyl is optionally and independently substituted with from 1 to 3 substituents selected from -halo, -OH, -NR₁₁R₁₂, -O-C₁₋₆alkyl, and -S-C₁₋₆alkyl;
R₂ is selected from -H, -halo, -OH, -C₁₋₆alkyl, -O-C₁₋₆alkyl, -S-C₁₋₆alkyl, -(C=O)-C₁₋₆alkyl,-(C=O)-O-C₁₋₆alkyl, -(C=O)-NR₂₇R₂₈, -Het₃, -(C=O)-Het₃, -SO₂-C₁₋₆alkyl, and -C₃₋₆cycloalkyl; wherein each of said -C₁₋₆alkyl is optionally and independently substituted with from 1 to 3 substituents selected from -halo, -OH, -O-C₁₋₆alkyl, -S-C₁₋₆alkyl, -Het₃, -Ar₂, and -NR₁₃R₁₄;
R₃ is selected from -H, -halo, -OH, -C₁₋₆alkyl, -O-C₁₋₆alkyl, -S-C₁₋₆alkyl, -(C=O)-C₁₋₆alkyl, -(C=O)-O-C₁₋₆alkyl, -Het₂, -C₃₋₆cycloalkyl -(C=O)-Het₂, -(C=O)-NR₂₉R₃₀, and -SO₂-C₁₋₆alkyl; wherein each of said -C₁₋₆alkyl is optionally and independently substituted with from 1 to 3 substituents selected from -halo, -OH, -O-C₁₋₆alkyl, -S-C₁₋₆alkyl, -NR₁₅R₁₆, -Het₂, and -Ar₃;
R₄ is independently selected from -halo, -OH, -C₁₋₆alkyl, -O-C₁₋₆alkyl, -S-C₁₋₆alkyl, -NR₁₇R₁₈, and -Het₄;
R₅ is selected from -H, -C₁₋₆alkyl, -C₃₋₆cycloalkyl; wherein each of said C₁₋₆alkyl or -C₃₋₆cycloalkyl is optionally and independently substituted with from 1 to 3 substituents selected from -halo, -OH, -OC₁₋₆alkyl, -SC₁₋₆alkyl, -Het₅, and -NR₃₁R₃₂;
R₆ is selected from -H, -OH, -halo, -C₁₋₆alkyl, -O-C₁₋₆alkyl, -S-C₁₋₆alkyl, -NR₃₃R₃₄, and -Het₈;
R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉,R₃₀, R₃₁, R₃₂, R₃₃, R₃₄, R₃₇ and R₃₈ are each independently selected from -H, =O, -C₁₋₆alkyl, and -Het₁; wherein each of said -C₁₋₆alkyl is optionally and independently substituted with from 1 to 3 substituents selected from -halo, -OH, -O-C₁₋₆alkyl, -S-C₁₋₆alkyl, -NR₃₅R₃₆, -Het₇, and -Ar₄;
R₃₅ and R₃₆ are each independently selected from -H, =O, and -C₁₋₆alkyl; wherein each of said-C₁₋₆alkyl is optionally and independently substituted with from 1 to 3 substituents selected from -halo, -OH, -O-C₁₋₆alkyl, and -S-C₁₋₆alkyl;
X₁ is selected from -C₁₋₆alkyl-, -O-C₁₋₆alkyl-, -S-C₁₋₆alkyl-, -(C=O)-, -NR₃-(C=O)-, -C₁₋₆alkyl-NR₃-, -NR₃-, -(C=O)-, -NR₃-(C=O)-NR₃₇-, -NR₃-C₁₋₆alkyl-, -NR₃-SO₂-, -NR₃(C=O)-C₁₋₆alkyl-, -(C=O)-NR₃-C₁₋₆alkyl-, -O-C₁₋₆alkyl-O-C₁₋₆alkyl- and -C₁₋₆alkyl-NR₃-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl, and -NR₂₃R₂₄
X₂ is selected from -C₁₋₆alkyl-, -O-C₁₋₆alkyl-, -S-C₁₋₆alkyl-, -(C=O)-, -NR₂-(C=O)-, -C₁₋₆alkyl-NR₂-, -NR₂-, -(C=O)-, -NR₂-(C=O)-NR₃₈-, -NR₂-C_{1_6}alkyl-, -NR₂-SO₂-, -NR₂-(C=O)-C₁₋₆alkyl-, -(C=O)-NR₂-C₁₋₆alkyl-, -O-C₁₋₆alkyl-O-C₁₋₆alkyl- and -C₁₋₆alkyl-NR₂-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl and-NR₂₅R₂₆;
Y is selected from a direct bond, -CHR₆-, -O-, -S-, and -NR₅-;
Ar₂, Ar₃, and Ar₄ are each independently a 5- or 6-membered aromatic heterocycle optionally comprising 1 or 2 heteroatoms selected from O, N and S; wherein each of said Ar₂, Ar₃, and Ar₄ is optionally and independently substituted with from 1 to 3 substituents selected from -NR₁₉R₂₀, -C₁₋₆alkyl, -O-C₁₋₆alkyl, and -S-C₁₋₆alkyl;
Het₁, Het₂, Het₃, Het₄, Het₅, Het₆, Het₇ and Het₈ are each independently a 5-or 6-membered monocyclic heterocycle having from 1 to 3 heteroatoms selected from O, N and S, wherein each of said Het₁, Het₂, Het₃, Het₄, Het₅, Het₆, Het₇ and Het₈ is optionally substituted with from 1 to 3 substituents selected from -C₁₋₆alkyl, -O-C₁₋₆alkyl, -S-C₁₋₆alkyl, and -NR₂₁R₂₂; wherein each of said -C₁₋₆alkyl is optionally and independently substituted with from 1 to 3-halo;
Z₁, Z₂, Z₃, Z₄ and Z₅ are each independently selected from C and N;
m and n are each independently 1, 2, 3, or 4.
for use in the diagnosis, prevention and/or treatment of a LRRK2-kinase associated disease.

In a particular embodiment, the present invention provides compounds for use in the diagnosis, prevention and/or treatment of a LRRK2-kinase associated disease wherein said compounds are selected from:

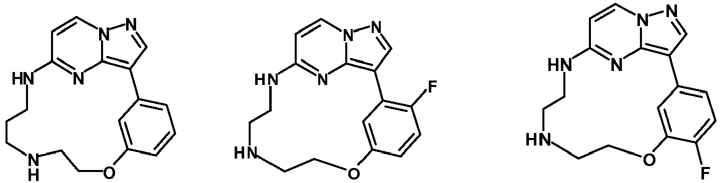

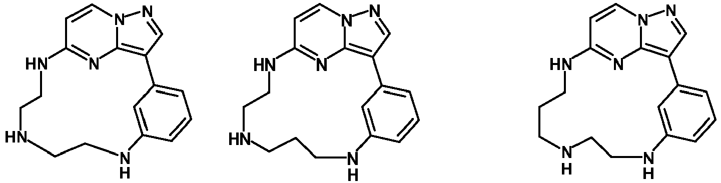

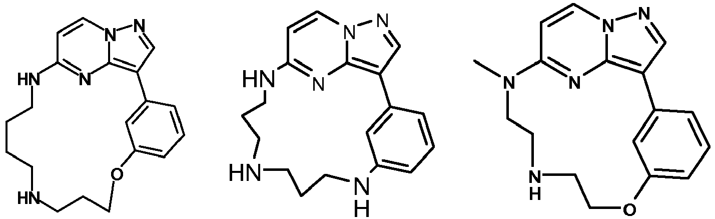

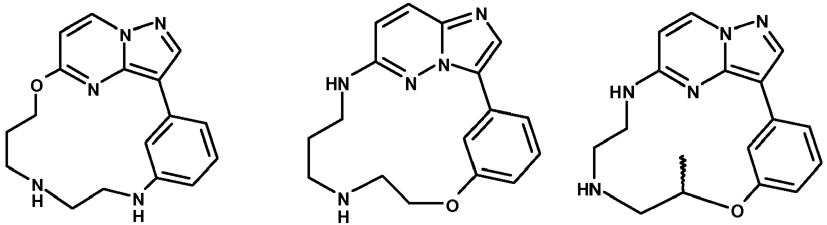

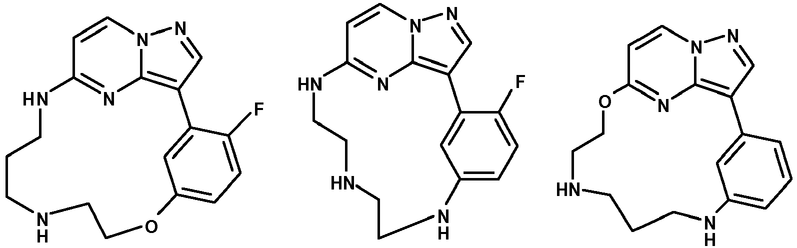

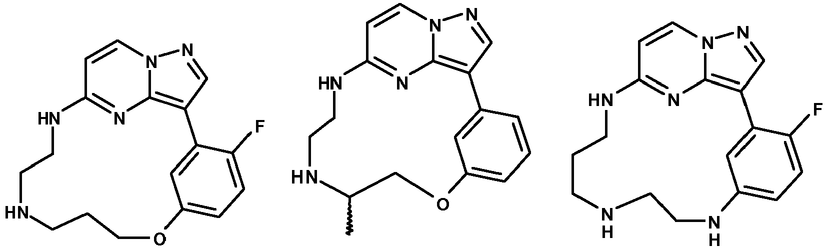

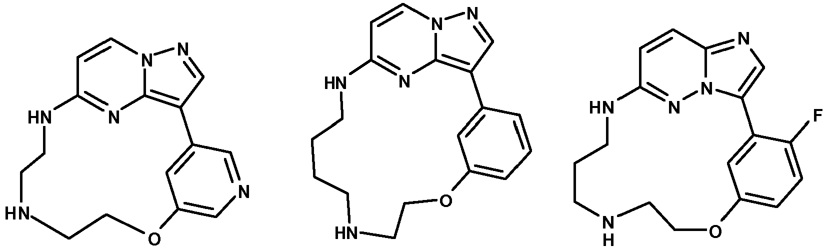

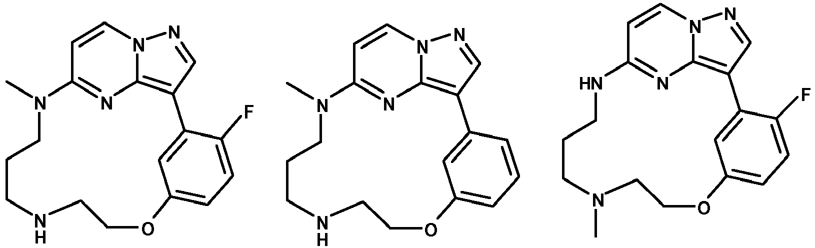

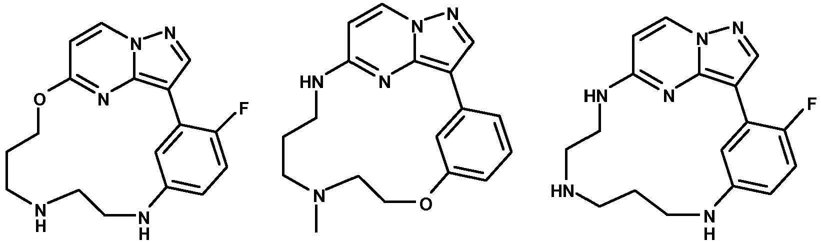

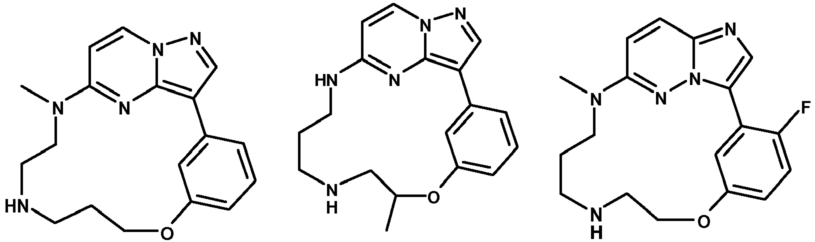

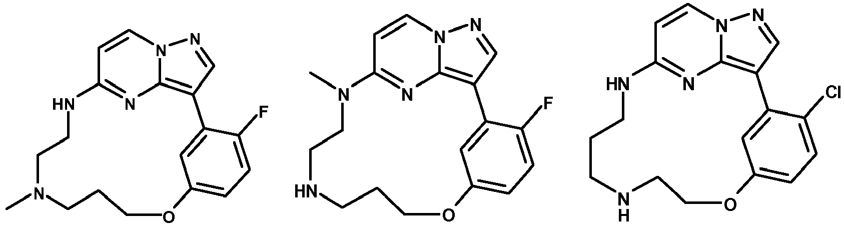

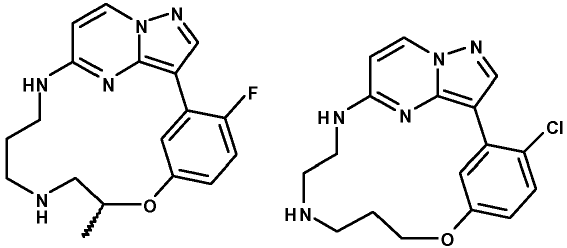

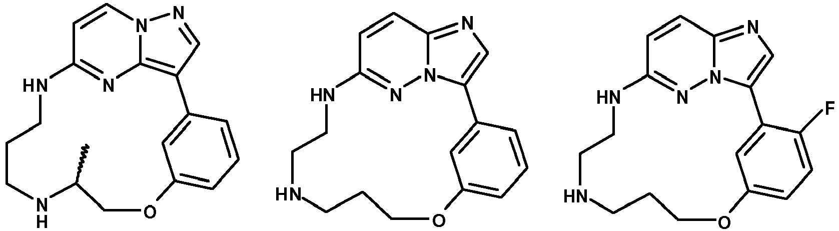

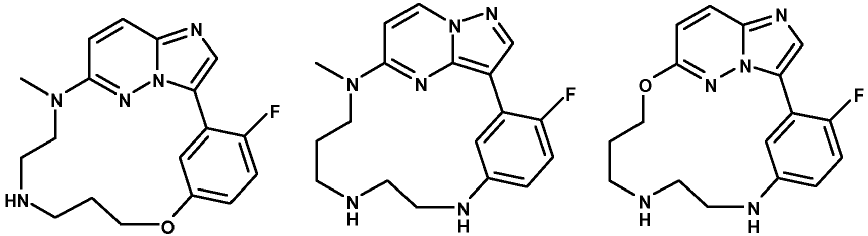

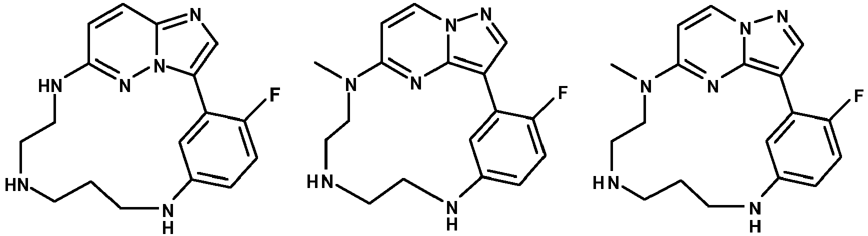

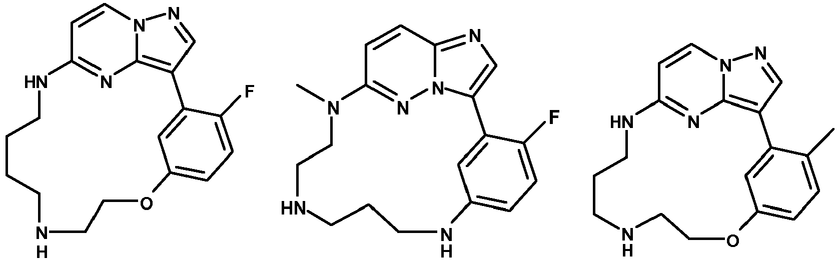

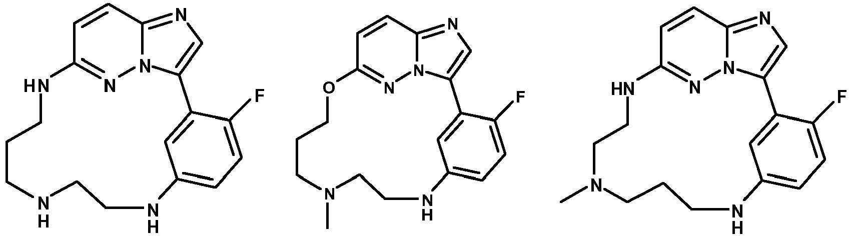

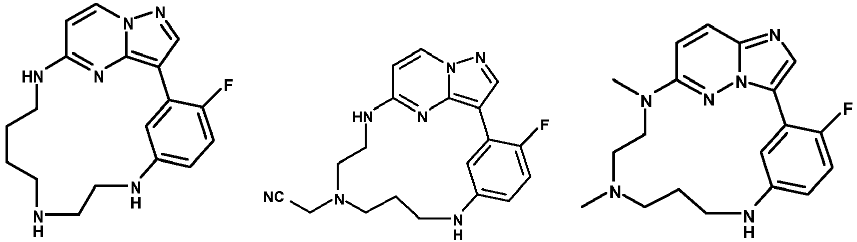

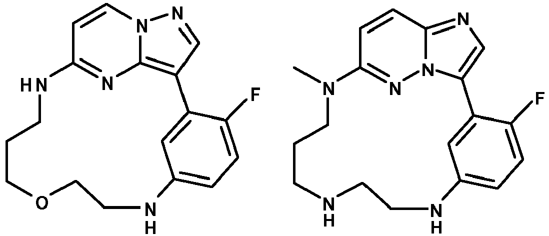

In particular in the compounds according to this invention, the pyrazolopyrimidine or the imidazopyridazine moiety is linked to the aryl or heteroaryl moiety at position Z₄ or Z₅, in accordance with the numbering as provided in Formula I. Furthermore, the R₁ of the compounds according to this invention is preferably linked to the aryl or heteroaryl moiety at position Z₁, Z₂ or Z₃, in accordance with the numbering as provided in Formula I.

In a further embodiment, the present invention provides a compound of Formula (IIc) or a stereoisomer, tautomer, racemic, salt, hydrate, N-oxide form, or solvate thereof, 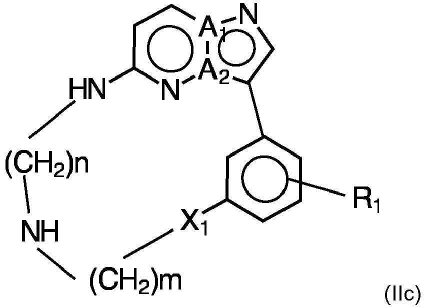 wherein
- A₁ and A₂: are selected from C and N; wherein when A₁ is C, then A₂ is N; and wherein when A₂ is C, then A₁ is N;
- R₁: is selected from the list comprising -H, -F, -CH₃, and -CN
- X₁: is selected from the list comprising -NH- and -O-
- m and n: are each independently 1, 2, 3, or 4
for use in the prevention and/or treatment of a LRRK2-kinase associated disease.

In a particular embodiment, the present invention provides compounds for use in the diagnosis, prevention and/or treatment of a LRRK2-kinase associated disease wherein said compounds are selected from: 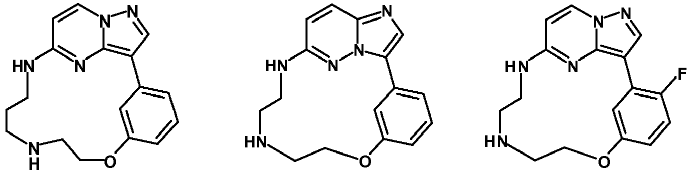 and

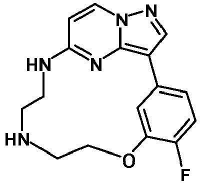

In a particular embodiment, the LRRK2-kinase associated disease is chosen between Crohn's disease, leprosy, and a neurological disorder. Preferably, the neurological disorder is Parkinson's disease or Alzheimer's disease.

The present invention further provides a pharmaceutical composition for use in the diagnosis, prevention and/or treatment of a LRRK2-kinase associated disease comprising a compound according to the present invention.

The present disclosure also provides the use of a compound, or a composition according to this invention, suitable for inhibiting the activity of a kinase; in particular a LRRK2 kinase.

Furthermore, the present disclosure provides the use of a compound or a composition according to this invention, for the diagnosis, prevention and/or treatment of a LRRK2-kinase associated disease.

Moreover, the present disclosure provides a method for the prevention and/or treatment of a LRRK2-kinase associated disease; said method comprising administering to a subject in need thereof a compound or a composition according to this invention.

Furthermore, the present invention provides new compounds of Formula (IIIc) or a stereoisomer, tautomer, racemic, salt, hydrate, N-oxide form, or solvate thereof, 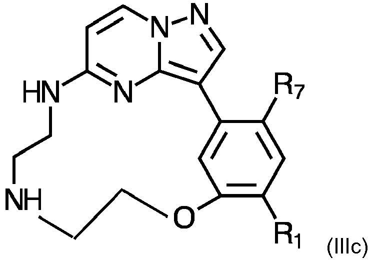 wherein
- R₁: is -H and R₇ is -F; or
- R₇: is -H and R₁ is -F.

In another embodiment, the present invention provides a compound of formula (IVc) or a stereoisomer, tautomer, racemic, salt, hydrate, N-oxide form, or solvate thereof: 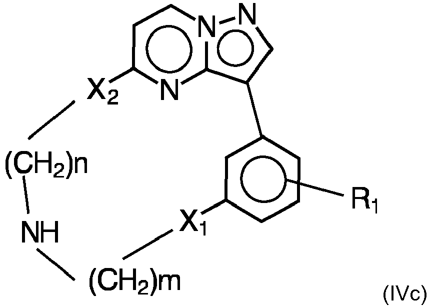 wherein
- R₁: is selected from the list comprising -H, -F, -CH₃, and -CN
- X₁ and X₂: are each independently selected from the list comprising -NH- and -O-
- m and n: are each independently 1, 2, 3, or 4.

with the proviso that said compound is not

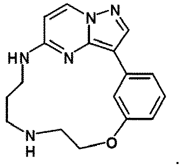

In a further aspect, the present invention provides a compound according to this invention for use as a medicine or diagnostic agent.

The present invention also provides a pharmaceutical composition comprising a compound as defined herein.

In another embodiment, the present invention provides a compound of formula (Vc) 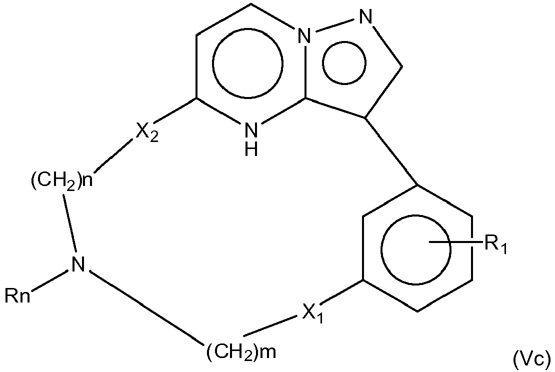 wherein
- R₁: is selected from the list comprising -H, and -F,
- X₁ and X₂: are each independently selected from the list comprising -NRx- and -O-,
- Rx: is H or a methyl group,
- m and n: are each independently 1, 2, 3, or 4,
- Rn: is H or a methyl group,
with the proviso that said compound is not

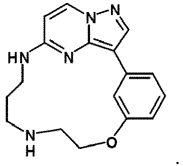

In another embodiment, the present invention provides a compound selected from the list comprising:

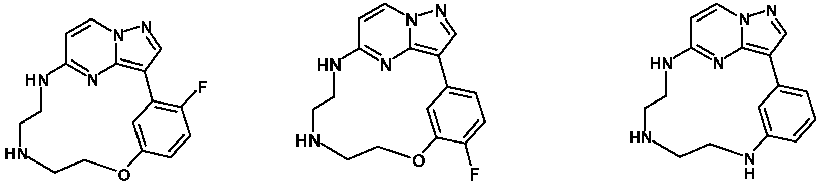

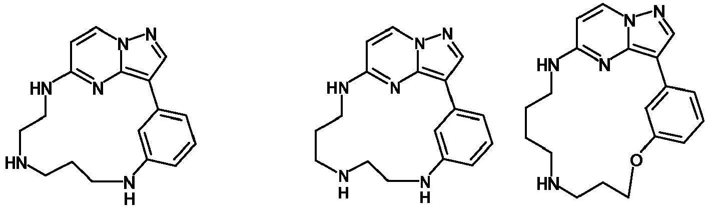

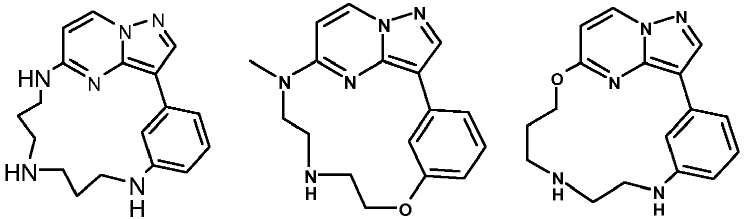

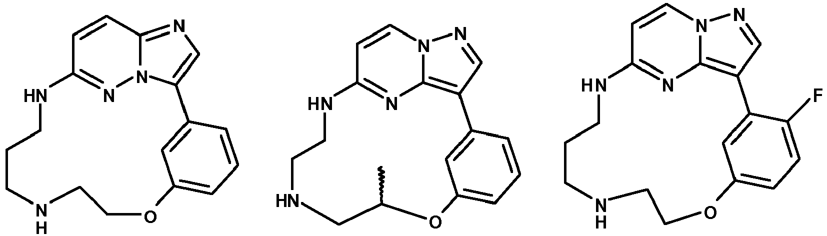

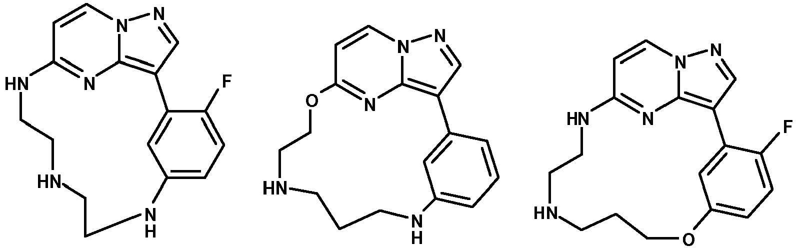

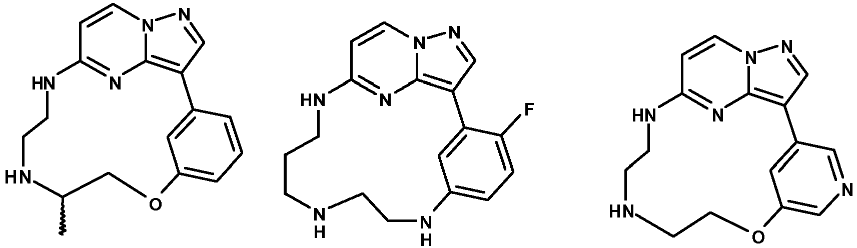

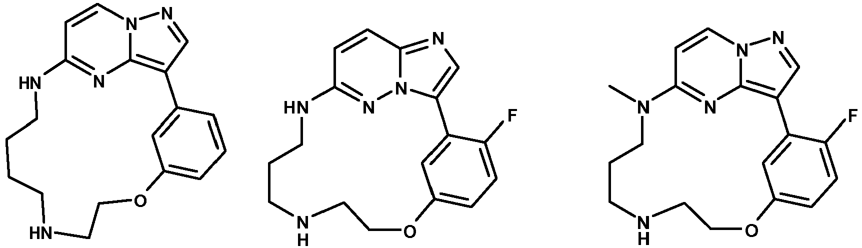

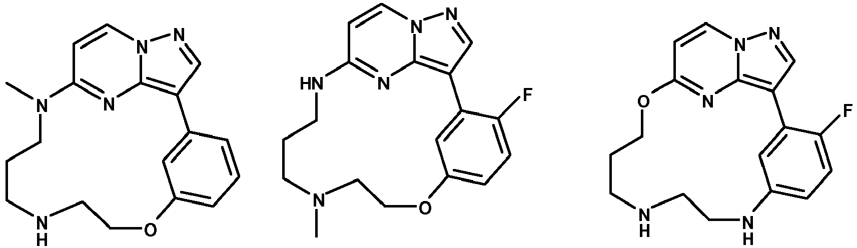

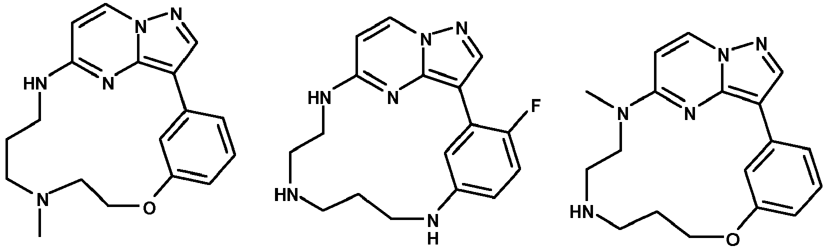

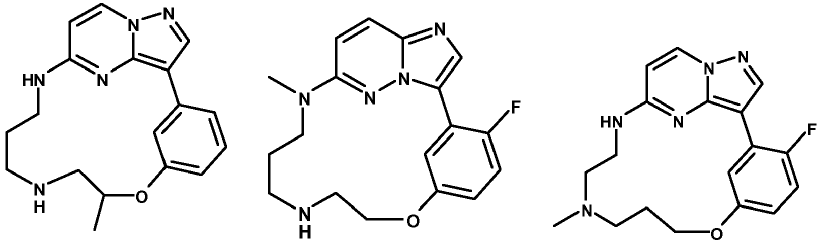

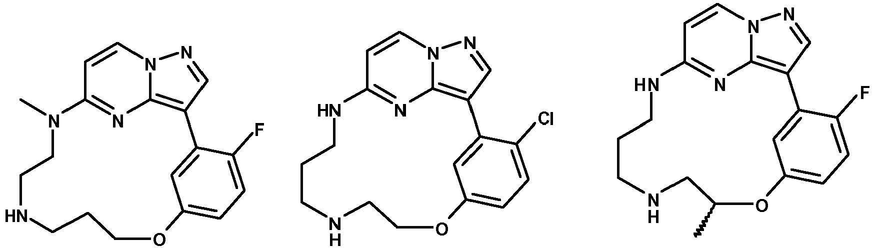

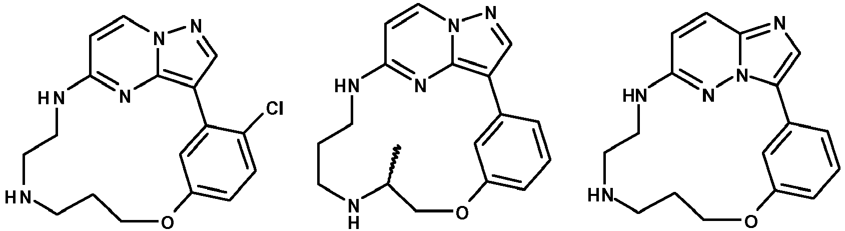

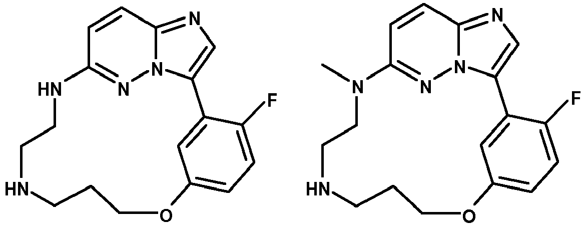

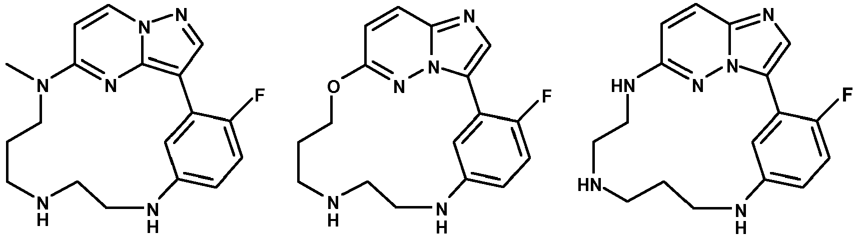

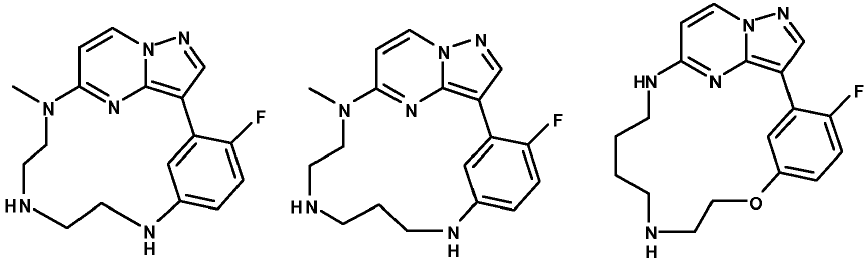

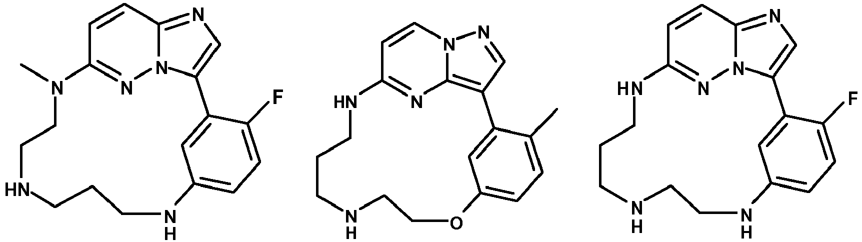

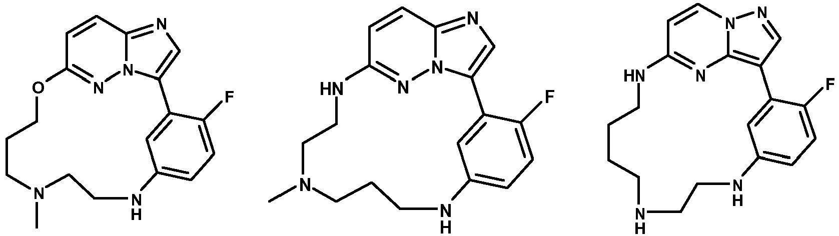

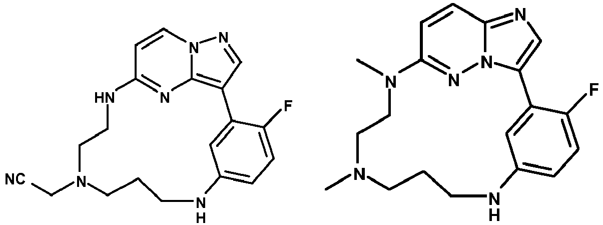

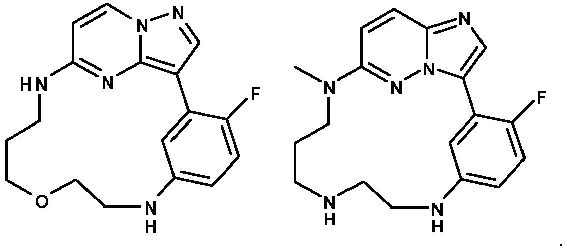

### DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Unless a context dictates otherwise, asterisks are used herein to indicate the point at which a mono- or bivalent radical depicted is connected to the structure to which it relates and of which the radical forms part.

As already mentioned hereinbefore, in a first aspect the present invention provides a compound of Formula I or a stereoisomer, tautomer, racemic, salt, hydrate, N-oxide form, or solvate thereof, 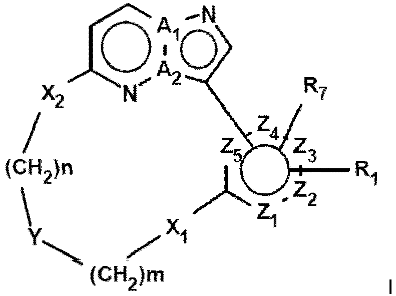 Wherein
A₁ and A₂ are selected from C and N; wherein when A₁ is C, then A₂ is N; and wherein when A₂ is C, then A₁ is N;
R₁ and R₇ are each independently selected from -H, -halo, -OH, -C₁₋₆alkyl, -O-C₁₋₆alkyl, -S-C₁₋₆alkyl, -NR₉R₁₀, -(C=O)-R₄, -SO₂-R₄, -CN, -NR₉-SO₂-R₄, -C₃₋₆cycloalkyl, and -Het₆; wherein each of said -C₁₋₆alkyl is optionally and independently substituted with from 1 to 3 substituents selected from -halo, -OH, -NR₁₁R₁₂, -O-C₁₋₆alkyl, and -S-C₁₋₆alkyl;
R₂ is selected from -H, -halo, -OH, -C₁₋₆alkyl, -O-C₁₋₆alkyl, -S-C₁₋₆alkyl, -(C=O)-C₁₋₆alkyl,-(C=O)-O-C₁₋₆alkyl, -(C=O)-NR₂₇R₂₈, -Het₃, -(C=O)-Het₃, -SO₂-C₁₋₆alkyl, and -C₃₋₆cycloalkyl; wherein each of said -C₁₋₆alkyl is optionally and independently substituted with from 1 to 3 substituents selected from -halo, -OH, -O-C₁₋₆alkyl, -S-C₁₋₆alkyl, -Het₃, -Ar₂, and -NR₁₃R₁₄;
R₃ is selected from -H, -halo, -OH, -C₁₋₆alkyl, -O-C₁₋₆alkyl, -S-C₁₋₆alkyl, -(C=O)-C₁₋₆alkyl, -(C=O)-O-C₁₋₆alkyl, -Het₂, -C₃₋₆cycloalkyl -(C=O)-Het₂, -(C=O)-NR₂₉R₃₀, and -SO₂-C₁₋₆alkyl; wherein each of said -C₁₋₆alkyl is optionally and independently substituted with from 1 to 3 substituents selected from -halo, -OH, -O-C₁₋₆alkyl, -S-C₁₋₆alkyl, -NR₁₅R₁₆, -Het₂, and -Ar₃;
R₄ is independently selected from -halo, -OH, -C₁₋₆alkyl, -O-C₁₋₆alkyl, -S-C₁₋₆alkyl, -NR₁₇R₁₈, and -Het₄;
R₅ is selected from -H, -C₁₋₆alkyl, -C₃₋₆cycloalkyl; wherein each of said C₁₋₆alkyl or -C₃₋₆cycloalkyl is optionally and independently substituted with from 1 to 3 substituents selected from -halo, -OH, -OC₁₋₆alkyl, -SC₁₋₆alkyl, -Het₅, and -NR₃₁R₃₂;
R₆ is selected from -H, -OH, -halo, -C₁₋₆alkyl, -O-C₁₋₆alkyl, -S-C₁₋₆alkyl, -NR₃₃R₃₄, and -Het₈;
R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉,R₃₀, R₃₁, R₃₂, R₃₃, R₃₄, R₃₇ and R₃₈ are each independently selected from -H, =O, -C₁₋₆alkyl, and -Het₁; wherein each of said -C₁₋₆alkyl is optionally and independently substituted with from 1 to 3 substituents selected from -halo, -OH, -O-C₁₋₆alkyl, -S-C₁₋₆alkyl, -NR₃₅R₃₆,-Het₇, and -Ar₄;
R₃₅ and R₃₆ are each independently selected from -H, =O, and -C₁₋₆alkyl; wherein each of said-C₁₋₆alkyl is optionally and independently substituted with from 1 to 3 substituents selected from -halo, -OH, -O-C₁₋₆alkyl, and -S-C₁₋₆alkyl;
X₁ is selected from -C₁₋₆alkyl-, -O-C₁₋₆alkyl-, -S-C₁₋₆alkyl-, -(C=O)-, -NR₃-(C=O)-, -C₁₋₆alkyl-NR₃-, -NR₃-, -(C=O)-, -NR₃-(C=O)-NR₃₇-, -NR₃-C₁₋₆alkyl-, -NR₃-SO₂-, -NR₃-(C=O)-C₁₋₆alkyl-, -(C=O)-NR₃-C₁₋₆alkyl-, -O-C₁₋₆alkyl-O-C₁₋₆alkyl- and -C₁₋₆alkyl-NR₃-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl, and -NR₂₃R₂₄
X₂ is selected from -C₁₋₆alkyl-, -O-C₁₋₆alkyl-, -S-C₁₋₆alkyl-, -(C=O)-, -NR₂-(C=O)-, -C₁₋₆alkyl-NR₂-, -NR₂-, -(C=O)-, -NR₂-(C=O)-NR₃₈-, -NR₂-C₁₋₆alkyl-, -NR₂-SO₂-, -NR₂-(C=O)-C₁₋₆alkyl-, -(C=O)-NR₂-C₁₋₆alkyl-, -O-C₁₋₆alkyl-O-C₁₋₆alkyl- and -C₁₋₆alkyl-NR₂-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl and-NR₂₅R₂₆;
Y is selected from a direct bond, -CHR₆-, -O-, -S-, and -NR₅-;
Ar₂, Ar₃, and Ar₄ are each independently a 5- or 6-membered aromatic heterocycle optionally comprising 1 or 2 heteroatoms selected from O, N and S; wherein each of said Ar₂, Ar₃, and Ar₄ is optionally and independently substituted with from 1 to 3 substituents selected from -NR₁₉R₂₀, -C₁₋₆alkyl, -O-C₁₋₆alkyl, and -S-C₁₋₆alkyl;
Het₁, Het₂, Het₃, Het₄, Het₅, Het₆, Het₇ and Het₈ are each independently a 5-or 6-membered monocyclic heterocycle having from 1 to 3 heteroatoms selected from O, N and S, wherein each of said Het₁, Het₂, Het₃, Het₄, Het₅, Het₆, Het₇ and Het₈ is optionally substituted with from 1 to 3 substituents selected from -C₁₋₆alkyl, -O-C₁₋₆alkyl, -S-C₁₋₆alkyl, and -NR₂₁R₂₂; wherein each of said -C₁₋₆alkyl is optionally and independently substituted with from 1 to 3-halo;
Z₁, Z₂, Z₃, Z₄ and Z₅ are each independently selected from C and N;
m and n are each independently 1, 2, 3, or 4.
for use in the diagnosis, prevention and/or treatment of a LRRK2-kinase associated disease. Unless indicated otherwise, all of the above radicals can be read both ways. For example, when A is -(C=O)-NR₅-, the -(C=O)- may be attached to X₂ and -NR₅- attached to X₁. Alternatively, the -(C=O)- may be attached to X₁ and -NR₅- attached to X₁. What is called "left part" of a radical is for example when A is -(C=O)-NR₅-, -(C=O)-, and the "right part" is -NR₅-.

Preferably, Y is such as the left part of the possible values of Y (i.e. in particular -CH from-CHR₆-) is attached to X₁. Alternatively, Y is such as the right part of the possible values of Y (i.e. in particular -R₆- from -CHR₆- is attached to X₁.

Preferably, X₁ is such as the left part of the possible values of X₁ (i.e. in particular -O from -O-C₁₋₆alkyl, -S from -S-C₁₋₆alkyl, -NR₃ from -NR₃-(C=O) and -NR₃-C₁₋₆alkyl, -SO₂ from -SO₂-NR₃, etc) is attached to the Z₁-Z₅ aryl or heteroaryl moiety. Alternatively, X₁ is such as the right part of the possible values of X₁ (i.e. in particular (C₁₋₆alkyl)- from -O-C₁₋₆alkyl, -S-C₁₋₆alkyl and-NR₃-C₁₋₆alkyl, -(C=O) from -NR₃-(C=O), (NR₃)- from -SO₂-NR₃, etc) is attached to the Z₁-Z₅ aryl or heteroaryl moiety.

Preferably, X₂ is such as the left part of the possible values of X₂ (i.e. in particular -O from -O-C₁₋₂alkyl, -S from -S-C₁₋₆alkyl, -(C=O) from -(C=O)-NR₂, -NR₂ from -NR₂-C₁₋₆alkyl, -SO₂ from-SO₂-NR₂, etc) is attached to the pyrazolopyrimidine moiety. Alternatively, X₂ is such as the right part of the possible values of X₂ (i.e. in particular (C₁₋₆alkyl)- from -O-C₁₋₆alkyl, -S-C₁₋₆alkyl and -NR₂-C₁₋₆alkyl, (NR₂)- from -(C=O)-NR₂ and -SO₂-NR₂, etc) is attached to the pyrazolopyrimidine moiety.

The same principle applies to all the radicals of the invention unless specified otherwise.

When describing the compounds of the invention, the terms used are to be construed in accordance with the following definitions, unless a context dictates otherwise:
The term "alkyl" by itself or as part of another substituent refers to fully saturated hydrocarbon radicals. Generally, alkyl groups of this invention comprise from 1 to 6 carbon atoms. Alkyl groups may be linear or branched and may be substituted as indicated herein. When a subscript is used herein following a carbon atom, the subscript refers to the number of carbon atoms that the named group may contain. Thus, for example, C₁₋₆alkyl means an alkyl of one to six carbon atoms. Examples of alkyl groups are methyl, ethyl, n-propyl, i-propyl, butyl, and its isomers (e.g. n-butyl, i-butyl and t-butyl); pentyl and its isomers, hexyl and its isomers. C₁-C₆ alkyl includes all linear, branched, or cyclic alkyl groups with between 1 and 6 carbon atoms, and thus includes methyl, ethyl, n-propyl, i-propyl, butyl and its isomers (e.g. n-butyl, i-butyl and t-butyl); pentyl and its isomers, hexyl and its isomers, cyclopropyl, cyclobutyl, cyclopentyl, and cyclohexyl.

The term "optionally substituted alkyl" refers to an alkyl group optionally substituted with one or more substituents (for example 1 to 3 substituents, for example 1, 2 or 3 substituents or 1 to 2 substituents) at any available point of attachment. Non-limiting examples of such substituents include -halo, -OH, primary and secondary amides, -O-C₁₋₆alkyl, -S-C₁₋₆alkyl, heteroaryl, aryl, and the like.

The term "cycloalkyl" by itself or as part of another substituent is a cyclic alkyl group, that is to say, a monovalent, saturated, or unsaturated hydrocarbyl group having a cyclic structure. Cycloalkyl includes all saturated or partially saturated (containing 1 or 2 double bonds) hydrocarbon groups having a cyclic structure. Cycloalkyl groups may comprise 3 or more carbon atoms in the ring and generally, according to this invention comprise from 3 to 6 atoms. Examples of cycloalkyl groups include but are not limited to cyclopropyl, cyclobutyl, cyclopentyl, cyclohexyl.

Where alkyl groups as defined are divalent, i.e., with two single bonds for attachment to two other groups, they are termed "alkylene" groups. Non-limiting examples of alkylene groups includes methylene, ethylene, methylmethylene, trimethylene, propylene, tetramethylene, ethylethylene, 1,2-dimethylethylene, pentamethylene and hexamethylene.

Generally, alkylene groups of this invention preferably comprise the same number of carbon atoms as their alkyl counterparts. Where an alkylene or cycloalkylene biradical is present, connectivity to the molecular structure of which it forms part may be through a common carbon atom or different carbon atom. To illustrate this applying the asterisk nomenclature of this invention, a C₃ alkylene group may be for example *-CH₂CH₂CH₂-*, *-CH(-CH₂CH₃)-*, or *-CH₂CH(-CH₃)-*. Likewise a C₃ cycloalkylene group may be

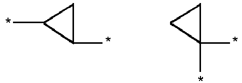

The terms "heterocycle" as used herein by itself or as part of another group refer to nonaromatic, fully saturated or partially unsaturated cyclic groups (for example, 3 to 6 membered monocyclic ring systems, or 8-10 membered bicyclic rings) which have at least one heteroatom in at least one carbon atom-containing ring. Each ring of the heterocyclic group containing a heteroatom may have 1, 2, 3 or 4 heteroatoms selected from nitrogen atoms, oxygen atoms and/or sulfur atoms. An optionally substituted heterocyclic refers to a heterocyclic having optionally one or more substituents (for example 1 to 4 substituents, or for example 1, 2, 3 or 4), selected from those defined above for substituted alkyl.

Exemplary heterocyclic groups include piperidinyl, azetidinyl, imidazolinyl, imidazolidinyl, isoxazolinyl, oxazolidinyl, isoxazolidinyl, thiazolidinyl, isothiazolidinyl, piperidyl, succinimidyl, 3H-indolyl, isoindolinyl, chromenyl, isochromanyl, xanthenyl, 2H-pyrrolyl, 1-pyrrolinyl, 2-pyrrolinyl, 3-pyrrolinyl, pyrrolidinyl, 4H-quinolizinyl, 4aH-carbazolyl, 2-oxopiperazinyl, piperazinyl, homopiperazinyl, 2-pyrazolinyl, 3-pyrazolinyl, pyranyl, dihydro-2H-pyranyl, 4H-pyranyl, 3,4-dihydro- 2H-pyranyl, phthalazinyl, oxetanyl, thietanyl, 3-dioxolanyl, 1,3-dioxanyl, 2,5-dioximidazolidinyl, 2,2,4-piperidonyl, 2-oxopiperidinyl, 2-oxopyrrolodinyl, 2-oxoazepinyl, indolinyl, tetrahydropyranyl, tetrahydrofuranyl, tetrehydrothienyl, tetrahydroquinolinyl, tetrahydroisoquinolinyl, thiomorpholinyl, thiomorpholinyl sulfoxide, thiomorpholinyl sulfone, 1,3-dioxolanyl, 1,4-oxathianyl, 1,4-dithianyl, 1,3,5-trioxanyl, 6H-1,2,5-thiadiazinyl, 2H-1,5,2-dithiazinyl, 2H-oxocinyl, 1H-pyrrolizinyl, tetrahydro- 1,1-dioxothienyl, N- formylpiperazinyl, and morpholinyl; in particular pyrrolidinyl, imidazolidinyl, pyrazolidinyl, piperidinyl, dioxolanyl, dioxanyl, morpholinyl, thiomorpholinyl, piperazinyl, thiazolidinyl, tetrahydropyranyl, and tetrahyd rofu ranyl.

8-10 membered heterocyclic groups are also meant to include spiro-groups, which are bicyclic compounds with both rings connected through a single atom, such as for example spiro[4.5]decane, which is a spiro compound consisting of a cyclohexane ring and a cyclopentane ring.

The term "aryl" as used herein refers to a polyunsaturated, aromatic hydrocarbyl group having from 5-10 atoms. Aryl is also intended to include the partially hydrogenated derivatives of the carbocyclic systems enumerated herein. Non-limiting examples of aryl comprise phenyl, biphenylyl, biphenylenyl, 5- or 6-tetralinyl, 1-, 2-, 3-, 4-, 5-, 6-, 7-, or 8-azulenyl, 1- or 2-naphthyl, 1-, 2-, or 3-indenyl, 1-, 2-, or 9-anthryl, 1- 2-, 3-, 4-, or 5-acenaphtylenyl, 3-, 4-, or 5-acenaphtenyl, 1-, 2-, 3-, 4-, or 10-phenanthryl, 1- or 2-pentalenyl, 1, 2-, 3-, or 4-fluorenyl, 4- or 5-indanyl, 5-, 6-, 7-, or 8-tetrahydronaphthyl, 1,2,3,4-tetrahydronaphthyl, 1,4-dihydronaphthyl, dibenzo[a,d]cylcoheptenyl, and 1-, 2-, 3-, 4-, or 5-pyrenyl; in particular phenyl.

The aryl ring can optionally be substituted by one or more substituents. An "optionally substituted aryl" refers to an aryl having optionally one or more substituents (for example 1 to 5 substituents, for example 1, 2, 3 or 4) at any available point of attachment, selected from those defined above for substituted alkyl.

Where a carbon atom in an aryl group is replaced with a heteroatom, the resultant ring is referred to herein as a heteroaryl ring.

The term "heteroaryl" as used herein by itself or as part of another group refers but is not limited to 5 to 10 carbon-atom aromatic rings in which one or more carbon atoms can be replaced by oxygen, nitrogen or sulfur atoms. Non-limiting examples of such heteroaryl, include: pyrrolyl, furanyl, thiophenyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, imidazo[2,1-b][1,3]thiazolyl, thieno[3,2-b]furanyl, thieno[3,2-b]thiophenyl, thieno[2,3-d][1,3]thiazolyl, thieno[2,3-d]imidazolyl, tetrazolo[1,5-a]pyridinyl, indolyl, indolizinyl, isoindolyl, benzofuranyl, isobenzofuranyl, benzothiophenyl, isobenzothiophenyl, indazolyl, benzimidazolyl, 1,3-benzoxazolyl, 1,2-benzisoxazolyl, 2,1-benzisoxazolyl, 1,3-benzothiazolyl, 1,2-benzoisothiazolyl, 2,1-benzoisothiazolyl, benzotriazolyl, 1,2,3-benzoxadiazolyl, 2,1,3-benzoxadiazolyl, 1,2,3-benzothiadiazolyl, 2,1,3-benzothiadiazolyl, thienopyridinyl, purinyl, imidazo[1,2-a]pyridinyl, 6-oxo-pyridazin-1(6H)-yl, 2-oxopyridin-1(2H)-yl, 6-oxo-pyridazin-1(6H)-yl, 2-oxopyridin-1(2H)-yl, 1,3-benzodioxolyl, quinolinyl, isoquinolinyl, cinnolinyl, quinazolinyl, quinoxalinyl, 7-azaindolyl, 6-azaindolyl, 5-azaindolyl, 4-azaindolyl.

An "optionally substituted heteroaryl" refers to a heteroaryl having optionally one or more substituents (for example 1 to 4 substituents, for example 1, 2, 3 or 4), selected from those defined above for substituted alkyl.

The term "halo" or "halogen" as a group or part of a group is generic for fluoro, chloro, bromo, or iodo, as well as any suitable isotope thereof.

Whenever the term "substituted" is used in the present invention, it is meant to indicate that one or more hydrogens on the atom indicated in the expression using "substituted" is replaced with a selection from the indicated group, provided that the indicated atom's normal valency is not exceeded, and that the substitution results in a chemically stable compound, i.e. a compound that is sufficiently robust to survive isolation to a useful degree of purity from a reaction mixture, and formulation into a therapeutic and/or diagnostic agent.

Where groups may be optionally substituted, such groups may be substituted once or more, and preferably once, twice or thrice. Substituents may be selected from, those defined above for substituted alkyl.

As used herein the terms such as "alkyl, aryl, or cycloalkyl, each being optionally substituted with" or "alkyl, aryl, or cycloalkyl, optionally substituted with" refers to optionally substituted alkyl, optionally substituted aryl and optionally substituted cycloalkyl.

More generally, from the above, it will be clear to the skilled person that the compounds of the invention may exist in the form of different isomers and/or tautomers, including but not limited to geometrical isomers, conformational isomers, E/Z-isomers, stereochemical isomers (i.e. enantiomers and diastereoisomers) and isomers that correspond to the presence of the same substituents on different positions of the rings present in the compounds of the invention. All such possible isomers, tautomers and mixtures thereof are included within the scope of the invention.

In addition, the disclosure includes isotopically-labelled compounds and salts, which are identical to compounds of formula (I), but for the fact that one or more atoms are replaced by an atom having an atomic mass or mass number different from the atomic mass or mass number most commonly found in nature. Examples of isotopes that can be incorporated into compounds of formula (I) are isotopes of hydrogen, carbon, nitrogen, fluorine, such as ³H, ¹¹C, ¹³N, ¹⁴C, ¹⁵O and ¹⁸F. Such isotopically-labelled compounds of formula (I) are useful in drug and/or substrate tissue distribution assays. For example ¹¹C and ¹⁸F isotopes are particularly useful in PET (Positron Emission Tomography). PET is useful in brain imaging. Isotopically labeled compounds of formula (I) can generally be prepared by carrying out the procedures disclosed below, by substituting a readily available non-isotopically labeled reagent with an isotopically labeled reagent.

Whenever used in the present disclosure the term "compounds of the invention" or a similar term is meant to include the compounds of general Formula I and any subgroup thereof. This term also refers to the compounds as depicted in Table 1, their derivatives, *N*-oxides, salts, solvates, hydrates, stereoisomeric forms, racemic mixtures, tautomeric forms, optical isomers, analogues, pro-drugs, esters, and metabolites, as well as their quaternized nitrogen analogues. The *N-*oxide forms of said compounds are meant to comprise compounds wherein one or several nitrogen atoms are oxidized to the so-called *N*-oxide.

As used in the specification and the appended claims, the singular forms "a", "an", and "the" include plural referents unless the context clearly dictates otherwise. By way of example, "a compound" means one compound or more than one compound.

The terms described above and others used in the specification are well understood to those in the art.

In a particular embodiment, the present invention provides compounds of Formula I or a stereoisomer, tautomer, racemic, salt, hydrate, N-oxide form, or solvate thereof; for use in the diagnosis prevention and/or treatment of a LRRK2-kinase associated disease; wherein one or more of the following applies
A₁ and A₂ are selected from C and N; wherein when A₁ is C, then A₂ is N; and wherein when A₂ is C, then A₁ is N;
R₁ and R₇ are each independently selected from -H, -halo, -OH, -C₁₋₆alkyl, -O-C₁₋₆alkyl, -S-C₁₋₆alkyl, -NR₉R₁₀, -(C=O)-R₄, -SO₂-R₄, -CN, -NR₉-SO₂-R₄, -C₃₋₆cycloalkyl, and -Het₆; wherein each of said -C₁₋₆alkyl is optionally and independently substituted with from 1 to 3 substituents selected from -halo, -OH, -NR₁₁R₁₂, -O-C₁₋₆alkyl, and -S-C₁₋₆alkyl;
R₂ is selected from -H, -halo, -OH, -C₁₋₆alkyl, -O-C₁₋₆alkyl, -S-C₁₋₆alkyl, -(C=O)-C₁₋₆alkyl,-(C=O)-O-C₁₋₆alkyl, -(C=O)-NR₂₇R₂₈, -Het₃, -(C=O)-Het₃, -SO₂-C₁₋₆alkyl, and -C₃₋₆cycloalkyl; wherein each of said -C₁₋₆alkyl is optionally and independently substituted with from 1 to 3 substituents selected from -halo, -OH, -O-C₁₋₆alkyl, -S-C₁₋₆alkyl, -Het₃, -Ar₂, and -NR₁₃R₁₄;
R₃ is selected from -H, -halo, -OH, -C₁₋₆alkyl, -O-C₁₋₆alkyl, -S-C₁₋₆alkyl, -(C=O)-C₁₋₆alkyl, -(C=O)-O-C₁₋₆alkyl, -Het₂, -C₃₋₆cycloalkyl -(C=O)-Het₂, -(C=O)-NR₂₉R₃₀, and -SO₂-C₁₋₆alkyl; wherein each of said -C₁₋₆alkyl is optionally and independently substituted with from 1 to 3 substituents selected from -halo, -OH, -O-C₁₋₆alkyl, -S-C₁₋₆alkyl, -NR₁₅R₁₆, -Het₂, and -Ar₃;
R₄ is independently selected from -halo, -OH, -C₁₋₆alkyl, -O-C₁₋₆alkyl, -S-C₁₋₆alkyl, -NR₁₇R₁₈, and -Het₄;
R₅ is selected from -H, -C₁₋₆alkyl, -C₃₋₆cycloalkyl; wherein each of said C₁₋₆alkyl or -C₃₋₆cycloalkyl is optionally and independently substituted with from 1 to 3 substituents selected from -halo, -OH, -OC₁₋₆alkyl, -SC₁₋₆alkyl, -Het₅, and -NR₃₁R₃₂;
R₆ is selected from -H, -OH, -halo, -C₁₋₆alkyl, -O-C₁₋₆alkyl, -S-C₁₋₆alkyl, -NR₃₃R₃₄, and -Het₈;
R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉,R₃₀, R₃₁, R₃₂, R₃₃, R₃₄, R₃₇ and R₃₈ are each independently selected from -H, =O, -C₁₋₆alkyl, and -Het₁; wherein each of said -C₁₋₆alkyl is optionally and independently substituted with from 1 to 3 substituents selected from -halo, -OH, -O-C₁₋₆alkyl, -S-C₁₋₆alkyl, -NR₃₅R₃₆,-Het₇, and -Ar₄;
R₃₅ and R₃₆ are each independently selected from -H, =O and -C₁₋₆alkyl; wherein each of said-C₁₋₆alkyl is optionally and independently substituted with from 1 to 3 substituents selected from -halo, -OH, -O-C₁₋₆alkyl, and -S-C₁₋₆alkyl;
X₁ is selected from -C₁₋₆alkyl-, -O-C₁₋₆alkyl-, -S-C₁₋₆alkyl-, -(C=O)-, -NR₃-(C=O)-, -C₁₋₆alkyl-NR₃-, -NR₃-, -(C=O)-, -NR₃-(C=O)-NR₃₇-, -NR₃-C₁₋₆alkyl-, -NR₃-SO₂-, -NR₃-(C=O)-C₁₋₆alkyl-, -(C=O)-NR₃-C₁₋₆alkyl-, -O-C₁₋₆alkyl-O-C₁₋₆alkyl- and -C₁₋₆alkyl-NR₃-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl, and -NR₂₃R₂₄
X₂ is selected from -C₁₋₆alkyl-, -O-C₁₋₆alkyl-, -S-C₁₋₆alkyl-, -(C=O)-, -NR₂-(C=O)-, -C₁₋₆alkyl-NR₂-, -NR₂-, -(C=O)-, -NR₂-(C=O)-NR₃₈-, -NR₂-C₁₋₆alkyl-, -NR₂-SO₂-, -NR₂-(C=O)-C₁₋₆alkyl-, -(C=O)-NR₂-C₁₋₆alkyl-, -O-C₁₋₆alkyl-O-C₁₋₆alkyl- and -C₁₋₆alkyl-NR₂-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl and-NR₂₅R₂₆;
Y is selected from a direct bond, -CHR₆-, -O-, -S-, and -NR₅-;
Ar₂, Ar₃, and Ar₄ are each independently a 5- or 6-membered aromatic heterocycle optionally comprising 1 or 2 heteroatoms selected from O, N and S; wherein each of said Ar₂, Ar₃, and Ar₄ is optionally and independently substituted with from 1 to 3 substituents selected from -NR₁₉R₂₀, -C₁₋₆alkyl, -O-C₁₋₆alkyl, and -S-C₁₋₆alkyl;
Het₁, Het₂, Het₃, Het₄, Het₅, Het₆, Het₇ and Het₈ are each independently a 5-or 6-membered monocyclic heterocycle having from 1 to 3 heteroatoms selected from O, N and S, wherein each of said Het₁, Het₂, Het₃, Het₄, Het₅, Het₆, Het₇ and Het₈ is optionally substituted with from 1 to 3 substituents selected from -C₁₋₆alkyl, -O-C₁₋₆alkyl, -S-C₁₋₆alkyl, and -NR₂₁R₂₂; wherein each of said -C₁₋₆alkyl is optionally and independently substituted with from 1 to 3-halo;
Z₁, Z₂, Z₃, Z₄ and Z₅ are each independently selected from C and N;
m and n are each independently 1, 2, 3, or 4.

In particular, X₁, and X₂ as used herein, represent biradicals, which taken together with the radicals to which they are attached form a macrocyclic pyrazolopyrimidine compound. Said biradicals may be present in either of both directions in the macrocyclic pyrazolopyrimidine, but are preferably present in the direction as described below:
Referring to formula I:
   X₁ is selected from the list comprising *-C₁₋₆alkyl-, *-O-C₁₋₆alkyl-, *-S-C₁₋₆alkyl-, *-(C=O)-, *-NR₃-(C=O)-, *-C₁₋₆alkyl-NR₃-, *-NR₃-, *-(C=O)-, *-NR₃-(C=O)-NR₃₇-, *-NR₃-C₁₋₆alkyl-, *-NR₃-SO₂-, *-NR₃-(C=O)-C₁₋₆alkyl-, *-(C=O)-NR₃-C₁₋₆alkyl-, *-O-C₁₋₆alkyl-O-C₁₋₆alkyl- and *-C₁₋₆alkyl-NR₃-C₁₋₆alkyl-; wherein said biradical is preferably attached to the aryl or heteroaryl moiety via *;
   X₂ is selected from the list comprising *-C₁₋₆alkyl-, *-O-C₁₋₆alkyl-, *-S-C₁₋₆alkyl-, *-(C=O)-, *-NR₂-(C=O)-, *-C₁₋₆alkyl-NR₂-, *-NR₂-, *-(C=O)-, *-NR₂-(C=O)-NR₃₈-, *-NR₂-C₁₋₆alkyl-, *-NR₂-SO₂-, *-NR₂(C=O)-C₁₋₆alkyl-, *-(C=O)-NR₂-C₁₋₆alkyl-, *-O-C₁₋₆alkyl-O-C₁₋₆alkyl- and *-C₁₋₆alkyl-NR₂-C₁₋₆alkyl-; wherein said biradical is preferably attached to the pyrazolopyrimidine moiety via *;

In a preferred embodiment, the present invention provides compounds of formula I or a stereoisomer, tautomer, racemic, salt, hydrate, N-oxide form, or solvate thereof, for use in the diagnosis, prevention and/or treatment of a LRRK2-kinase associated disease wherein
A₁ is C and A₂ is N
R₁ and R₇ are each independently selected from -H, -halo, -OH, -C₁₋₆alkyl, -O-C₁₋₆alkyl, -S-C₁₋₆alkyl, -NR₉R₁₀, -(C=O)-R₄, -SO₂-R₄, -CN, -NR₉-SO₂-R₄, -C₃-₆cycloalkyl, and -Het₆; wherein each of said -C₁₋₆alkyl is optionally and independently substituted with from 1 to 3 substituents selected from -halo, -OH, -NR₁₁R₁₂, -O-C₁₋₆alkyl, and -S-C₁₋₆alkyl;
R₂ is selected from -H, -halo, -OH, -C₁₋₆alkyl, -O-C₁₋₆alkyl, -S-C₁₋₆alkyl, -(C=O)-C₁₋₆alkyl,-(C=O)-O-C₁₋₆alkyl, -(C=O)-NR₂₇R₂₈, -Het₃, -(C=O)-Het₃, -SO₂-C₁₋₆alkyl, and -C₃₋₆cycloalkyl; wherein each of said -C₁₋₆alkyl is optionally and independently substituted with from 1 to 3 substituents selected from -halo, -OH, -O-C₁₋₆alkyl, -S-C₁₋₆alkyl, -Het₃, -Ar₂, and -NR₁₃R₁₄;
R₃ is selected from -H, -halo, -OH, -C₁₋₆alkyl, -O-C₁₋₆alkyl, -S-C₁₋₆alkyl, -(C=O)-C₁₋₆alkyl, -(C=O)-O-C₁₋₆alkyl, -Het₂, -C₃₋₆cycloalkyl -(C=O)-Het₂, -(C=O)-NR₂₉R₃₀, and -SO₂-C₁₋₆alkyl; wherein each of said -C₁₋₆alkyl is optionally and independently substituted with from 1 to 3 substituents selected from -halo, -OH, -O-C₁₋₆alkyl, -S-C₁₋₆alkyl, -NR₁₅R₁₆, -Het₂, and -Ar₃;
R₄ is independently selected from -halo, -OH, -C₁₋₆alkyl, -O-C₁₋₆alkyl, -S-C₁₋₆alkyl, -NR₁₇R₁₈, and -Het₄;
R₅ is selected from -H, -C₁₋₆alkyl, -C₃₋₆cycloalkyl; wherein each of said C₁₋₆alkyl or -C₃₋₆cycloalkyl is optionally and independently substituted with from 1 to 3 substituents selected from -halo, -OH, -OC₁₋₆alkyl, -SC₁₋₆alkyl, -Het₅, and -NR₃₁R₃₂;
R₆ is selected from -H, -OH, -halo, -C₁₋₆alkyl, -O-C₁₋₆alkyl, -S-C₁₋₆alkyl, -NR₃₃R₃₄, and -Het₈;
R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉,R₃₀, R₃₁, R₃₂, R₃₃, R₃₄, R₃₇ and R₃₈ are each independently selected from -H, =O, -C₁₋₆alkyl, and -Het₁; wherein each of said -C₁₋₆alkyl is optionally and independently substituted with from 1 to 3 substituents selected from -halo, -OH, -O-C₁₋₆alkyl, -S-C₁₋₆alkyl, -NR₃₅R₃₆,-Het₇, and -Ar₄;
R₃₅ and R₃₆ are each independently selected from -H, =O, and -C₁₋₆alkyl; wherein each of said-C₁₋₆alkyl is optionally and independently substituted with from 1 to 3 substituents selected from -halo, -OH, -O-C₁₋₆alkyl, and -S-C₁₋₆alkyl;
X₁ is selected from -C₁₋₆alkyl-, -O-C₁₋₆alkyl-, -S-C₁₋₆alkyl-, -(C=O)-, -NR₃-(C=O)-, -C₁₋₆alkyl-NR₃-, -NR₃-, -(C=O)-, -NR₃-(C=O)-NR₃₇-, -NR₃-C₁₋₆alkyl-, -NR₃-SO₂-, -NR₃-(C=O)-C₁₋₆alkyl-, -(C=O)-NR₃-C₁₋₆alkyl-, -O-C₁₋₆alkyl-O-C₁₋₆alkyl- and -C₁₋₆alkyl-NR₃-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl, and -NR₂₃R₂₄
X₂ is selected from -C₁₋₆alkyl-, -O-C₁₋₆alkyl-, -S-C₁₋₆alkyl-, -(C=O)-, -NR₂-(C=O)-, -C₁₋₆alkyl-NR₂-, -NR₂-, -(C=O)-, -NR₂-(C=O)-NR₃₈-, -NR₂-C₁₋₆alkyl-, -NR₂-SO₂-, -NR₂-(C=O)-C₁₋₆alkyl-, -(C=O)-NR₂-C₁₋₆alkyl-, -O-C₁₋₆alkyl-O-C₁₋₆alkyl- and -C₁₋₆alkyl-NR₂C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl and -NR₂₅R₂₆;
Y is selected from a direct bond, -CHR₆-, -O-, -S-, and -NR₅-;
Ar₂, Ar₃, and Ar₄ are each independently a 5- or 6-membered aromatic heterocycle optionally comprising 1 or 2 heteroatoms selected from O, N and S; wherein each of said Ar₂, Ar₃, and Ar₄ is optionally and independently substituted with from 1 to 3 substituents selected from -NR₁₉R₂₀, -C₁₋₆alkyl, -O-C₁₋₆alkyl, and -S-C₁₋₆alkyl;
Het₁, Het₂, Het₃, Het₄, Het₅, Het₆, Het₇ and Het₈ are each independently a 5-or 6-membered monocyclic heterocycle having from 1 to 3 heteroatoms selected from O, N and S, wherein each of said Het₁, Het₂, Het₃, Het₄, Het₅, Het₆, Het₇ and Het₈ is optionally substituted with from 1 to 3 substituents selected from -C₁₋₆alkyl, -O-C₁₋₆alkyl, -S-C₁₋₆alkyl, and -NR₂₁R₂₂; wherein each of said -C₁₋₆alkyl is optionally and independently substituted with from 1 to 3-halo;
Z₁, Z₂, Z₃, Z₄ and Z₅ are each independently selected from C and N;
m and n are each independently 1,2,3, or 4.

In yet another particular embodiment, the present invention provides a compound or a stereoisomer, tautomer, racemic, salt, hydrate, N-oxide form, or solvate thereof, for use in the diagnosis, prevention and/or treatment of a LRRK2-kinase associated disease, wherein
A₁ is N and A₂ is C
R₁ and R₇ are each independently selected from -H, -halo, -OH, -C₁₋₆alkyl, -O-C₁₋₆alkyl, -S-C₁₋₆alkyl, -NR₉R₁₀, -(C=O)-R₄, -SO₂-R₄, -CN, -NR₉-SO₂-R₄, -C₃₋₆cycloalkyl, and -Het₆; wherein each of said -C₁₋₆alkyl is optionally and independently substituted with from 1 to 3 substituents selected from -halo, -OH, -NR₁₁R₁₂, -O-C₁₋₆alkyl, and -S-C₁₋₆alkyl;
R₂ is selected from -H, -halo, -OH, -C₁₋₆alkyl, -O-C₁₋₆alkyl, -S-C₁₋₆alkyl, -(C=O)-C₁₋₆alkyl,-(C=O)-O-C₁₋₆alkyl, -(C=O)-NR₂₇R₂₈, -Het₃, -(C=O)-Het₃, -SO₂-C₁₋₆alkyl, and -C₃₋₆cycloalkyl;
   wherein each of said -C₁₋₆alkyl is optionally and independently substituted with from 1 to 3 substituents selected from -halo, -OH, -O-C₁₋₆alkyl, -S-C₁₋₆alkyl, -Het₃, -Ar₂, and -NR₁₃R₁₄;
R₃ is selected from -H, -halo, -OH, -C₁_₆alkyl, -O-C₁₋₆alkyl, -S-C₁₋₆alkyl, -(C=O)-C₁₋₆alkyl, -(C=O)-O-C₁₋₆alkyl, -Het₂, -C₃₋₆cycloalkyl -(C=O)-Het₂, -(C=O)-NR₂₉R₃₀, and -SO₂-C₁₋₆alkyl; wherein each of said -C₁₋₆alkyl is optionally and independently substituted with from 1 to 3 substituents selected from -halo, -OH, -O-C₁₋₆alkyl, -S-C₁₋₆alkyl, -NR₁₅R₁₆, -Het₂, and -Ar₃;
R₄ is independently selected from -halo, -OH, -C₁₋₆alkyl, -O-C₁₋₆alkyl, -S-C₁₋₆alkyl, -NR₁₇R₁₈, and -Het₄;
R₅ is selected from -H, -C₁₋₆alkyl, -C₃₋₆cycloalkyl; wherein each of said C₁₋₆alkyl or -C₃₋₆cycloalkyl is optionally and independently substituted with from 1 to 3 substituents selected from -halo, -OH, -OC₁₋₆alkyl, -SC₁₋₆alkyl, -Het₅, and -NR₃₁R₃₂;
R₆ is selected from -H, -OH, -halo, -C₁₋₆alkyl, -O-C₁₋₆alkyl, -S-C₁₋₆alkyl, -NR₃₃R₃₄, and -Het₈;
R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉,R₃₀, R₃₁, R₃₂, R₃₃, R₃₄, R₃₇ and R₃₈ are each independently selected from -H, =O, -C₁₋₆alkyl, and -Het₁; wherein each of said -C₁₋₆alkyl is optionally and independently substituted with from 1 to 3 substituents selected from -halo, -OH, -O-C₁₋₆alkyl, -S-C₁₋₆alkyl, -NR₃₅R₃₆, -Het₇, and -Ar₄;
R₃₅ and R₃₆ are each independently selected from -H, =O, and -C₁₋₆alkyl; wherein each of said-C₁₋₆alkyl is optionally and independently substituted with from 1 to 3 substituents selected from -halo, -OH, -O-C₁₋₆alkyl, and -S-C₁₋₆alkyl;
X₁ is selected from -C₁₋₆alkyl-, -O-C₁₋₆alkyl-, -S-C₁₋₆alkyl-, -(C=O)-, -NR₃-(C=O)-, -C₁₋₆alkyl-NR₃-, -NR₃-, -(C=O)-, -NR₃-(C=O)-NR₃₇-, -NR₃-C₁₋₆alkyl-, -NR₃-SO₂-, -NR₃-(C=O)-C₁₋₆alkyl-, -(C=O)-NR₃-C₁₋₆alkyl-, -O-C₁₋₆alkyl-O-C₁₋₆alkyl- and -C₁₋₆alkyl-NR₃-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl, and -NR₂₃R₂₄
X₂ is selected from -C₁₋₆alkyl-, -O-C₁₋₆alkyl-, -S-C₁₋₆alkyl-, -(C=O)-, -NR₂-(C=O)-, -C₁₋₆alkyl-NR₂-, -NR₂-, -(C=O)-, -NR₂-(C=O)-NR₃₈-, -NR₂-C₁₋₆alkyl-, -NR₂-SO₂-, -NR₂-(C=O)-C₁₋₆alkyl-, -(C=O)-NR₂-C₁₋₆alkyl-, -O-C₁₋₆alkyl-O-C₁₋₆alkyl- and -C₁₋₆alkyl-NR₂-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl and -NR₂₅R₂₆;
Y is selected from a direct bond, -CHR₆-, -O-, -S-, and -NR₅-;
Ar₂, Ar₃, and Ar₄ are each independently a 5- or 6-membered aromatic heterocycle optionally comprising 1 or 2 heteroatoms selected from O, N and S; wherein each of said Ar₂, Ar₃, and Ar₄ is optionally and independently substituted with from 1 to 3 substituents selected from -NR₁₉R₂₀, -C₁₋₆alkyl, -O-C₁₋₆alkyl, and -S-C₁₋₆alkyl;
Het₁, Het₂, Het₃, Het₄, Het₅, Het₆, Het₇ and Het₈ are each independently a 5-or 6-membered monocyclic heterocycle having from 1 to 3 heteroatoms selected from O, N and S, wherein each of said Het₁, Het₂, Het₃, Het₄, Het₅, Het₆, Het₇ and Het₈ is optionally substituted with from 1 to 3 substituents selected from -C₁₋₆alkyl, -O-C₁₋₆alkyl, -S-C₁₋₆alkyl, and -NR₂₁R₂₂;
wherein each of said -C₁₋₆alkyl is optionally and independently substituted with from 1 to 3 - halo;
Z₁, Z₂, Z₃, Z₄ and Z₅ are each independently selected from C and N;
m and n are each independently 1, 2, 3, or 4.

Preferably the present invention provides compounds of Formula (I), for use in the diagnosis, prevention and/or treatment of a LRRK2-kinase associated disease, wherein A₁ is N and A₂ is C.

Preferably, the compounds of Formula (I) of the invention are such that both R₁ and R₇ are -H.

Alternatively, the compounds of Formula (I) of the invention are such that R₁ is -Halo and R₇ is -H.

Preferably, when R₁ is -Halo, R₇ is -F.

For example, the present invention provides compounds of Formula (I) for use in the diagnosis, prevention and/or treatment of a LRRK2-kinase associated disease wherein
A₁ is N, A₂ is C;
R₁ and R₇ are both -H;
and one of Z₁, Z₂, Z₃, Z₄ and Z₅ is N and the other ones are each C.

For example, the present invention provides compounds of Formula (I) for use in the diagnosis, prevention and/or treatment of a LRRK2-kinase associated disease wherein
A₁ is N, A₂ is C;
R₁ and R₇ are both -H;
and Z₁, Z₂, Z₃, Z₄ and Z₅ are each C.

For example, the present invention provides compounds of Formula (I) for use in the diagnosis, prevention and/or treatment of a LRRK2-kinase associated disease wherein
A₁ is N, A₂ is C;
R₁ is -F and R₇ is -H;
and Z₁, Z₂, Z₃, Z₄ and Z₅ are each C.

For example, the present invention provides compounds of Formula (I) for use in the diagnosis, prevention and/or treatment of a LRRK2-kinase associated disease wherein
A₁ is N, A₂ is C;
R₁ is -F and R₇ is -H;
and one of Z₁, Z₂, Z₃, Z₄ and Z₅ is N and the other ones are each C.

For example, the present invention provides compounds of Formula (I) for use in the diagnosis, prevention and/or treatment of a LRRK2-kinase associated disease wherein
A₁ is C, A₂ is N;
and R₁ is -F and R₇ is -H.

Preferably, the compounds of Formula (I) for use in the diagnosis, prevention and/or treatment of a LRRK2-kinase associated disease of the invention are such that X₁ is -O-C₁₋₆alkyl or NR₃-Preferably, X₁ is -O-C₁₋₆alkyl. Alternatively, X₁ is NR₃-.

Preferably, the compounds of Formula (I) for use in the diagnosis, prevention and/or treatment of a LRRK2-kinase associated disease of the invention are such that X₂ is NR₂- or -O-C₁₋₆alkyl.

Preferably, X₂ is NR₂-. Alternatively, X₂ is -O-C₁₋₆alkyl, for example -O-CH₂-, -O-CH₂₋CH₂- or-O-CH₂-CH₂-.

Preferably, the compounds of Formula (I) for use in the diagnosis, prevention and/or treatment of a LRRK2-kinase associated disease of the invention are such that R₂ is -H.

Preferably, the compounds of Formula (I) for use in the diagnosis, prevention and/or treatment of a LRRK2-kinase associated disease of the invention are such that R₃ is -H.

Preferably, the compounds of Formula (I) for use in the diagnosis, prevention and/or treatment of a LRRK2-kinase associated disease of the invention are such that Y is -NR₅ or -O-.

Preferably, the compounds of Formula (I) for use in the diagnosis, prevention and/or treatment of a LRRK2-kinase associated disease of the invention are such that Y is -NR₅.

Preferably, the compounds of Formula (I) for use in the diagnosis, prevention and/or treatment of a LRRK2-kinase associated disease of the invention are such that R₅ is -H or -C₁₋₆alkyl, more preferably R₅ is -H.

Preferably, the compounds of Formula (I) for use in the diagnosis, prevention and/or treatment of a LRRK2-kinase associated disease of the invention are such that Y is -O-.

Preferably, the compounds of Formula (I) for use in the diagnosis, prevention and/or treatment of a LRRK2-kinase associated disease of the invention are such that Z₁, Z₂, Z₃, Z₄ and Z₅ are each C. Alternatively, one of Z₁, Z₂, Z₃, Z₄ and Z₅ is N and the other ones are each C.

Preferably the compounds of Formula (I) for use in the diagnosis, prevention and/or treatment of a LRRK2-kinase associated disease of the invention are such that m is 1. Alternatively, m is 2. Alternatively, m is 3. Alternatively, m is 4.

Preferably the compounds of Formula (I) for use in the diagnosis, prevention and/or treatment of a LRRK2-kinase associated disease of the invention are such that n is 1. More preferably, n is 2. Even more preferably, n is 3. Alternatively, n is 4.

For example, m is 1 or 2 and n is 2 or 3.

For example, the present invention provides compounds of Formula (I) for use in the diagnosis, prevention and/or treatment of a LRRK2-kinase associated disease wherein
- A₁: is N, A₂ is C;
- X₁: is -O-C₁₋₆alkyl or NR₃-; and
- X₂: is NR₂- or -O-C₁₋₆alkyl.

For example, the present invention provides compounds of Formula (I) for use in the diagnosis, prevention and/or treatment of a LRRK2-kinase associated disease wherein
- A₁: is N, A₂ is C;
- X₁: is -O-methyl; and
- X₂: is NR₂-.

For example, the present invention provides compounds of Formula (I) for use in the diagnosis, prevention and/or treatment of a LRRK2-kinase associated disease wherein
- A₁: is N, A₂ is C;
- X₁: is NR₅-; and
- X₂: is NR₂-.

For example, the present invention provides compounds of Formula (I) for use in the diagnosis, prevention and/or treatment of a LRRK2-kinase associated disease wherein
- A₁: is N, A₂ is C;
- X₁: is -O-methyl or NR₃-;
- X₂: is NR₂- or -O-methyl; and
- n and m: are each independently 1, 2 or 3.

In particular the present invention provides compounds, for use in the diagnosis, prevention and/or treatment of a LRRK2-kinase associated disease, wherein said compounds are selected from:

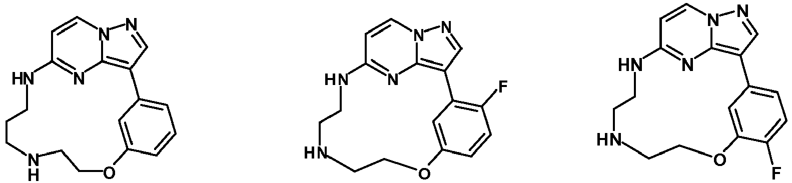

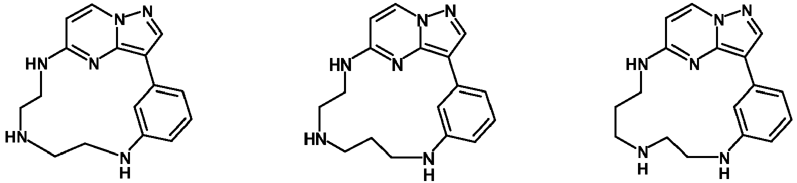

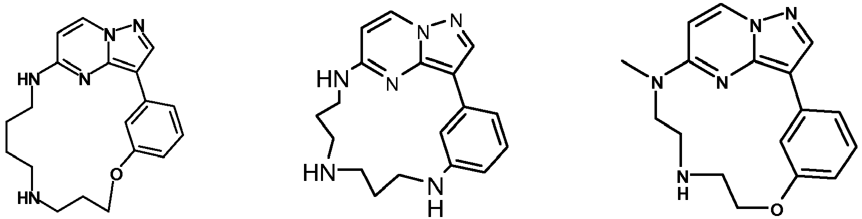

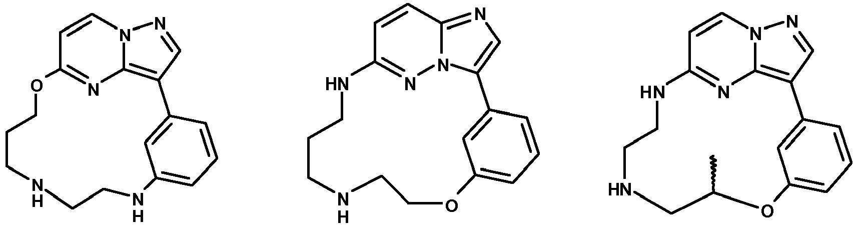

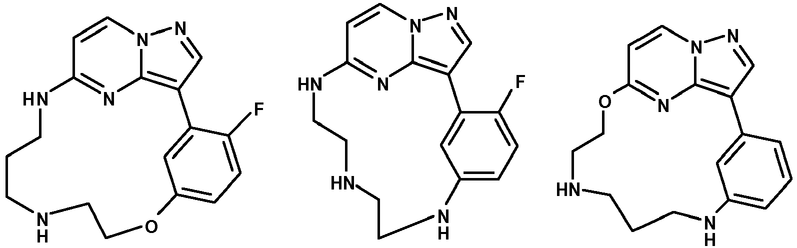

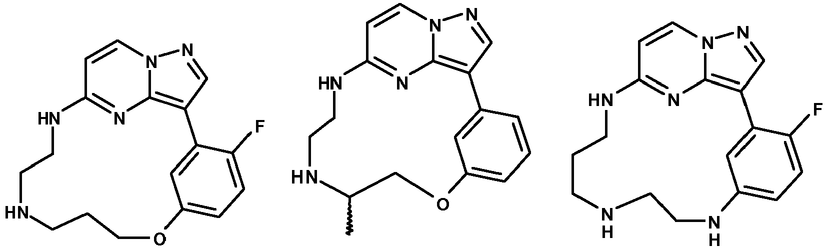

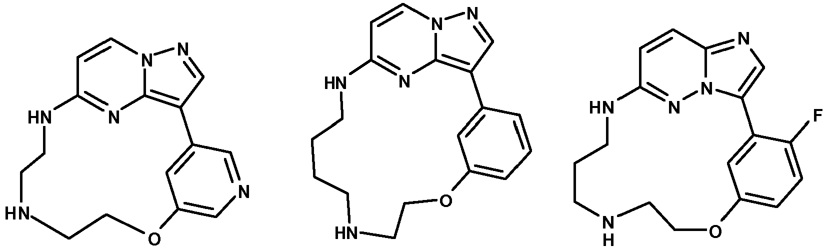

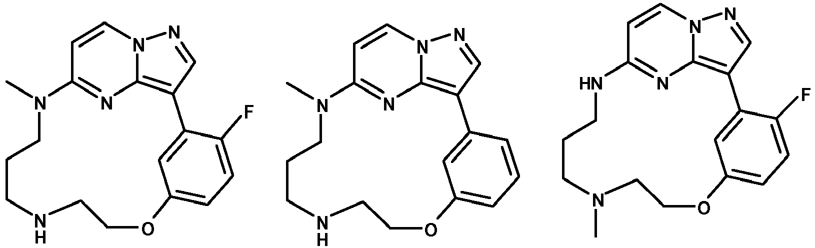

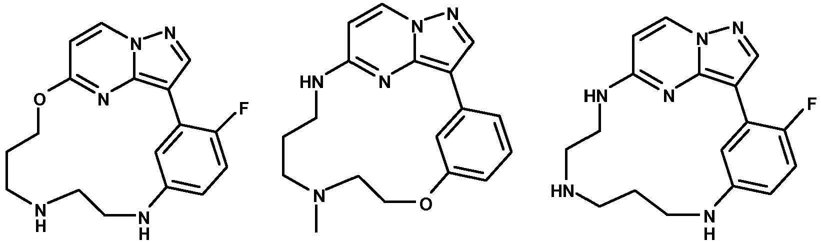

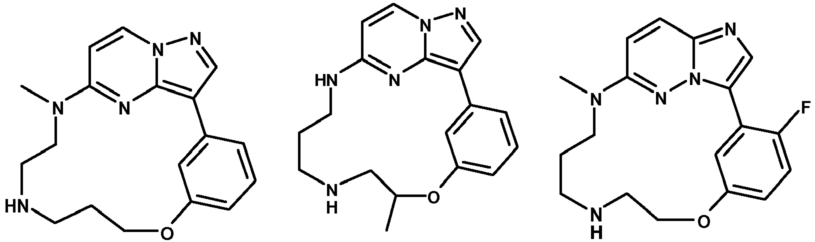

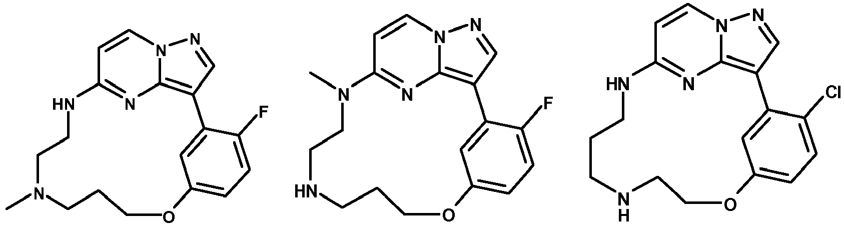

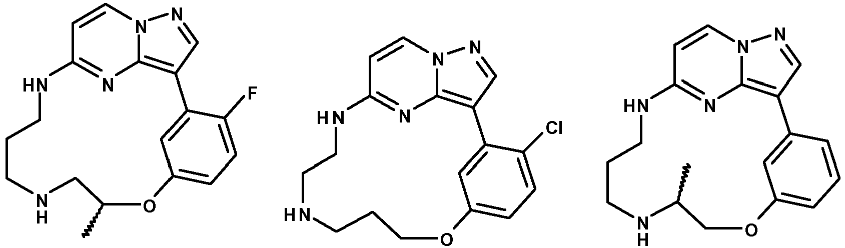

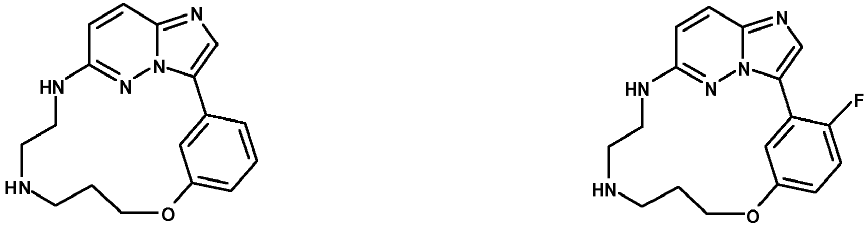

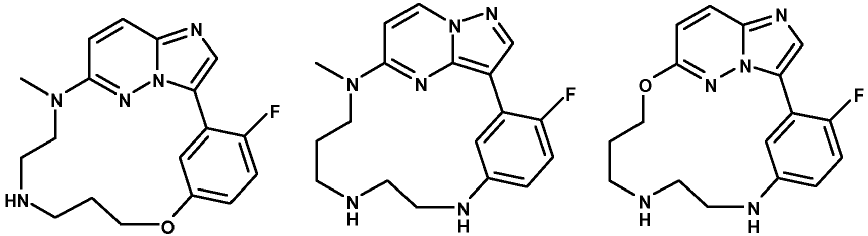

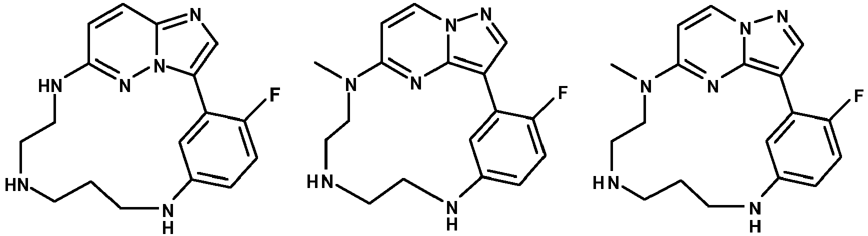

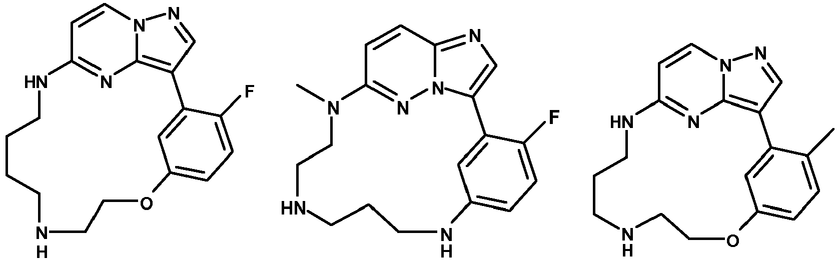

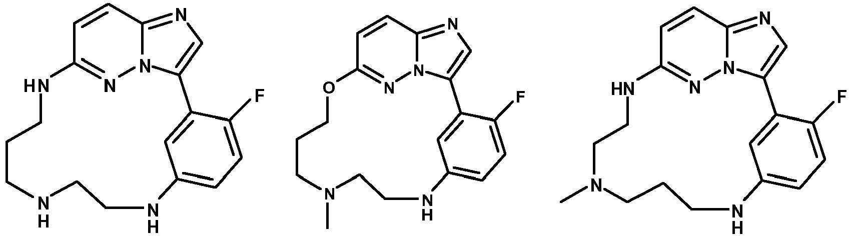

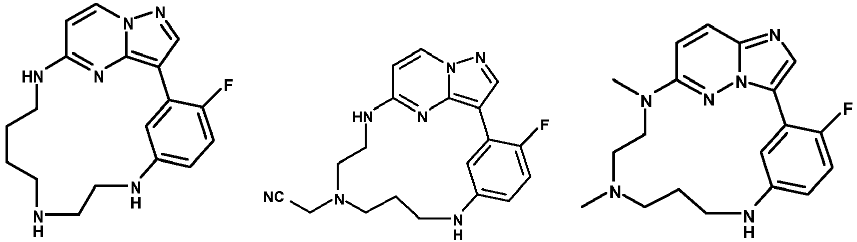

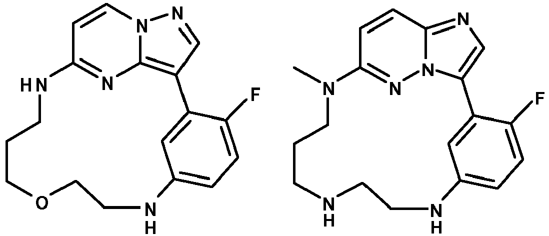

In particular in the compounds according to this invention, the pyrazolopyrimidine moiety is linked to the aryl or heteroaryl moiety at position Z₄ or Z₅, in accordance with the numbering as provided in Formula I. Furthermore, the R₁ of the compounds according to this invention is preferably linked to the aryl or heteroaryl moiety at position Z₁, Z₂ or Z₃, in accordance with the numbering as provided in Formula I.

In a particular embodiment, the present invention provides a compound of Formula (IIc) or a stereoisomer, tautomer, racemic, salt, hydrate, N-oxide form, or solvate thereof, 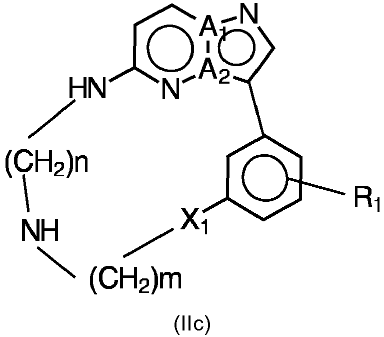 wherein
- A₁ and A₂: are selected from C and N; wherein when A₁ is C, then A₂ is N; and wherein when A₂ is C, then A₁ is N;
- R₁: is selected from the list comprising -H, -F, -CH₃, and -CN
- X₁: is selected from the list comprising -NH- and -O-
- m and n: are each independently 1, 2, 3, or 4
for use in the prevention and/or treatment of a LRRK2-kinase associated disease.

In particular the present invention provides compounds, for use in the diagnosis, prevention and/or treatment of a LRRK2-kinase associated disease, wherein said compounds are selected from:

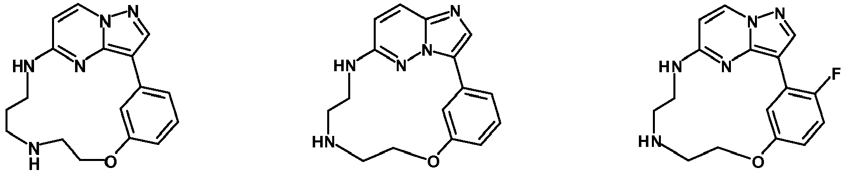

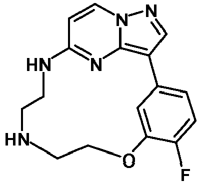

In a particular embodiment, the LRRK2-kinase associated disease is a neurological disorder, in particular selected from the list comprising Parkinson's disease or Alzheimer's disease.

The present invention further provides a pharmaceutical composition for use in the diagnosis, prevention and/or treatment of a LRRK2-kinase associated disease comprising a compound according to the present invention.

The present disclosure also provides the use of a compound, or a composition according to this invention, suitable for inhibiting the activity of a kinase; in particular a LRRK2 kinase.

Furthermore, the present disclosure provides the use of a compound or a composition according to this invention, for the diagnosis, prevention and/or treatment of a LRRK2-kinase associated disease.

Moreover, the present disclosure provides a method for the prevention and/or treatment of a LRRK2-kinase associated disease; said method comprising administering to a subject in need thereof a compound or a composition according to this invention.

Finally, the present invention provides new compounds of Formula (IIIc) or a stereoisomer, tautomer, racemic, salt, hydrate, N-oxide form, or solvate thereof, according to the general formula (IIIc) 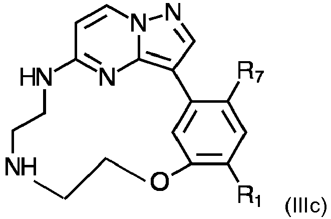 wherein
- R₁: is -H and R₇ is -F; or
- R₇: is -H and R₁ is -F.

In another embodiment, the present invention provides a compound of formula (IIIc), said compound being

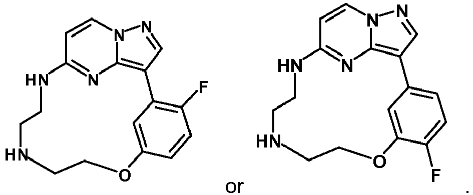

As already mentioned hereinbefore, in another aspect the present invention provides a compound of formula (IVc) or a stereoisomer, tautomer, racemic, salt, hydrate, N-oxide form, or solvate thereof: 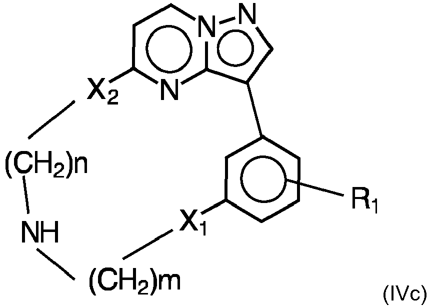 wherein
- R₁: is selected from the list comprising -H, -F, -CH₃, and -CN
- X₁ and X₂: are each independently selected from the list comprising -NH- and -O-
- m and n: are each independently 1, 2, 3, or 4.
with the proviso that said compound is not

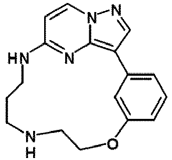

Preferably, R₁ is -H or -F.

Preferably R₁ is -H.

Alternatively, R₁ is -F, -CH₃ or -CN. Preferably, R₁ is F.

Preferably m and n are each independently 2, 3 or 4.

Preferably n is 2 and m is 3. Alternatively, n is 3 and m is 2. Alternatively, n is 4 and m is 3. Alternatively, n is 2 and m is 2.

In a particular embodiment, the present invention provides a compound of formula (IVc₁) or a stereoisomer, tautomer, racemic, salt, hydrate, N-oxide form, or solvate thereof, wherein R₁ is -F, -CH₃ or -CN , and X₁, X₂, n and m are as defined above

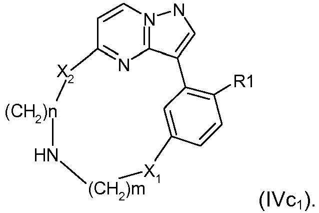

In a particular embodiment, the present invention provides a compound of formula (IVc₂) or a stereoisomer, tautomer, racemic, salt, hydrate, N-oxide form, or solvate thereof, wherein R₁ is -F, -CH₃ or -CN , and X₁, X₂, n and m are as defined above

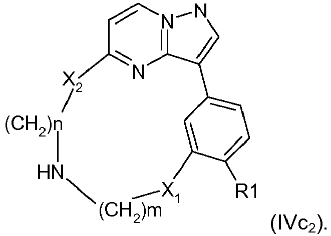

In another embodiment, the present invention provides a compound of formula (IVc₃) or a stereoisomer, tautomer, racemic, salt, hydrate, N-oxide form, or solvate thereof, wherein R₁ is -F, -CH₃ or -CN , and X₁, X₂, n and m are as defined above

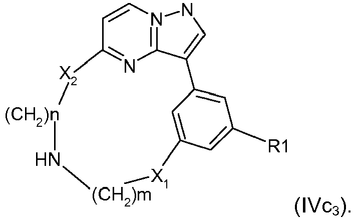

In another embodiment, the present invention provides a compound of formula (IVc) or a stereoisomer, tautomer, racemic, salt, hydrate, N-oxide form, or solvate thereof, wherein X₂ is -NH-.

In another embodiment, the present invention provides a compound of formula (IVc) or a stereoisomer, tautomer, racemic, salt, hydrate, N-oxide form, or solvate thereof, wherein X₂ is -O-.

In another embodiment, the present invention provides a compound of formula (IVc) or a stereoisomer, tautomer, racemic, salt, hydrate, N-oxide form, or solvate thereof, wherein X₁ is -NH-.

In another embodiment, the present invention provides a compound of formula (IVc) or a stereoisomer, tautomer, racemic, salt, hydrate, N-oxide form, or solvate thereof, wherein X₁ is -O-.

Preferably, X₁ and X₂ are -NH-.

In a particular embodiment, the present invention provides a compound of formula (IVc) or a stereoisomer, tautomer, racemic, salt, hydrate, N-oxide form, or solvate thereof, selected from the list comprising:

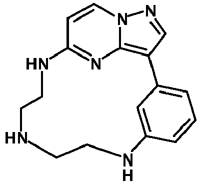

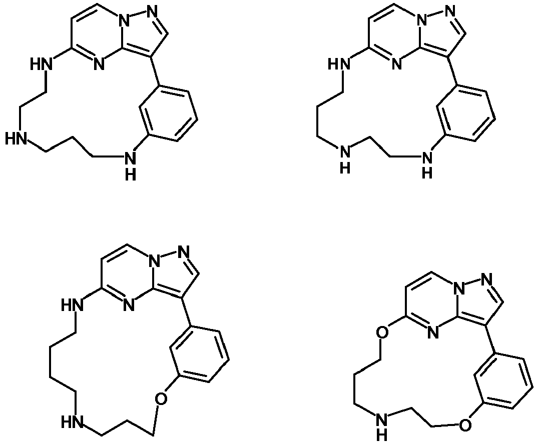

As already mentioned hereinbefore, in another aspect the present invention provides a compound of formula (Vc) 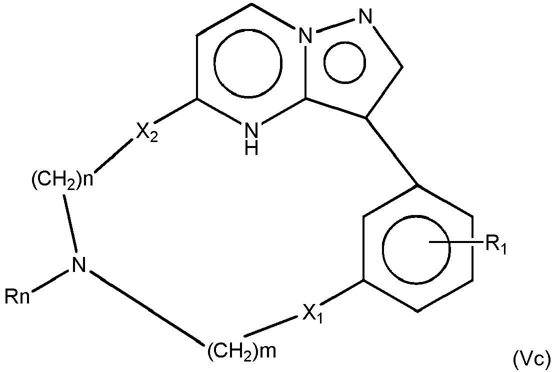 wherein
- R₁: is selected from the list comprising -H, and -F,
- X₁ and X₂: are each independently selected from the list comprising -NRx- and -O-,
- Rx: is H or a methyl group,
- m and n: are each independently 1, 2, 3, or 4,
- Rn: is H or a methyl group,
with the proviso that said compound is not

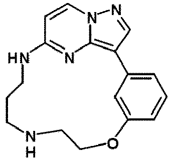

Preferably, R₁ is F.

Preferably m and n are each independently 2, 3 or 4.

Preferably n is 2 and m is 3. Alternatively, n is 3 and m is 2. Alternatively, n is 4 and m is 3. Alternatively, n is 2 and m is 2.

In a particular embodiment, the present invention provides a compound of formula (Vc₁), wherein R₁ is -F, and X₁, X₂, n and m are as defined above

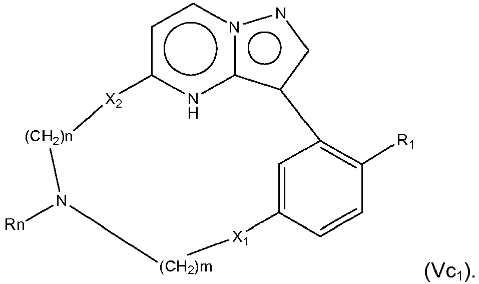

In a particular embodiment, the present invention provides a compound of formula (Vc₂), wherein R₁ is -F, and X₁, X₂, n and m are as defined above

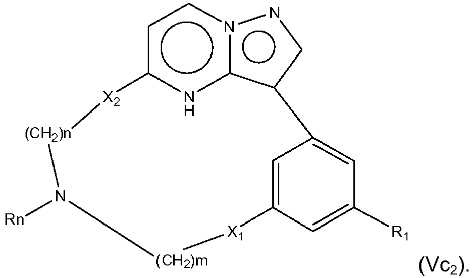

In another embodiment, the present invention provides a compound of formula (IV₃) or a stereoisomer, tautomer, racemic, salt, hydrate, N-oxide form, or solvate thereof, wherein R₁ is -F, -CH₃ or -CN , and X₁, X₂, n and m are as defined above

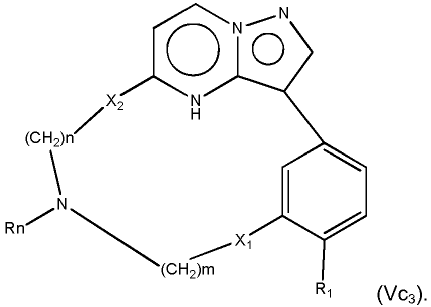

In another embodiment, the present invention provides a compound of formula (Vc), wherein X₂ is -NRx-.

In another embodiment, the present invention provides a compound of formula (Vc), wherein X₂ its-0-.

In another embodiment, the present invention provides a compound of formula (Vc), wherein X₁ is -NRx-.

In another embodiment, the present invention provides a compound of formula (Vc), wherein X₁ is -0-.

Preferably, X₁ and X₂ are -NRx-.

The compounds of the present invention can be prepared according to the reaction schemes provided in the examples hereinafter, but those skilled in the art will appreciate that these are only illustrative for the invention and that the compounds of this invention can be prepared by any of several standard synthetic processes commonly used by those skilled in the art of organic chemistry.

Compounds of formula (I), (IIc), (IIIc), (IVc) and (Vc), a stereoisomer, tautomer, racemic, metabolite, pro- or predrug, salt, hydrate, N-oxide form, or solvate thereof, are inhibitors of LRRK2 kinase activity and are thus believed to be of potential use in the prevention and/or treatment of neurological disorders including Parkinson's disease, Alzheimer's disease, dementia (including Lewy body dementia and vascular dementia), age related memory dysfunction, mild cognitive impairment, argyrophilic grain disease, Pick's disease, corticobasal degeneration, progressive supranuclear palsy, inherited frontotemporal dementia and parkinsonism linked to chromosome 17 (FTDP-17), withdrawal symptoms/relapse associated with drug addiction, L-Dopa induced dyskinesia, and renal, breast, lung, prostate cancers as well as acute myelogenous leukemia (AML).

In the context of the present invention, treatment of Parkinson's disease refers to the treatment of idiopathic Parkinson's disease and familial Parkinson's disease. In one embodiment, familial Parkinson's disease includes patients expressing LRRK2 kinase bearing the G2019S mutation or the R1441G mutation. Treatment of Parkinson's disease may be symptomatic or may be disease modifying. In one embodiment, treatment of Parkinson's disease refers to symptomatic treatment. Compounds of the present invention may also be useful in treating patients identified as susceptible to progression to severe Parkinsonism by means of one of more subtle features associated with disease progression such as family history, olfaction deficits, constipation, cognitive defects, gait or biological indicators of disease progression gained from molecular, biochemical, immunological or Imaging technologies. In this context, treatment may be symptomatic or disease modifying.

In the context of the present invention, treatment of Alzheimer's disease refers to the treatment of idiopathic Alzheimer's disease and familial Alzheimer's disease. Treatment of Alzheimer's disease may be symptomatic or may be disease modifying. In one embodiment, treatment of Alzheimer's disease refers to symptomatic treatment.

Similarly, treatment of dementia (including Lewy body dementia and vascular dementia), age related memory dysfunction, mild cognitive impairment argyrophilic grain disease, Pick's disease, corticobasal degeneration, progressive supranuclear palsy, inherited frontotemporal dementia and parkinsonism linked to chromosome 17 (FTDP- 7) and renal, breast, lung, prostate cancers as well as acute myelogenous leukemia (AML) may be symptomatic or disease modifying. In one embodiment, treatment of dementia (including Lewy body dementia and vascular dementia), age related memory dysfunction, mild cognitive impairment, argyrophilic grain disease, Pick's disease, corticobasal degeneration, progressive supranuclear palsy, inherited frontotemporal dementia and parkinsonism linked to chromosome 17 (FTDP-17), and renal, breast, lung, prostate cancers as well as acute myelogenous leukemia (A L) refers to symptomatic treatment.

In the context of the present invention, treatment of withdrawal symptoms/relapse associated with drug addiction and L-Dopa induced dyskinesia refers to symptomatic treatment. Accordingy, the present disclosure further provides a method for the prevention and/or treatment of neurological disorders such as but not limited to Parkinson's disease and Alzheimer's disease, said method comprising administering to a subject in need thereof a therapeutic effective amount of a compound or a composition as defined herein. The methods of the present disclosure can be utilized in a variety of settings, including, for example, in selecting the optimal treatment course for a patient, in predicting the likelihood of success when treating an individual patient with a particular treatment regimen, in assessing disease progression, in monitoring treatment efficacy, in determining prognosis for individual patients and in assessing predisposition of an individual to benefit from a particular therapy.

In the invention, particular preference is given to compounds of Formula I or any subgroup thereof that in the inhibition assay for LRRK2 described below inhibit kinase activity with an IC₅₀ value of less than 10 µM, preferably less than 1 µM, most preferably less than 100 nM.

Said inhibition may be effected *in vitro* and/or *in vivo,* and when effected *in vivo*, is preferably effected in a selective manner, as defined above.

The term "LRRK2 kinase-mediated condition" or "disease", as used herein, means any disease or other deleterious condition in which the LRKK2 kinase is known to play a role. The term "LRRK2 kinase-mediated condition" or "disease" also means those diseases or conditions that are alleviated by treatment with a LRRK2 kinase inhibitor. Accordingly, another embodiment of the present disclosure relates to treating or lessening the severity of one or more diseases in which the LRRK2 kinase is known to play a role.

For pharmaceutical use, the compounds of the invention may be used as a free acid or base, and/or in the form of a pharmaceutically acceptable acid-addition and/or base-addition salt (e.g. obtained with non-toxic organic or inorganic acid or base), in the form of a hydrate, solvate and/or complex, and/or in the form or a pro-drug or pre-drug, such as an ester. As used herein and unless otherwise stated, the term "solvate" includes any combination which may be formed by a compound of this invention with a suitable inorganic solvent (e.g. hydrates) or organic solvent, such as but not limited to alcohols, ketones, esters and the like. Such salts, hydrates, solvates, etc. and the preparation thereof will be clear to the skilled person; reference is for instance made to the salts, hydrates, solvates, etc. described in US-A-6,372,778, US-A-6,369,086, US-A-6,369,087 and US-A-6,372,733.

The pharmaceutically acceptable salts of the compounds according to the invention, i.e. in the form of water-, oil-soluble, or dispersible products, include the conventional non-toxic salts or the quaternary ammonium salts which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalene-sulfonate, nicotinate, oxalate, palmoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. In addition, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl-bromides and others. Other pharmaceutically acceptable salts include the sulfate salt ethanolate and sulfate salts.

Generally, for pharmaceutical use, the compounds of the inventions may be formulated as a pharmaceutical preparation or pharmaceutical composition comprising at least one compound of the invention and at least one pharmaceutically acceptable carrier, diluent or excipient and/or adjuvant, and optionally one or more further pharmaceutically active compounds.

By means of non-limiting examples, such a formulation may be in a form suitable for oral administration, for parenteral administration (such as by intravenous, intramuscular or subcutaneous injection or intravenous infusion), for administration by inhalation, by a skin patch, by an implant, by a suppository, etc.. Such suitable administration forms - which may be solid, semi-solid or liquid, depending on the manner of administration - as well as methods and carriers, diluents and excipients for use in the preparation thereof, will be clear to the skilled person; reference is again made to for instance US-A-6,372,778, US-A-6,369,086, US-A-6,369,087 and US-A-6,372,733, as well as to the standard handbooks, such as the latest edition of Remington's Pharmaceutical Sciences.

Some preferred, but non-limiting examples of such preparations include tablets, pills, powders, lozenges, sachets, cachets, elixirs, suspensions, emulsions, solutions, syrups, aerosols, ointments, creams, lotions, soft and hard gelatin capsules, suppositories, eye drops, sterile injectable solutions and sterile packaged powders (which are usually reconstituted prior to use) for administration as a bolus and/or for continuous administration, which may be formulated with carriers, excipients, and diluents that are suitable per se for such formulations, such as lactose, dextrose, sucrose, sorbitol, mannitol, starches, gum acacia, calcium phosphate, alginates, tragacanth, gelatin, calcium silicate, microcrystalline cellulose, polyvinylpyrrolidone, polyethylene glycol, cellulose, (sterile) water, methylcellulose, methyl- and propylhydroxybenzoates, talc, magnesium stearate, edible oils, vegetable oils and mineral oils or suitable mixtures thereof. The formulations can optionally contain other pharmaceutically active substances (which may or may not lead to a synergistic effect with the compounds of the invention) and other substances that are commonly used in pharmaceutical formulations, such as lubricating agents, wetting agents, emulsifying and suspending agents, dispersing agents, desintegrants, bulking agents, fillers, preserving agents, sweetening agents, flavoring agents, flow regulators, release agents, etc.. The compositions may also be formulated so as to provide rapid, sustained or delayed release of the active compound(s) contained therein, for example using liposomes or hydrophilic polymeric matrices based on natural gels or synthetic polymers. In order to enhance the solubility and/or the stability of the compounds of a pharmaceutical composition according to the invention, it can be advantageous to employ α-, β- or γ-cyclodextrins or their derivatives. An interesting way of formulating the compounds in combination with a cyclodextrin or a derivative thereof has been described in EP-A-721,331. In particular, the present invention encompasses a pharmaceutical composition comprising an effective amount of a compound according to the invention with a pharmaceutically acceptable cyclodextrin.

In addition, co-solvents such as alcohols may improve the solubility and/or the stability of the compounds. In the preparation of aqueous compositions, addition of salts of the compounds of the invention can be more suitable due to their increased water solubility.

For local administration, the compounds may advantageously be used in the form of a spray, ointment or transdermal patch or another suitable form for topical, transdermal and/or intradermal administration.

More in particular, the compositions may be formulated in a pharmaceutical formulation comprising a therapeutically effective amount of particles consisting of a solid dispersion of the compounds of the invention and one or more pharmaceutically acceptable water-soluble polymers.

The term "a solid dispersion" defines a system in a solid state (as opposed to a liquid or gaseous state) comprising at least two components, wherein one component is dispersed more or less evenly throughout the other component or components. When said dispersion of the components is such that the system is chemically and physically uniform or homogenous throughout or consists of one phase as defined in thermodynamics, such a solid dispersion is referred to as "a solid solution". Solid solutions are preferred physical systems because the components therein are usually readily bioavailable to the organisms to which they are administered.

It may further be convenient to formulate the compounds in the form of nanoparticles which have a surface modifier adsorbed on the surface thereof in an amount sufficient to maintain an effective average particle size of less than 1000 nm. Suitable surface modifiers can preferably be selected from known organic and inorganic pharmaceutical excipients. Such excipients include various polymers, low molecular weight oligomers, natural products and surfactants. Preferred surface modifiers include nonionic and anionic surfactants.

Yet another interesting way of formulating the compounds according to the invention involves a pharmaceutical composition whereby the compounds are incorporated in hydrophilic polymers and applying this mixture as a coat film over many small beads, thus yielding a composition with good bio-availability which can conveniently be manufactured and which is suitable for preparing pharmaceutical dosage forms for oral administration. Materials suitable for use as cores in the beads are manifold, provided that said materials are pharmaceutically acceptable and have appropriate dimensions and firmness. Examples of such materials are polymers, inorganic substances, organic substances, and saccharides and derivatives thereof.

The preparations may be prepared in a manner known per se, which usually involves mixing at least one compound according to the invention with the one or more pharmaceutically acceptable carriers, and, if desired, in combination with other pharmaceutical active compounds, when necessary under aseptic conditions. Reference is again made to US-A-6,372,778, US-A-6,369,086, US-A-6,369,087 and US-A-6,372,733 and the further prior art mentioned above, as well as to the standard handbooks, such as the latest edition of Remington's Pharmaceutical Sciences.

The pharmaceutical preparations of the invention are preferably in a unit dosage form, and may be suitably packaged, for example in a box, blister, vial, bottle, sachet, ampoule or in any other suitable single-dose or multi-dose holder or container (which may be properly labeled); optionally with one or more leaflets containing product information and/or instructions for use. Generally, such unit dosages will contain between 1 and 1000 mg, and usually between 5 and 500 mg, of the at least one compound of the invention, e.g. about 10, 25, 50, 100, 200, 300 or 400 mg per unit dosage.

The compounds can be administered by a variety of routes including the oral, rectal, ocular, transdermal, subcutaneous, intravenous, intramuscular or intranasal routes, depending mainly on the specific preparation used and the condition to be treated or prevented, and with oral and intravenous administration usually being preferred. The at least one compound of the invention will generally be administered in an "effective amount", by which is meant any amount of a compound of Formula or any subgroup thereof that, upon suitable administration, is sufficient to achieve the desired therapeutic or prophylactic effect in the individual to which it is administered. Usually, depending on the condition to be prevented or treated and the route of administration, such an effective amount will usually be between 0.01 to 1000 mg per kilogram body weight day of the patient per day, more often between 0.1 and 500 mg, such as between 1 and 250 mg, for example about 5, 10, 20, 50, 100, 150, 200 or 250 mg, per kilogram body weight day of the patient per day, which may be administered as a single daily dose, divided over one or more daily doses, or essentially continuously, e.g. using a drip infusion. The amount(s) to be administered, the route of administration and the further treatment regimen may be determined by the treating clinician, depending on factors such as the age, gender and general condition of the patient and the nature and severity of the disease/symptoms to be treated. Reference is again made to US-A-6,372,778,US-A-6,369,086, US-A-6,369,087 and US-A-6,372,733 and the further prior art mentioned above, as well as to the standard handbooks, such as the latest edition of Remington's Pharmaceutical Sciences.

In accordance with the method of the present invention, said pharmaceutical composition can be administered separately at different times during the course of therapy or concurrently in divided or single combination forms. The present invention is therefore to be understood as embracing all such regimes of simultaneous or alternating treatment and the term "administering" is to be interpreted accordingly.

For an oral administration form, the compositions of the present invention can be mixed with suitable additives, such as excipients, stabilizers, or inert diluents, and brought by means of the customary methods into the suitable administration forms, such as tablets, coated tablets, hard capsules, aqueous, alcoholic, or oily solutions. Examples of suitable inert carriers are gum arabic, magnesia, magnesium carbonate, potassium phosphate, lactose, glucose, or starch, in particular, corn starch. In this case, the preparation can be carried out both as dry and as moist granules. Suitable oily excipients or solvents are vegetable or animal oils, such as sunflower oil or cod liver oil. Suitable solvents for aqueous or alcoholic solutions are water, ethanol, sugar solutions, or mixtures thereof. Polyethylene glycols and polypropylene glycols are also useful as further auxiliaries for other administration forms. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions may be prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. Suitable pharmaceutical formulations for administration in the form of aerosols or sprays are, for example, solutions, suspensions or emulsions of the compounds of the invention or their physiologically tolerable salts in a pharmaceutically acceptable solvent, such as ethanol or water, or a mixture of such solvents. If required, the formulation can also additionally contain other pharmaceutical auxiliaries such as surfactants, emulsifiers and stabilizers as well as a propellant.

For subcutaneous administration, the compound according to the invention, if desired with the substances customary therefore such as solubilizers, emulsifiers or further auxiliaries are brought into solution, suspension, or emulsion. The compounds of the invention can also be lyophilized and the lyophilizates obtained used, for example, for the production of injection or infusion preparations. Suitable solvents are, for example, water, physiological saline solution or alcohols, e.g. ethanol, propanol, glycerol, in addition also sugar solutions such as glucose or mannitol solutions, or alternatively mixtures of the various solvents mentioned. The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these formulations may be prepared by mixing the compounds according to the invention with a suitable non-irritating excipient, such as cocoa butter, synthetic glyceride esters or polyethylene glycols, which are solid at ordinary temperatures, but liquefy and/or dissolve in the rectal cavity to release the drug.

In preferred embodiments, the compounds and compositions of the invention are used orally or parenterally.

The invention will now be illustrated by means of the following synthetic and biological examples, which do not limit the scope of the invention in any way.

### EXAMPLES

### A. Compound synthesis and physicochemical properties

The compounds of this invention can be prepared by any of several standard synthetic processes commonly used by those skilled in the art of organic chemistry. The compounds are generally prepared from starting materials which are either commercially available or prepared by standard means obvious to those skilled in the art.

For some compounds that were purified by reversed phase high-performance liquis chromatography (HPLC) the used method is described below (indicated in the compound procedure with HPLC method A. When necessary, these methods can be slightly adjusted by a person skilled in the art to obtain a more optimal result for the separation.

### HPLC method A

The crude product was purified by reverse phase HPLC, using a Gilson semi-preparative HPLC system operated by Gilson UNIPOINT software.

The purification was carried out on a Phenomenex Luna column (100 mm long x 21.2 mm i.d.; 5µm particles) at room temperature, with a constant flow rate of 20.0 mL/min. A gradient elution was performed from 32% (25 mM NH4HCO3 aqueous solution) / 68% (Acetonitrile-Methanol 1:1) to 4% (25 mM NH4HCO3 aqueous solution) / 96% (Acetonitrile-Methanol 1:1) in 20 minutes. The UV detector was set to 226nm, which corresponds to the wavelenght of maximum absorbance observed for the compound.

### General schemes:

In general the compounds of formula (I) can be prepared as shown in scheme 1 below wherein a pyrazolo[1,5-a]pyrimidine or a imidazo[2,1-f]pyridazine of formula (II) is converted by reaction with a compound of formula (III) into a compound of formula (IV), which is then reacted with a (hetero-)aryl of formula (V) to form a compound of formula (VI). The compound of formula (VI) can then be optionally deprotected if desired before cyclisation to form a compound of formula (VII). The compound of formula (VII) can be optionally converted into a compound of general formula (I).

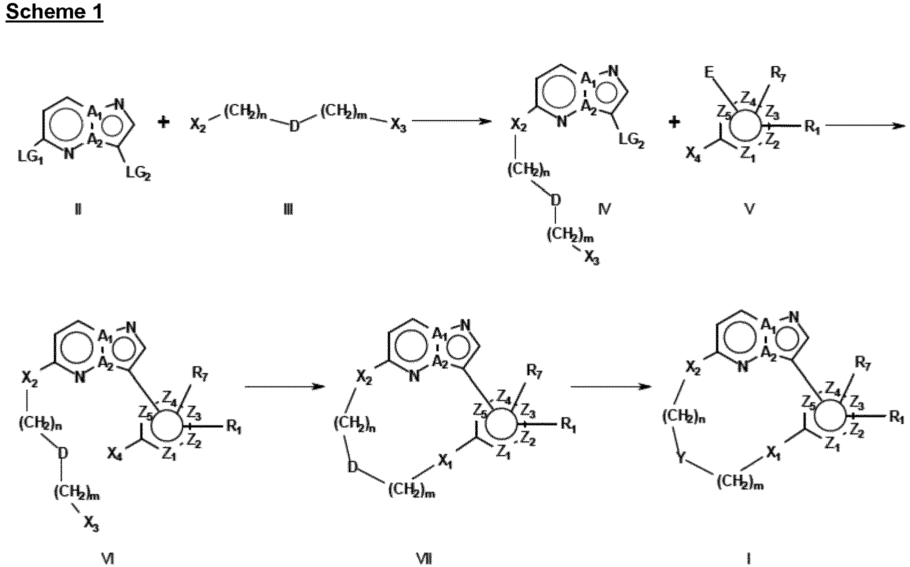

In the above scheme:
LG₁ and LG₂ each independently represent suitable leaving or functional groups;
X₃ and X₄ together with the functional moiety to which they are attached represent an unprotected or a protected functional group which upon reaction (after deprotection) produce together X₁ as defined in formula I;
E represents a suitable functional group that can be used to form a direct bond between the (hetero-)aryl group and the scaffold.
D represents a functional group such as Y or a protected functional group, which upon further reaction and/or deprotection produces a functional group such as Y as defined in formula I;
In the above reaction of the compound of formula (II) with the compound of formula (III) the leaving groups LG₁ and LG₂ are advantageously a halo group such as a chlorine or a bromine group. The reaction can be affected by a substitution for example by treating the compound of formula (II) with the compound of formula (III) in an organic solvent such as acetonitrile with an appropriate base such as for example diisopropylethylamine at an elevated temperature for example under reflux.

Compounds of formula (III) can be obtained through various selective protection and deprotection steps. The protection reactions can be effected using for example isoindoline-1,3-dione in a solvent such as toluene at an elevated temperature for example reflux or it can be effected by using for example benzaldehyde in the presence of a reducing agent for example sodium triacetoxyborohydride in a solvent such as 1,2-dichloroethane at room temperature or it can be effected using for example tert-butyldimethylsilyl chloride and triethylamine in a solvent such as N,N-dimethylformamide at room temperature. The deprotection reaction can be effected in a conventional manner using for example hydrazine in a solvent such as ethanol at an elevated temperature for example under reflux.

The compound of formula (IV) can optionally be protected with a suitable protecting group such as a tert-butyloxycarbonylamino group in a conventional manner for example by treatment with tert-butoxycarbonyl anhydride in basic conditions using for example triethylamine and 4-(dimethylamino)pyridine in a solvent such as tetrahydrofurane at an elevated temperature such as under reflux.

The reaction of the resulting compound (IV) with a (hetero-)aryl compound of formula (V) is advantageously effected through the coupling of a boronic acid E or boronic ester E derivative of the (hetero-)aryl compound under Suzuki conditions using for example tetrakis(triphenylphosphine)palladium(0), 2-dicyclohexylphosphino-2',4',6'-triisopropylbiphenyl (Xphos) and potassium phosphate tribasic in a solvent mixture such as 1,4-dioxane/water at an elevated temperature for example under reflux.

The resulting compound of formula (VI) can optionally be treated to remove any desired protecting groups for example silyl ether groups such as tert-butyldimethylsilyl groups can be converted to the parent free hydroxy group. Such deprotection can be effected in a conventional manner for example using tetrabutylammonium fluoride in tetrahydrofuran at room temperature. The resulting compound of formula (VI) can also optionally be treated to remove any desired protecting groups for example benzyl groups can be removed in a conventional manner for example using hydrogen gas and palladium on activated charcoal (10%) in a solvent such as methanol at a temperature such as room temperature. The compound of formula (VI) can optionally be treated to remove any desired protecting groups for example tert-butyloxycarbonylamino groups can be converted to the parent free amino group. Such deprotection can be effected in a conventional manner for example by treatment under acidic conditions for example using a 4N acetyl chloride solution in a solvent such as methanol at for example room temperature.

The cyclisation of the compound of formula (VI) can be effected for example under Mitsunobu conditions using for example diisopropyl azodicarboxylate and triphenylphosphine in a solvent mixture such as 2-methyl-1,4-dioxane and toluene at an elevated temperature such as 90°C.

The resulting compound of formula (VII) can optionally be treated to remove any desired protecting groups for example tert-butyloxycarbonylamino groups can be converted to the parent free amino group. Such deprotection can be effected in a conventional manner for example by treatment under acidic conditions for example using a 4N hydrochloric acid solution in methanol at room temperature.

Compounds C1, C2, D1, D3, D4, D5, G2, G4, G6, G7, G10, G12, G14, G15, G16, G17, G18, G20, G21, G22, G24, G25, G26, G27, G30, G32, G33, G34, G35, G37, G38, G39, G40, G41, G42, G43, G45, G46, G47, G48, G49 and G50 may be prepared according to the synthesis described in Scheme 1.

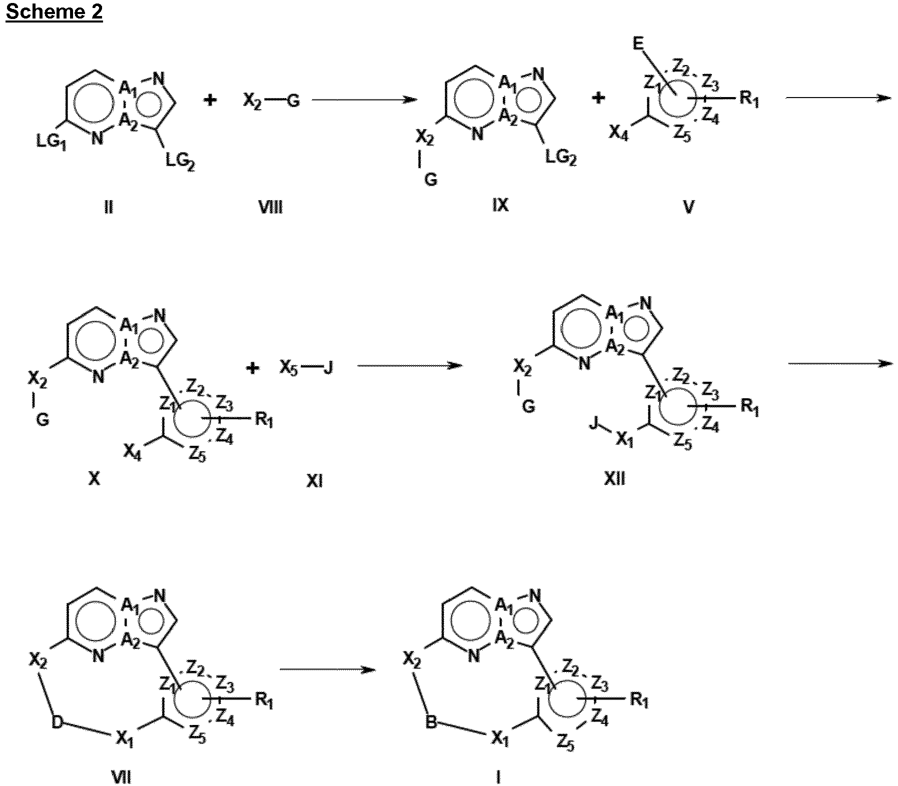

In the above reaction of the compound of formula (II) with the compound of formula (VIII) the leaving groups LG₁ and LG₂ are advantageously a halo group such as a chlorine or a bromine group. The reaction can be affected by a substitution for example by treating the compound of formula (II) with the compound of formula (VIII) in an organic solvent such as acetonitrile with an appropriate base such as for example diisopropylethylamine at an elevated temperature for example under reflux.

Compounds of formula (VIII) and (XI) can be either commercially acquired or obtained through various selective protection and deprotection steps.

The resulting compound of formula (IX) can optionally be protected with a suitable protecting group such as a tert-butyloxycarbonylamino group in a conventional manner for example by treatment with tert-butoxycarbonyl anhydride in basic conditions using for example triethylamine and 4-(dimethylamino)pyridine in a solvent such as tetrahydrofuran at an elevated temperature such as under reflux.

The reaction of the resulting compound (IX) with a (hetero-)aryl compound of formula (V) is advantageously effected through the coupling of a boronic acid E or boronic ester E derivative of the (hetero-)aryl compound under Suzuki conditions using for example tetrakis(triphenylphosphine)palladium(0), 2-dicyclohexylphosphino-2',4',6'-triisopropylbiphenyl (Xphos) and potassium phosphate tribasic in a solvent mixture such as 1,4-dioxane/water at an elevated temperature for example 80°C.

The reaction of the resulting compound of formula (X) with a compound of formula (XI) which can be advantageously effected under Williamson conditions using a base such as potassium carbonate in a solvent such as acetonitrile at an elevated temperature such as under reflux. This reaction can also be effected under Mitsunobu conditions using for example diisopropyl azodicarboxylate and triphenylphosphine in a solvent such as tetrahydrofuran at an elevated temperature such as 90°C.

The resulting compound of formula (XII) can optionally be treated to remove any desired protecting groups for example tert-butyloxycarbonylamino groups can be converted to the parent free amino group and for example ester groups can be converted to the parent free carboxylic acid groups. Such deprotection can be effected in a conventional manner for example by treatment under acidic conditions for example using an aqueous 6N hydrochloric acid solution in a solvent such as acetoniitrile at an elevated temperature for example 60°C or using an acid such as trifluoroacetic acid in a solvent such as dichloromethane at for example room temperature.

The cyclisation of the compound of formula (XII) can be effected for example by treatment with O-(benzotriazol-1-yl)-N,N,N',N'-tetramethyluronium hexafluorophosphate (HBTU) and N,N-diisopropylethylamine in a solvent such as N,N-dimethylformamide at for example room temperature. The cyclisation of the compound of formula (XII) can also be advantageously effected under Williamson conditions using a base such as potassium carbonate in a solvent such as acetonitrile at an elevated temperature such as under reflux or at room temperature. This reaction can also be effected under Mitsunobu conditions using for example diisopropyl azodicarboxylate and triphenylphosphine in a solvent such as tetrahydrofuran at an elevated temperature such as 90°C.

The resulting compound of formula (VII) can optionally be treated to form a compound of formula (I).

Compounds G1, G3, G8, G11, G13, G19, G23, G28, G31, G36 and G44 may be prepared according to the synthesis described in Scheme 1.

The above general processes are illustrated by the specific processes which are described in the patent applications WO2013/045653 A1 and WO2013/046029 A1.

The process to obtain Example A18 is described in the patent application WO2013/045653.

### Example C1

Example C1 is prepared following general scheme 1.
tert-Butyl N-(3-bromopyrazolo[1,5-a]pyrimidin-5-yl)-N-[2-[tert-butoxycarbonyl-[2-(tert-butyl(dimethyl)silyl)oxyethyl]amino]ethyl]carbamate was prepared according to the method described in the patent application WO2013/045653.

### Preparation of intermediate 1

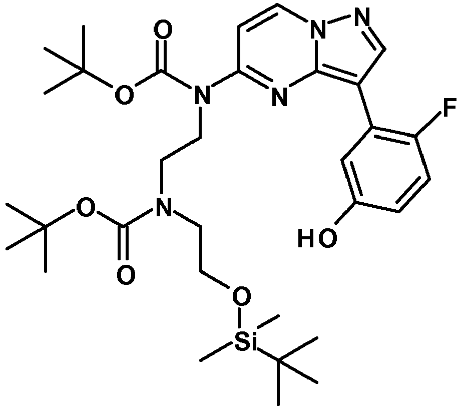

tert-Butyl N-(3-bromopyrazolo[1,5-a]pyrimidin-5-yl)-N-[2-[tert-butoxycarbonyl-[2-(tert-butyl(dimethyl)silyl)oxyethyl]amino]ethyl]carbamate can be prepared according to similar procedures described in the the patent application WO2013/045653 to obtain intermediate 23. A mixture of 1,4-dioxane and water (3:1, 10,3 ml) was degassed by bubbling nitrogen gas through the mixture. tert-Butyl N-(3-bromopyrazolo[1,5-a]pyrimidin-5-yl)-N-[2-[tert-butoxycarbonyl-[2-(tert-butyl(dimethyl)silyl)oxyethyl]amino]ethyl]carbamate (2.11 g, 3.43 mmol), 4-fluoro-3-(4,4,5,5-tetramethyl-1,3,2-dioxaborolan-2-yl)phenol (1.06 g, 4.46 mmol), tris(dibenzylideneacetone)dipalladium(0) (81 mg, 0.07 mmol), 2-dicyclohexylphosphino-2',4',6'-triisopropylbiphenyl (Xphos) (67 mg, 0.14 mmol) and potassium phosphate tribasic (3 eq.) were added and the mixture was stirred under nitrogen gas at 85°C overnight. The reaction mixture was cooled and 1,4-dioxane was removed under reduced pressure. Water was added and the aqueous layer was extracted with ethyl acetate. The organic layer was dried, filtered and the solvent was removed under reduced pressure. The residue was purified by flash column chromatography over silica gel using heptane and ethyl acetate as eluents (gradient elution from 0% to 20% ethyl acetate). The product fractions were collected and the solvent was evaporated.
Yield: 950 mg of intermediate 1 (43%)
LCMS method 1: MH⁺ = 546 (MW-Boc), RT = 1.585 min

### Preparation of intermediate 2

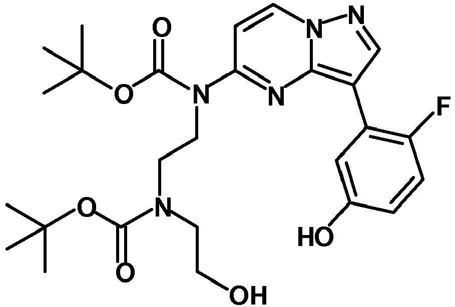

Tetrabutylammonium fluoride (461 mg, 1.76 mmol) was added to a solution of intermediate 1 (950 mg, 1.47 mmol) in tetrahydrofuran (4.41 ml). The reaction mixture was stirred at room temperature for 72 hours. The solvent was removed under reduced pressure. The residue was purified by flash column chromatography over silica gel using heptane and ethyl acetate as eluents (gradient elution from 0% to 70% ethyl acetate). The product fractions were collected and the solvent was evaporated.
Yield: 640 mg of intermediate C2 (82%)
LCMS method 1: MH⁺ = 532, RT = 1.025 min

### Preparation of intermediate 3

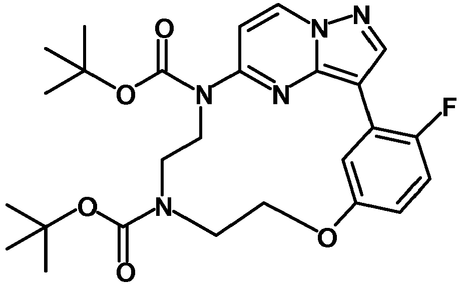

A solution of intermediate 2 (590 mg, 1.11 mmol) in 2-methyltetrahydrofuran (20 ml/mmol) and a solution of diisopropyl azodicarboxylate (660 mg, 3.33 mmol) in toluene (20 ml/mmol) were added drop wise and simultaneously to a solution of triphenylphosphine (873 mg, 3.33 mmol) in toluene (75 ml/mmol). The mixture was stirred at 90°C for 3 hours. The reaction mixture was cooled and the solvent was removed under reduced pressure. The residue was purified by flash column chromatography over silica gel using heptane and ethyl acetate as eluents (gradient elution from 0% to 40% ethyl acetate). The product fractions were collected and the solvent was evaporated.
Yield: 573 mg of intermediate 3 (100%)
LCMS method 1: MH⁺ = 514, RT = 5.132 min

### Preparation of example C1

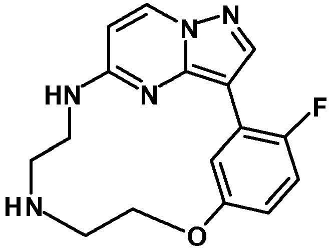

Intermediate 3 (523 mg, 1.02 mmol) was dissolved in 4N hydrochloric acid in methanol (3 ml). The mixture was stirred at room temperature for 2 hours. The solvent was removed under reduced pressure. Toluene was added and removed under reduced pressure (2 times). The compound was obtained as the hydrochloride salt.
Yield: 145 mg of example C1 (41 %)
LCMS method 2: MH⁺ = 314, RT = 1.971 min

### Example C2

Example C2 was prepared according to the general scheme 1 and more in particular to the methods described to obtain example C1.

### Example D1

Example D1 is prepared following general scheme 1.

### Preparation of intermediate 4

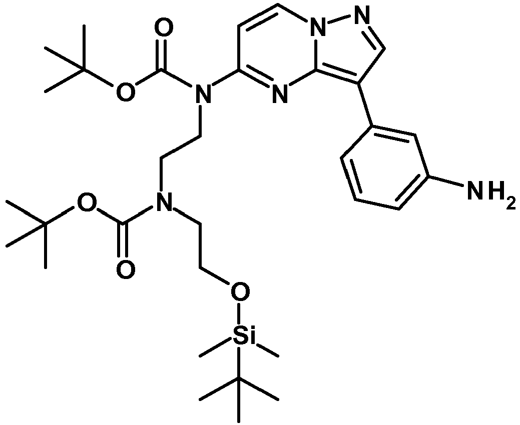

A mixture of 1,4-dioxane and water (3:1, 68,46 ml) was degassed by bubbling nitrogen gas through the mixture. tert-Butyl N-(3-bromopyrazolo[1,5-a]pyrimidin-5-yl)-N-[2-[tert-butoxycarbonyl-[2-(tert-butyl(dimethyl)silyl)oxyethyl]amino]ethyl]carbamate (14.025 g, 22.82 mmol), (3-aminophenyl)boronic acid hydrate (4.60 g, 29.67 mmol), tris(dibenzylideneacetone)dipalladium(0) (533 mg, 0.46 mmol), 2-dicyclohexylphosphino-2',4',6'-triisopropylbiphenyl (Xphos) (872 mg, 1.83 mmol) and potassium phosphate tribasic (3 eq.) were added and the mixture was stirred under nitrogen gas at 85°C for 4 hours. The reaction mixture was cooled and 1,4-dioxane was removed under reduced pressure. Water was added and the aqueous layer was extracted with ethyl acetate. The organic layer was dried, filtered and the solvent was removed under reduced pressure. The residue was purified by flash column chromatography over silica gel using heptane and ethyl acetate as eluents (gradient elution from 0% to 60% ethyl acetate). The product fractions were collected and the solvent was evaporated.
Yield: 13.741 g of intermediate 4 (96%)
LCMS method 1: MH⁺ = 527 (MH⁺-Boc), RT = 1.488 min

### Preparation of intermediate 5

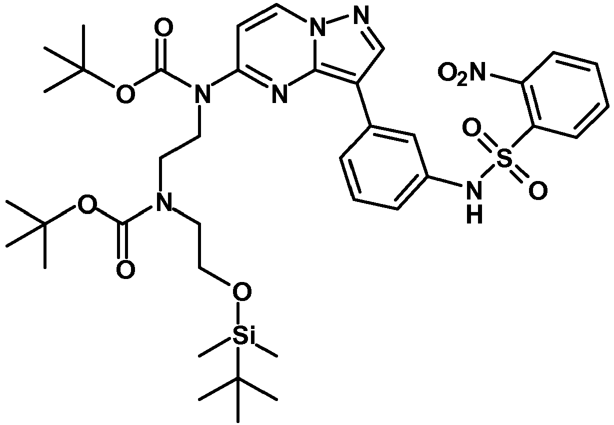

2-Nitrobenzenesulfonyl chloride (6.90 g, 31.14 mmol) was added portion wise at 0°C and under nitrogen atmosphere to a solution of intermediate 4 (13.013 g, 20.76 mmol), triethylamine (4.617 ml, 33.22 mmol) and 4-(dimethylamino)pyridine (127 mg, 1.04 mmol) in dichloromethane (62.28 ml). The reaction mixture was stirred overnight allowing it to reach room temperature. The solvent was removed under reduced pressure. Ethyl acetate was added and the organic layer was washed with water and brine. The organic layer was dried, filtered and the solvent was removed under reduced pressure. The residue was purified by flash column chromatography over silica gel using heptane and ethyl acetate as eluents (gradient elution from 0% to 80% ethyl acetate). The product fractions were collected and the solvent was evaporated.
Yield: 16.85 g of intermediate 5 (100%)
LCMS method 1: MH⁺ = 713 (MH⁺-Boc), RT = 1.544 min

### Preparation of intermediate 6

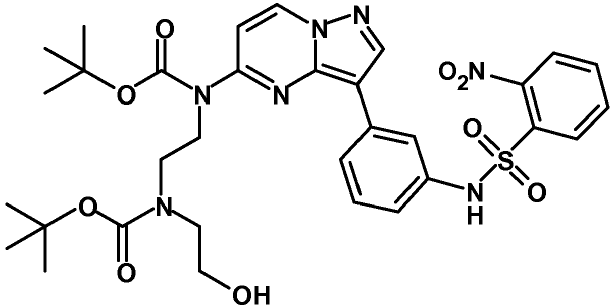

Tetrabutylammonium fluoride (4.39 g, 15.12 mmol) was added to a solution of intermediate 5 (8.184 g, 10.08 mmol) in tetrahydrofuran (30.24 ml). The reaction mixture was stirred at room temperature overnight. More tetrabutylammonium fluoride (2.93 g, 10.08 mmol) was added and the reaction mixture was stirred at room temperature overnight. The solvent was removed under reduced pressure. The residue was purified by flash column chromatography over silica gel using heptane and ethyl acetate as eluents (gradient elution from 0% to 70% ethyl acetate). The product fractions were collected and the solvent was removed under reduced pressure.
Yield: 3.78 g of intermediate 6 (54%)
LCMS method 1: MH⁺ = 598 (MH⁺-Boc), RT = 1.073 min

### Preparation of intermediate 7

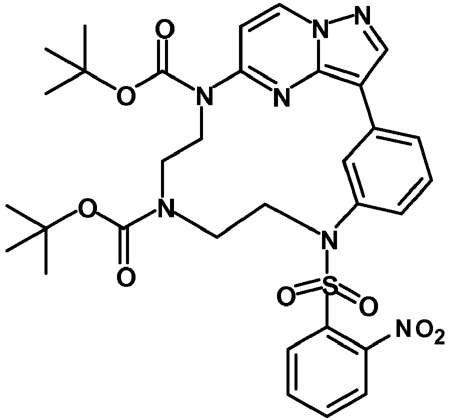

A solution of intermediate 6 (6.25 g, 8,96 mmol) in 2-methyltetrahydrofuran (20 ml/mmol) and a solution of diisopropyl azodicarboxylate (5.40 g, 26.88 mmol) in toluene (20 ml/mmol) were added drop wise and simultaneously to a solution of triphenylphosphine (7.05 g, 26.88 mmol) in toluene (75 ml/mmol). The mixture was stirred at 90°C for 3 hours. The reaction mixture was cooled and the solvent was removed under reduced pressure. The residue was purified by flash column chromatography over silica gel using heptane and ethyl acetate as eluents (gradient elution from 0% to 70% ethyl acetate). The product fractions were collected and the solvent was evaporated.
Yield: 3.02 g of intermediate 7 (50%)
LCMS method 1: MH⁺ = 680, RT = 1.348 min

### Preparation of intermediate 8

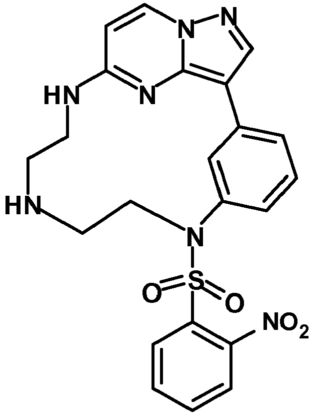

Intermediate 7 (3.02 g, 4.44 mmol) was dissolved in 4N hydrochloric acid in methanol (13.32 ml). The mixture was stirred at room temperature for 5 hours. The solvent was removed under reduced pressure and the residue was used in the next step without further purification.

### Preparation of example D1

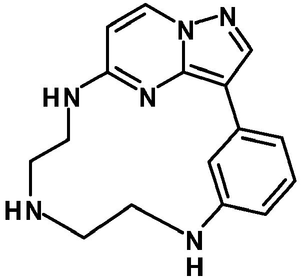

Intermediate 8 (150 mg, 0.29 mmol) and cesium carbonate (377 mg, 1.16 mmol) were suspended in N,N-dimethylformamide (0.87 ml). Thiophenol (40 µl, 0.35 mmol) was added and the mixture was stirred at room temperature for 18 hours. Sodium hydroxide was added and the solvent was removed under reduce pressure. The residue was suspended in a mixture of dichloromethane and methanol and filtered. The product was purified by reversed phase column chromatography (HPLC method A). The product fractions were collected and the solvent was removed under reduced pressure.
Yield: 25 mg of example D1 (29%)
LCMS method 2: MH⁺ = 295, RT = 1.753 min

Examples D3, D4 and D5 may be prepared according to the synthesis as described above.

### Example G1

Example G1 is prepared following general scheme 2.

### Preparation of intermediate 9

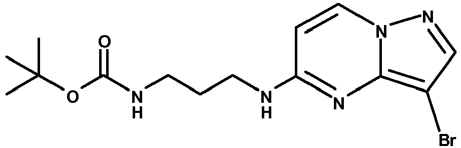

A mixture of 3-bromo-5-chloro-pyrazolo[1,5-a]pyrimidine (6.0 g, 25.812 mmol), tert-butyl N-(3-aminopropyl)carbamate (4.95 g, 28.39 mmol) and N,N-diisopropylethylamine (5.27 ml, 30.97 mmol) in acetonitrile (77 ml) was refluxed for 4 hours. More tert-butyl N-(3-aminopropyl)carbamate (450 mg, 2.58 mmol) was added and the reaction mixture was refluxed overnight. The reaction mixture was cooled and the solvent was removed under reduced pressure. The residue was dissolved in ethyl acetate and washed with water and brine. The organic layer was dried, filtered and the solvent was removed under reduced pressure. The residue was purified by flash column chromatography over silica gel using heptane and ethyl acetate as eluents (gradient elution from 20 % to 100 % of ethyl acetate). The product fractions were collected and the solvent was evaporated.
Yield: 6.99 g of intermediate 9 (73%)
LCMS method 1: MH⁺ = 371, RT = 0.792 min

### Preparation of intermediate 10

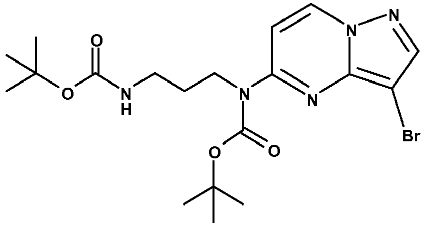

A mixture of intermediate 9 (14.43 g, 38.97 mmol) in tetrahydrofuran (117 ml) were added tert-butoxycarbonyl anhydride (8.93 g, 40.92 mmol), triethylamine (6 ml, 42.87 mmol) and 4-(dimethylamino)pyridine (238 mg, 1.95 mmol) was stirred under reflux overnight. The solvent was removed under reduced pressure. The residue was dissolved in ethyl acetate and washed with water and brine. The organic layer was dried, filtered and the solvent was removed under reduced pressure. The residue was purified by flash column chromatography over silica gel using heptane and ethyl acetate as eluents (gradient elution from 0 % to 33 % of ethyl acetate). The product fractions were collected and the solvent was evaporated.
Yield: 14.53 g of intermediate 10 (79%)
LCMS method 1: MH⁺ = 494, RT = 1.184 min

### Preparation of intermediate 11

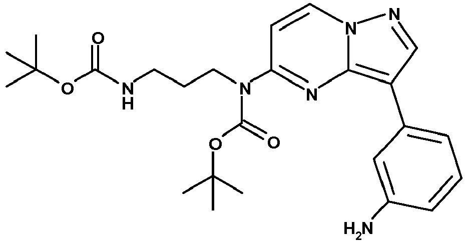

A mixture of 1,4-dioxane and water (3:1, 53 ml) was degassed by bubbling nitrogen gas through the mixture. Intermediate 10 (4.00 g, 8.50 mmol), (3-aminophenyl)boronic acid (1.71 g, 11.05 mmol), tetrakis(triphenylphosphine)palladium(0) (197 mg, 0.17 mmol), 2-dicyclohexylphosphino-2',4',6'-triisopropylbiphenyl (Xphos) (162 mg, 0.34 mmol) and potassium phosphate tribasic (5.41 g, 3 eq.) were added and the mixture was stirred under nitrogen gas at 80°C for 5 hours. The reaction mixture was cooled, diluted with ethyl acetate and the organic layer was washed with water and brine. The organic layer was dried, filtered and the solvent was removed under reduced pressure. The residue was purified by flash column chromatography over silica gel using heptane and ethyl acetate as eluents (gradient elution from 20 % to 100 % of ethyl acetate). The product fractions were collected and the solvent was removed under reduced pressure.
LCMS method 1: MH⁺ = 483, RT = 1.492 min

### Preparation of intermediate 12

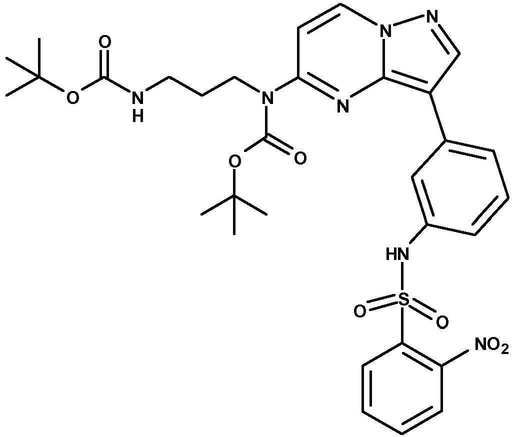

2-Nitrobenzenesulfonyl chloride (3.24 g, 14.61 mmol) was added to a solution of intermediate 11 (4.70 g, 9.74 mmol) and triethylamine (4 ml, 29.22 mmol) in dichloromethane (50 ml). 4-Dimethylaminopyridine (60 mg, 0.49 mmol) was added and the reaction mixture was stirred at room temperature overnight. More 2-nitrobenzenesulfonyl chloride (716 mg, 3.23 mmol) was added and the mixture was stirred at room temperature for 2 days. The crude reaction mixture was diluted with dichloromethane and washed with a saturated aqueous sodium bicarbonate solution and water. The organic layer was dried, filtered and the solvent was removed under reduced pressure. The residue was purified by flash column chromatography over silica gel using heptane and ethyl acetate as eluents (gradient elution from 0 % to 50 % of ethyl acetate). The product fractions were collected and the solvent was evaporated.
Yield: 4.40 g of intermediate 12 (68%)

### Preparation of intermediate 13

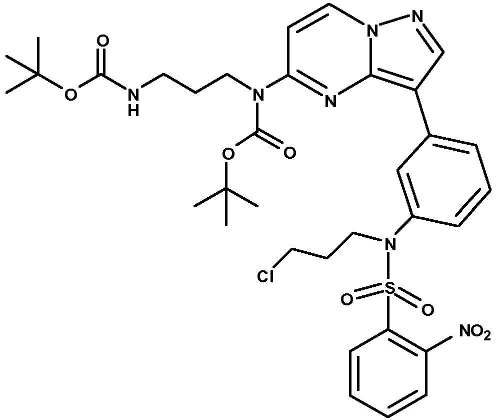

A mixture of intermediate 12 (4.40 g, 6.59 mmol), cesium carbonate (5.37 g, 16.48 mmol) and 1-bromo-3-chloro-propane (1.31 ml, 13.18 mmol) in N,N-dimethylformamide (20 ml) was stirred at room temperature overnight. More 1-bromo-3-chloro-propane (650 µl, 6.59 mmol) and cesium carbonate (1.15 g, 6.59 mmol) were added and the reaction mixture was stirred at 75°C overnight. The reaction mixture was cooled and concentrated under reduced pressure. The residue was diluted with ethyl acetate and the organic layer was washed with water. The organic layer was dried, filtered and the solvent was removed under reduced pressure. The product was used in the next step without further purification.
Yield: 1.82 g of intermediate 13 (37%)
LCMS method 1: MH⁺ = 644 (MW - Boc), RT = 1.278 min

### Preparation of intermediate 14

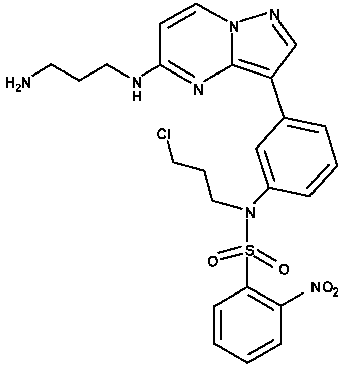

Intermediate 13 (1.82 g, 2.45 mmol) was dissolved in 4N hydrochloric acid in methanol (7.35 ml). The mixture was stirred at room temperature for 2 hours. The solvent was removed under reduced pressure. The compound was obtained as the hydrochloride salt and was used in the next step without further purification.

### Preparation of intermediate 15

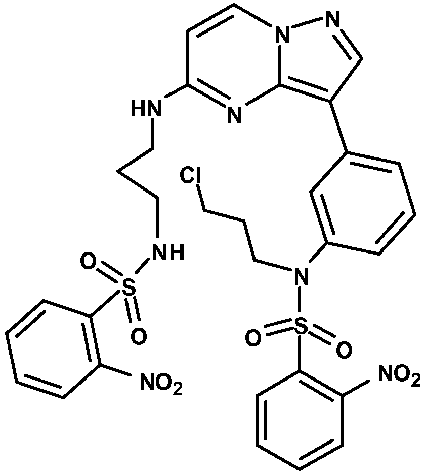

2-Nitrobenzenesulfonyl chloride (0.60 g, 2.69 mmol) was dissolved in N,N-dimethylacetamide (2 ml) and added drop wise at 0°C to a stirred solution of intermediate 14 (2.45 mmol) and triethylamine (1.70 ml, 12.25 mmol) in N,N-dimethylacetamide (5.35 ml). The reaction mixture was stirred at 0°C for 1 hour. The crude reaction mixture was diluted with dichloromethane and washed with water. The organic layer was dried, filtered and the solvent was removed under reduced pressure. The residue was purified by flash column chromatography over silica gel using heptane and ethyl acetate as eluents. The product fractions were collected and the solvent was evaporated.
Yield: 1.618 g of intermediate 15 (91%)
LCMS method 1: MH⁺ = 730, RT = 1.844 min

### Preparation of intermediate 16

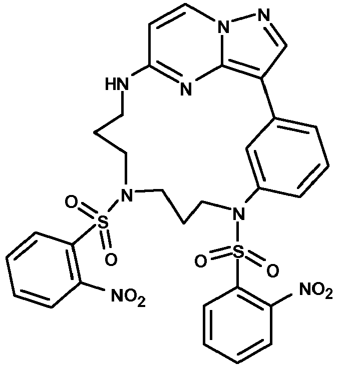

To a stirred suspension of cesium carbonate (3.61 g, 11.10 mmol) in N,N-dimethylformamide (56 ml) was added drop wise at 90°C a solution of intermediate 15 (1.618 g, 2.22 mmol) in N,N-dimethylformamide (166 ml). The reaction mixture was stirred at 90°C for 1 hour. The solvent was removed under reduced pressure. The product was used in the next step without further purification.

### Preparation of example G1

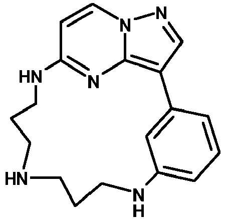

Intermediate 16 (2.22 mmol) and cesium carbonate (2.886 g, 8.88 mmol) were suspended in N,N-dimethylformamide (6.7 ml). Thiophenol (500 µl, 4.88 mmol) was added and the mixture was stirred at room temperature for 2 hours. Tert-butoxycarbonyl anhydride (1.06 g, 2.20 mmol) was added and the mixture was stirred at room temperature for 4 hours.The reaction mixture was diluted with ethyl acetate and washed with brine. The organic layer was dried, filtered and the solvent was removed under reduced pressure. The residue was dissolved in 4N hydrochloric acid in methanol (10 ml). The mixture was stirred at room temperature for 1 hour. The solid was filtered and washed with diethyl ether and methanol. The product was obtained as the HCl salt.
Yield: 0.470 g of example G1 (82%)
LCMS method 1: MH⁺ = 323, RT = 0.416 min

### Example G2

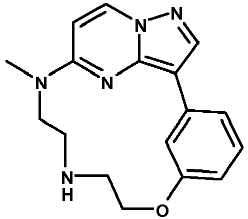

Example G2 may be prepared following general scheme 1 and according to the procedures illustrated above for the preparation of example C1.

### Example G3

Example G3 is prepared following general scheme 2.

### Preparation of intermediate 17

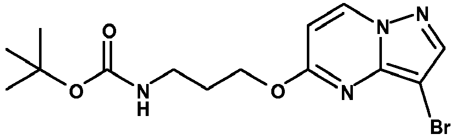

Sodium hydride (60% in mineral oil, 2.58 g, 64.53 mmol) and tert-butyl N-(3-hydroxypropyl)carbamate (30.15 g, 172.08 mmol) were dissolved in anhydrous tetrahydrofuran and stirred at room temperature for 30 minutes. 3-bromo-5-chloro-pyrazolo[1,5-a]pyrimidine (10.00 g, 43.02 mmol) was added portion wise and the reaction mixture was stirred at room temperature for 1 hour. Water was added and the tetrahydrofuran was removed under reduced reduced pressure. The residu was diluted with dichloromethane and washed with water. The organic layer was dried, filtered and the solvent was removed under reduced pressure. The residue was purified by flash column chromatography over silica gel using heptane and ethyl acetate as eluents (gradient elution from 10 % to 80 % of ethyl acetate). The product fractions were collected and the solvent was removed under reduced pressure. The product was triturated with diethyl ether, filtered and dried under reduced pressure.
Yield: 14.06 g of intermediate 17 (88%)
LCMS method 2: MH⁺ = 315, RT = 3.401 min

### Preparation of intermediate 18

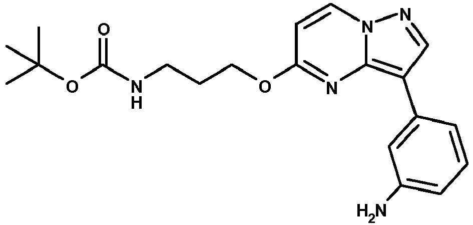

A mixture of 1,4-dioxane and water (3:1, 65 ml) was degassed by bubbling nitrogen gas through the mixture. Intermediate 17 (4.00 g, 10.77 mmol), (3-aminophenyl)boronic acid (2.17 g, 14.00 mmol), tetrakis(triphenylphosphine)palladium(0) (255 mg, 0.22 mmol), 2-dicyclohexylphosphino-2',4',6'-triisopropylbiphenyl (Xphos) (205 mg, 0.43 mmol) and potassium phosphate tribasic (85 g, 3 eq.) were added and the mixture was stirred under nitrogen gas at 80°C for 3 hours. The reaction mixture was cooled, diluted with ethyl acetate and the organic layer was washed with water and brine. The organic layer was dried, filtered and the solvent was removed under reduced pressure. The residue was purified by flash column chromatography over silica gel using heptane and ethyl acetate as eluents (gradient elution from 30 % to 100 % of ethyl acetate). The product fractions were collected and the solvent was removed under reduced pressure.
Yield: 3.12 g of intermediate 18 (76%)
LCMS method 1: MH⁺ = 384, RT = 1.012 min

### Preparation of intermediate 19

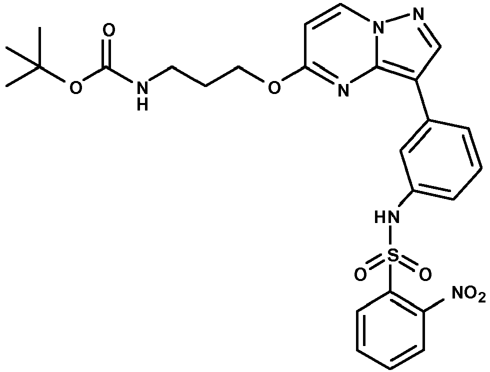

2-Nitrobenzenesulfonyl chloride (3.38 g, 15.52 mmol) was added portion wise to a solution of intermediate 18 (3.90 g, 10.17 mmol) and triethylamine (4.24 ml, 30.51 mmol) in dichloromethane (42 ml). 4-Dimethylaminopyridine (62 mg, 0.51 mmol) was added and the reaction mixture was stirred at room for 18 hours. The reaction mixture was diluted with dichloromethane and washed with a saturated aqueous sodium bicarbonate solution and water. The organic layer was dried, filtered and the solvent was removed under reduced pressure. The residue was purified by flash column chromatography over silica gel using heptane and ethyl acetate as eluents (gradient elution from 0 % to 40 % of ethyl acetate). The product fractions were collected and the solvent was removed under reduced pressure.
Yield: 5.71 g of intermediate 19 (99%)

### Preparation of intermediate 20

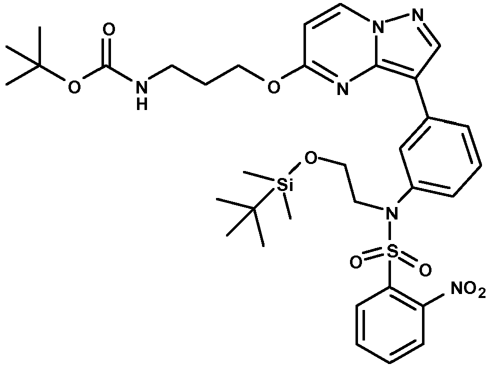

2-Bromoethoxy-tert-butyl-dimethyl-silane (360 mg, 1.68 mmol) was added to a mixture of intermediate 19 (716 mg, 1.53 mmol) and cesium carbonate (600 mg, 1.84 mmol) in N,N-dimethylformamide (4.6 ml). The reaction mixture was stirred at 75°C overnight. The reaction mixture was cooled and concentrated under reduced pressure. The residue was purified by flash column chromatography over silica gel using heptane and ethyl acetate as eluents (gradient elution from 10 % to 60 % of ethyl acetate). The product fractions were collected and the solvent was removed under reduced pressure.
Yield: 560 mg of intermediate 20 (58%)
LCMS method 2: MH⁺ = 627, RT = 4.960 min

### Preparation of intermediate 21

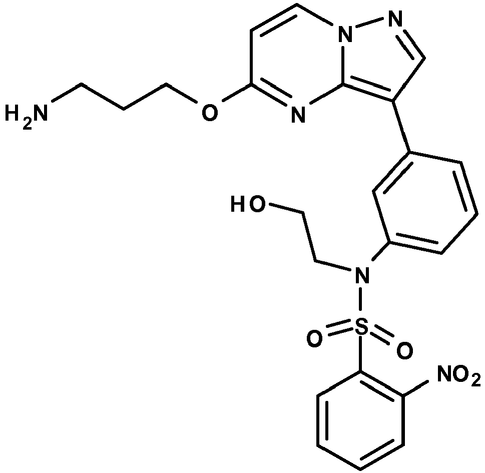

Intermediate 20 (3.42 g, 4.70 mmol) was dissolved in 4N hydrochloric acid in methanol (14.1 ml). The mixture was stirred at room temperature for overnight. The solvent was removed under reduced pressure, toluene was added twice and removed twice under reduced pressure. The residue was used in the next step without further purification.
LCMS method 2: MH⁺ = 513, RT = 2.276 min

### Preparation of intermediate 22

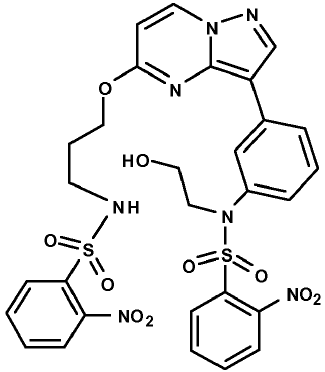

2-Nitrobenzenesulfonyl chloride (510 mg, 2.29 mmol) dissolved in N,N-dimethylacetamide (8.2 ml) was added at 0°C to a solution of intermediate 21 (2.29 mmol) and triethylamine (1.592 ml, 11.45 mmol) in N,N-dimethylacetamide (6.9 ml). The reaction mixture was stirred at 0°C for 1 hour. More triethylamine (1.0 ml, 7.19 mmol) was added and the mixture was stirred at room temperature overnight. The crude mixture was diluted with ethyl acetate and washed with water. The organic layer was dried, filtered and the solvent was removed under reduced pressure. The residue was purified by flash column chromatography over silica gel using heptane and ethyl acetate as eluents (gradient elution from 30 % to 80 % of ethyl acetate). The product fractions were collected and the solvent was removed under reduced pressure.
Yield: 1.40 g of intermediate 22 (88%)
LCMS method 1: MH⁺ = 698, RT = 2.011 min

### Preparation of intermediate 23

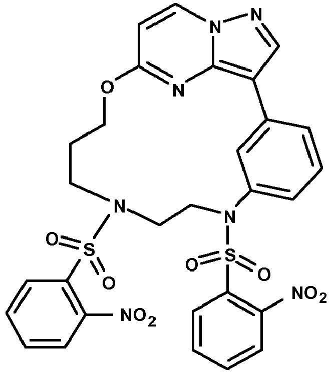

A solution of intermediate 22 (1.40 g, 2.01 mmol) in 2-methyltetrahydrofuran (20 ml/mmol) was degassed by bubbling nitrogen gas through the mixture. A degassed solution of diisopropyl azodicarboxylate (1.20 g, 6.03 mmol) in toluene (20 ml/mmol) was added drop wise at 90°C and simultaneously to a degassed solution of triphenylphosphine (1.582 g, 6.03 mmol) in toluene (75 ml/mmol). The mixture was stirred at 90°C for 30 minutes. The reaction mixture was cooled and the solvent was removed under reduced pressure. Methanol was added, the solid was filtered and washed with diethyl ether. The residue was dried under reduced pressure and without further purification used in the next step.
Yield: 1.05 g of intermediate 23 (77%)
LCMS method 2: MH⁺ = 680, RT = 3.810 min

### Preparation of intermediate 24

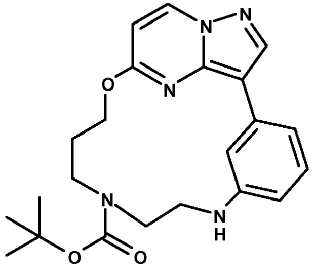

Intermediate 23 (1.05 g, 1.54 mmol) and cesium carbonate (2.00 g, 6.16 mmol) were suspended in N,N-dimethylformamide (4.6 ml). Thiophenol (350 µl, 3.39 mmol) was added and the mixture was stirred at room temperature overnight. Tert-butoxycarbonyl anhydride (740 mg, 3.39 mmol) was added and the mixture was stirred at room temperature for 4 hours. An aqueous solution of 1N sodium hydroxide was added and the mixture was extracted with ethyl acetate. The organic layer was washed with water and brine. The organic layer was dried, filtered and the solvent was removed under reduced pressure. The residue was purified by flash column chromatography over silica gel using heptane and ethyl acetate as eluents (gradient elution from 30 % to 80 % of ethyl acetate). The product fractions were collected and the solvent was removed under reduced pressure.
Yield: 392 mg of intermediate 24 (62%)
LCMS method 2: MH⁺ = 410, RT = 3.502 min

### Preparation of example G3

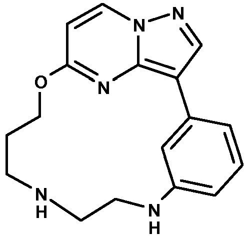

Intermediate 24 (392 mg, 0.96 mmol) was dissolved in 4N hydrochloric acid in dioxane (2.88 ml). The mixture was stirred at room temperature for 2 hours. The solvent was removed under reduced pressure, toluene was added twice and removed twice under reduced pressure. The product was obtained as the HCl salt.
LCMS method 2: MH⁺ = 310, RT = 1.940 min

### Example G4

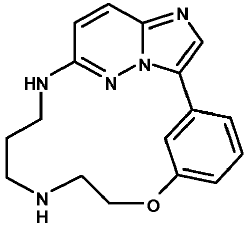

Example G4 may be prepared following general scheme 1 and according to the procedures illustrated above for the preparation of example C1.

### Example G5

Example G5 may be prepared following general scheme 2.

### Preparation of intermediate 25

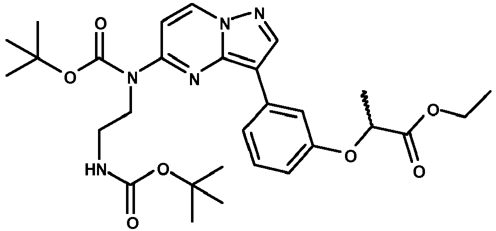

Tert-butyl N-[2-(tert-butoxycarbonylamino)ethyl]-N-[3-(3-hydroxyphenyl)pyrazolo[1,5-a]pyrimidin-5-yl]carbamate is prepared according to the method described in the patent application WO2013/0460029. A mixture of tert-butyl N-[2-(tert-butoxycarbonylamino)ethyl]-N-[3-(3-hydroxyphenyl)pyrazolo[1,5-a]pyrimidin-5-yl]carbamate (1.65 g, 3.51 mmol), ethyl 2-bromopropanoate (690 µl, 5.26 mmol) and potassium carbonate (970 mg, 7.02 mmol) in N,N-dimethylformamide (10.5 ml) was stirred at 80°C for 2 hours. The solvent was removed under reduced pressure and the residue was dissolved in ethyl acetate. The organic layer was washed with water and brine. The organic layer was dried, filtered and the solvent was removed under reduced pressure. The residue was purified by flash column chromatography over silica gel using heptane and ethyl acetate as eluents (gradient elution from 0 % to 50 % of ethyl acetate). The product fractions were collected and the solvent was removed under reduced pressure.
Yield: 1,60 g of intermediate 25 (80%)
LCMS method 1: MH⁺ = 470 (=MH⁺-Boc), RT = 1.320 min

### Preparation of intermediate 26

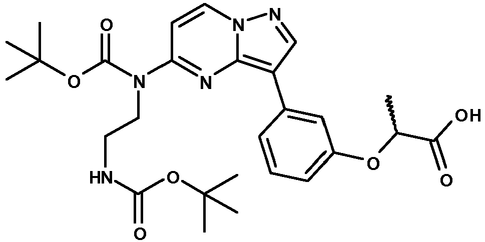

Intermediate 25 (1.60 g, 2.81 mmol) was dissolved in a mixture of tetrahydrofuran, methanol and water (2;2;1,8.43 ml) and lithium hydroxide monohydrate (340 mg, 8.43 mmol) was added. The reaction mixture was stirred at 50°C for 4 hours. The solvent was removed under reduced pressure and the residue was used in the next step without further purification.
LCMS method 1: MH⁺ = 542, RT = 1.129 min

### Preparation of intermediate 27

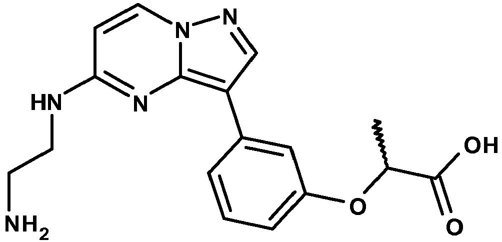

Intermediate 26 (2.81 mmol) was dissolved in 4N hydrochloric acid in dioxane (8.43 ml). The mixture was stirred at room temperature for 72 hours. The solvent was removed under reduced pressure, toluene was added twice and removed twice under reduced pressure. The product was obtained as the HCl salt.
LCMS method 1: MH⁺ = 342, RT = 0.391 min

### Preparation of intermediate 28

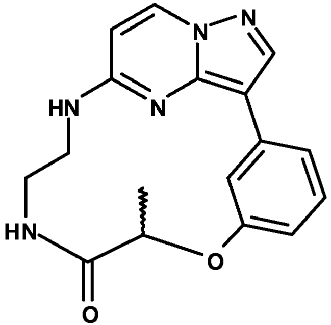

A solution of intermediate 27 (2.81 mmol) and N,N-diisopropylethylamine (2.4 ml, 5 eq.) in N,N-dimethylformamide (93 ml) was added drop wise to a solution O-(benzotriazol-1-yl)-N,N,N',N'-tetramethyluronium hexafluorophosphate (HBTU) (3.21 g, 8.43 mmol) and N,N'-diisopropylmethanediimine (4.9 ml, 10 eq.) in N,N-dimethylformamide (188 ml). The reaction mixture was stirred at room temperature for 1 hour after the addition was completed. The solvent was removed under reduced pressure. The residue was dissolved in ethyl acetate and washed with water and brine. The organic layer was dried, filtered and the solvent was removed under reduced pressure. The residue was purified by flash column chromatography over silica gel using dichloromethane and methanol as eluents (gradient elution from 2 % to 10 % of methanol). The product fractions were collected and the solvent was evaporated. The residue was purified by reversed phase column chromatography (HPLC method A).
Yield: 150 mg of intermediate 28 (17%)
LCMS method 2: MH⁺ = 324, RT = 2.126 min

### Preparation of example G5

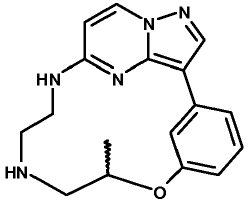

Intermediate 28 (130 mg, 0.4 mmol) was dissolved in 2N borane dimethylsulfide in tetrahydrofuran (1 ml). The reaction mixture was stirred at room temperature for 18 hours. More 2N borane dimethylsulfide in tetrahydrofuran (0.5 ml)was added. The reaction mixture was stirred at room temperature for 24 hours. An aqueous 2N hydrochloric acid solution was added and the reaction mixture was stirred at 100°C for 1 hour. The reaction mixture was neutralized to pH 7 with a saturated aqueous sodium bicarbonate solution. The product was extracted with a mixture of dichloromethane and methanol (9:1). The solvent of the organic layer was removed under reduced pressure. The residue was purified by reversed phase column chromatography (HPLC method A).
Yield: 14 mg of example G5 (1 %)
LCMS method 2: MH⁺ = 310, RT = 1.990 min

### Example G6

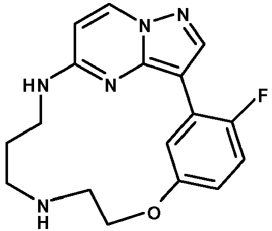

Example G6 may be prepared following general scheme 1 and according to the procedures illustrated above for the preparation of example C1.

### Example G7

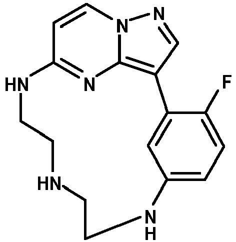

Example G7 may be prepared following general scheme 1 and according to the procedures illustrated above for the preparation of example D1.

### Example G8

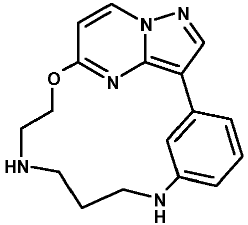

Example G8 may be prepared following general scheme 2 and according to the procedures illustrated above for the preparation of example G3.

### Example G10

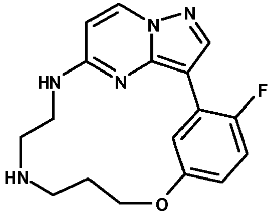

Example G10 may be prepared following general scheme 1 and according to the procedures illustrated above for the preparation of example C1.

### Example G11

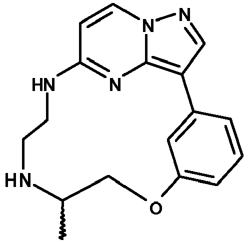

Example G11 may be prepared following general scheme 2 and according to the procedures illustrated above for the preparation of example G5.

### Example G12

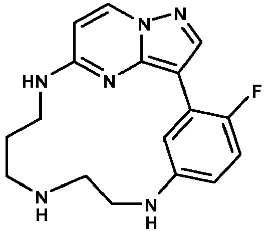

Example G12 may be prepared following general scheme 1 and according to the procedures illustrated above for the preparation of example D1.

### Example G13

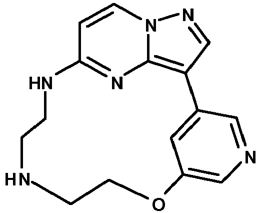

Example G13 may be prepared following general scheme 2 and according to the procedures illustrated above for the preparation of example G5.

### Example G14

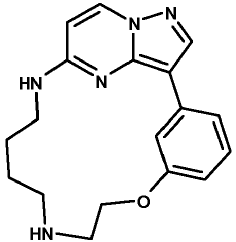

Example G14 may be prepared following general scheme 1 and according to the procedures illustrated above for the preparation of example C1.

### Example G15

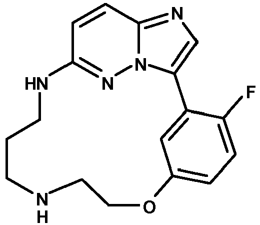

Example G15 may be prepared following general scheme 1 and according to the procedures illustrated above for the preparation of example C1.

### Example G16

Example G16 may be prepared following general scheme 1.

### Preparation of intermediate 29

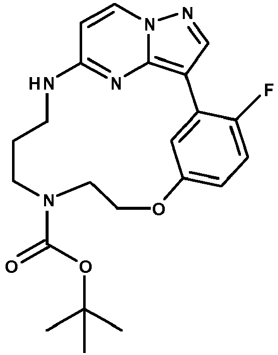

Example G6 (HCl salt, 400 mg, 1.10 mmol) and triethylamine (306 µl, 2.20 mmol) were suspended in tetrahydrofuran (3.3 ml). tert-Butoxycarbonyl anhydride (0.38 g, 1.65 mmol) was added and the mixture was stirred at room temperature for 4 hours. The solvent was removed under reduced pressure. The residue was dissolved in dichloromethane and washed with water. The organic layer was dried, filtered and the solvent was removed under reduced pressure. The residue was purified by flash column chromatography over silica gel using dichloromethane and methanol as eluents (gradient elution from 0 % to 3 % of methanol). The product fractions were collected and the solvent was evaporated.
Yield: 389 mg of intermediate 29 (83%)
LCMS method 1: MH⁺ = 428, RT = 1.084 min

### Preparation of intermediate 30

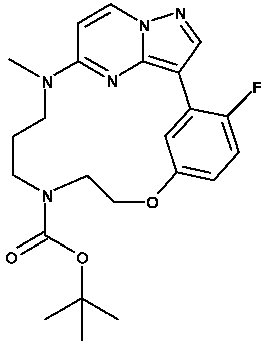

Sodium hydride (60% in mineral oil, 180 mg, 4.60 mmol) was added in one portion to a solution of intermediate 29 (460 mg, 0.195 mmol) in anhydrous N,N-dimethylformamide (5 ml). The mixture was stirred at room temperature for 50 minutes. Methyl iodide (33 µl, 0.53 mmol) was added and the mixture was stirred at room temperature for 2 hours. More methyl iodide (0.2 eq) was added and the mixtures was stirred at room temperature for 90 minutes. More sodium hydride (60% in mineral oil, 180 mg, 4.60 mmol) and methyl iodide (33 µl, 0.53 mmol) were added and the mixture was stirred at room temperature overnight. The reaction mixture was poured into a cold saturated aqueous ammonium chloride solution and the product was extracted with ethyl acetate. The combined organic layers were dried, filtered and the solvent was removed under reduced pressure. The residue was purified by flash column chromatography over silica gel using dichloromethane and methanol as eluents (gradient elution from 0 % to 2 % of methanol). The product fractions were collected and the solvent was evaporated.
Yield: 133 mg of intermediate 30 (65%)
LCMS method 2: MH⁺ = 442, RT = 4.471 min

### Preparation of example G16

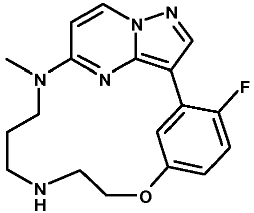

Intermediate 30 (133 mg, 0.30 mmol) was dissolved in 4N hydrochloric acid in dioxane (0.90 ml). The mixture was stirred at 50°C for 2 hours. The solvent was removed under reduced pressure, diethyl ether was added twice and removed twice under reduced pressure. The product was dried at 55°C under reduced pressure. The product was obtained as the HCl salt.
Yield: 112 mg of example G16 (99%)
LCMS method 2: MH⁺ = 342, RT = 1.907 min

### Example G17

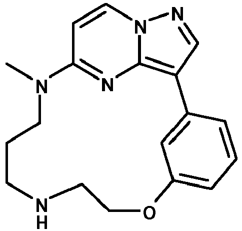

Example G17 may be prepared following general scheme 1 and according to the procedures illustrated above for the preparation of example G16.

### Example G18

Example G18 may be prepared following general scheme 1.

### Preparation of example G18

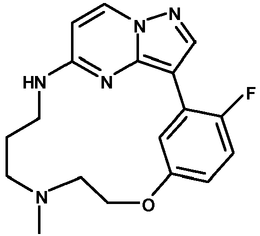

Example G6 (157 mg, 0.48 mmol) and formaldehyde (37% aqueous solution) were suspended in a mixture of dichloromethane/methanol (1:1, 1.44 ml) and the mixture was stirred at room temperature for 1 hour. Sodium triacetoxyborohydride (203 mg, 0.96 mmol) was added portion wise. The reaction mixture was stirred at room temperature for 16 hours. Ethyl acetate was added and the organic layer was washed with a saturated sodium bicarbonate solution and brine. The organic layer was dried and the solvent was removed under reduced pressure. The residue was purified by flash column chromatography over silica gel using dichloromethane and methanol as eluents (gradient elution from 0 % to 5 % of methanol). The product fractions were collected and the solvent was evaporated.
Yield: 119 mg of example G18 (73%)
LCMS method 2: MH⁺ = 342, RT = 1.865 min

Example G18 (119 mg, 0.35 mmol) was dissolved in a mixture of dichloromethane/methanol (4:1, 1.05 ml) and 4N hydrochloric acid in dioxane (0.13 ml, 0.52 mmol) was added. The reaction mixture was stirred at room temperature for 2 hours. The solvent was removed under reduced pressure, diethyl ether was added twice and removed twice under reduced pressure. The product was dried at 60°C under reduced pressure for 8 hours. The product was obtained as the HCl salt.
Yield: 117 mg of example G18 as HCl salt (883%)
LCMS method 2: MH⁺ = 342, RT = 1.853 min

### Example G19

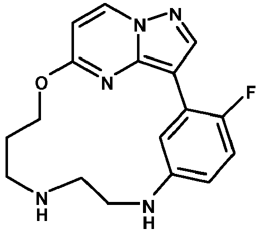

Example G19 may be prepared following general scheme 2 and according to the procedures illustrated above for the preparation of example G3.

### Example G20

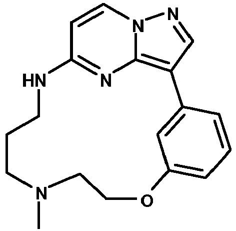

Example G20 may be prepared following general scheme 1 and according to the procedures illustrated above for the preparation of example G18.

### Example G21

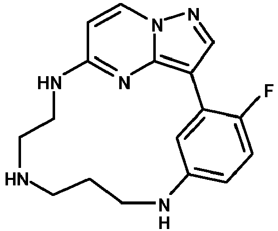

Example G21 may be prepared following general scheme 1 and according to the procedures illustrated above for the preparation of example D1.

### Example G22

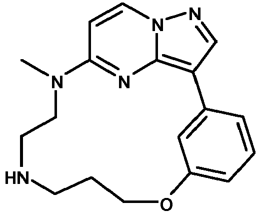

Example G22 may be prepared following general scheme 1 and according to the procedures illustrated above for the preparation of example G16.

### Example G23

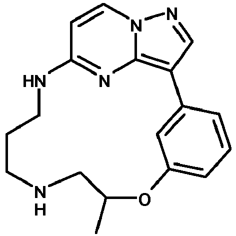

Example G23 may be prepared following general scheme 2 and according to the procedures illustrated above for the preparation of example G5.

### Example G24

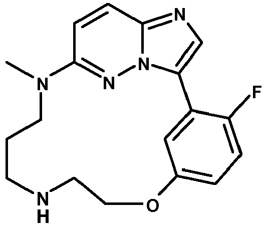

Example G24 may be prepared following general scheme 1 and according to the procedures illustrated above for the preparation of example G16.

### Example G25

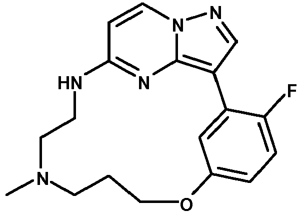

Example G25 may be prepared following general scheme 1 and according to the procedures illustrated above for the preparation of example G18.

### Example G26

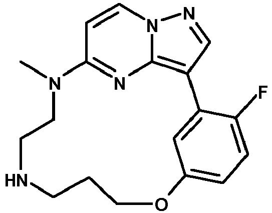

Example G26 may be prepared following general scheme 1 and according to the procedures illustrated above for the preparation of example G16.

### Example G27

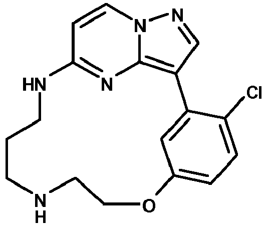

Example G27 may be prepared following general scheme 1 and according to the procedures illustrated above for the preparation of example C1.

### Example G28

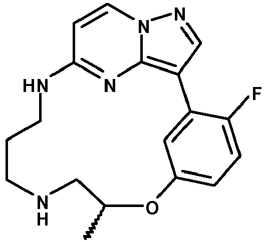

Example G28 may be prepared following general scheme 2 and according to the procedures illustrated above for the preparation of example G5.

### Example G30

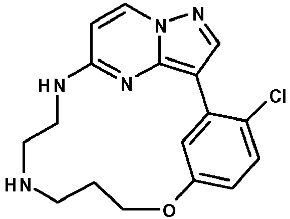

Example G30 may be prepared following general scheme 1 and according to the procedures illustrated above for the preparation of example C1.

### Example G31

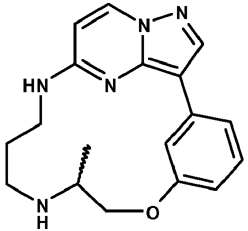

Example G31 may be prepared following general scheme 2 and according to the procedures illustrated above for the preparation of example G5.

### Example G32

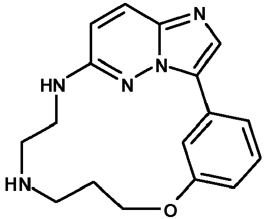

Example G32 may be prepared following general scheme 1 and according to the procedures illustrated above for the preparation of example C1.

### Example G33

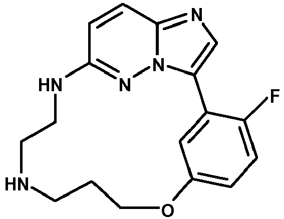

Example G33 may be prepared following general scheme 1 and according to the procedures illustrated above for the preparation of example C1.

### Example G34

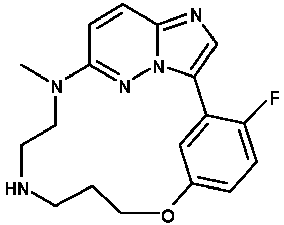

Example G34 may be prepared following general scheme 1 and according to the procedures illustrated above for the preparation of example G16.

### Example G35

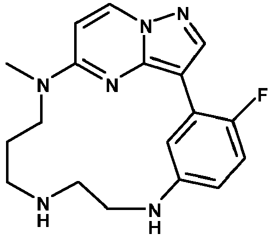

Example G35 may be prepared following general scheme 1.

### Example G36

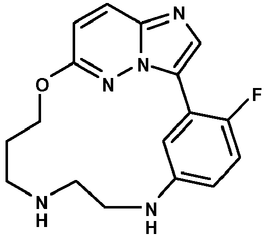

Example G36 may be prepared following general scheme 2 and according to the procedures illustrated above for the preparation of example G3.

### Example G37

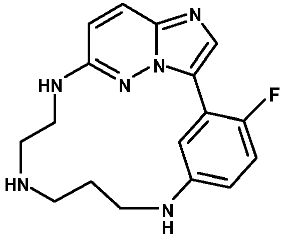

Example G37 may be prepared following general scheme 1 and according to the procedures illustrated above for the preparation of example D1.

### Example G38

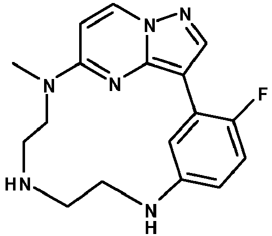

Example G38 may be prepared following general scheme 1.

### Example G39

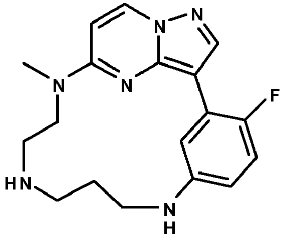

Example G39 may be prepared following general scheme 1.

### Example G40

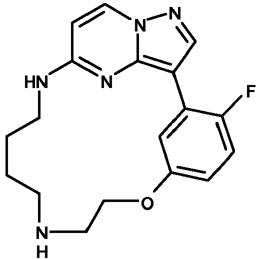

Example G40 may be prepared following general scheme 1 and according to the procedures illustrated above for the preparation of example C1.

### Example G41

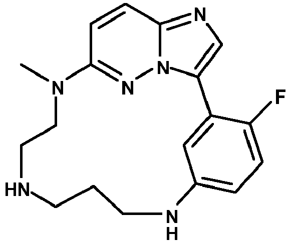

Example G41 may be prepared following general scheme 1.

### Example G42

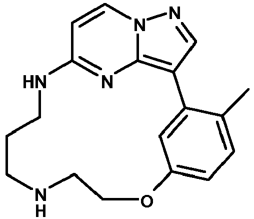

Example G42 may be prepared following general scheme 1 and according to the procedures illustrated above for the preparation of example C1.

### Example G43

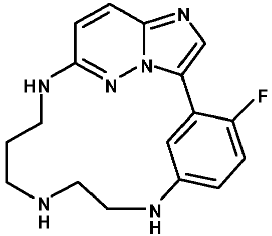

Example G43 may be prepared following general scheme 1 and according to the procedures illustrated above for the preparation of example D1.

### Example G44

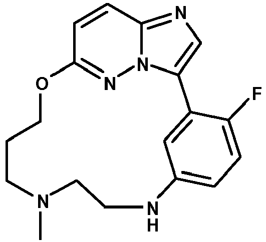

Example G44 may be prepared following general scheme 2.

### Example G45

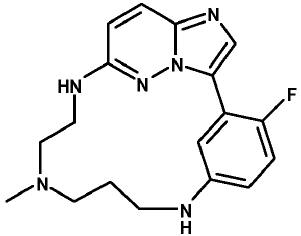

Example G45 may be prepared following general scheme 1.

### Example G46

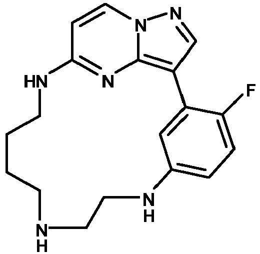

Example G46 may be prepared following general scheme 1 and according to the procedures illustrated above for the preparation of example D1.

### Example G47

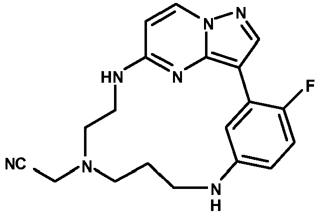

Example G47 may be prepared following general scheme 1.

### Example G48

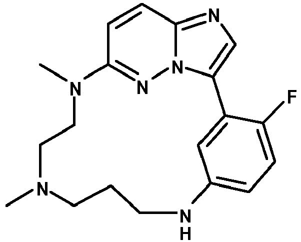

Example G48 may be prepared following general scheme 1.

### Example G49

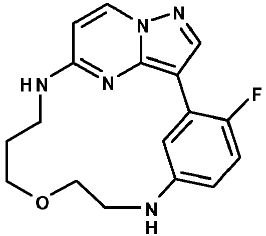

Example G49 may be prepared following general scheme 1.

### Example G50

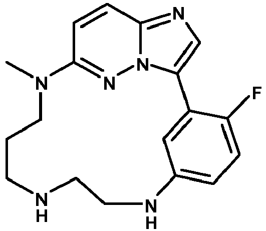

Example G50 may be prepared following general scheme 1.

The compounds in Table 1 were prepared according to one of the procedures described above.

**Table 1**

| | |
|---|---|
| 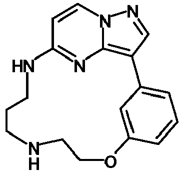 | 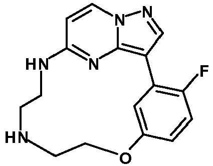 |
| Compound A33, Example A18 | Compound C1, Example C1 |
| 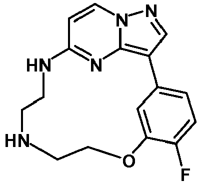 | 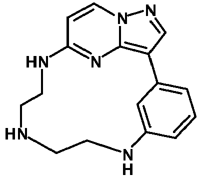 |
| Compound C2, Example C2 | Compound D1, Example D1 |
| 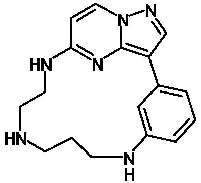 | 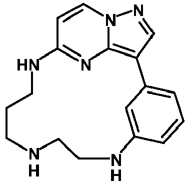 |
| Compound D3, Example D3 | Compound D4, Example D4 |
| 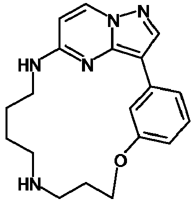 | 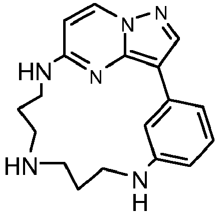 |
| Compound D5, Example D5 | Compound G1, Example G1 |
| 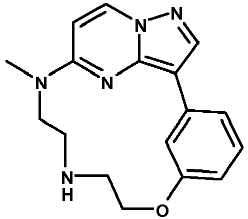 | 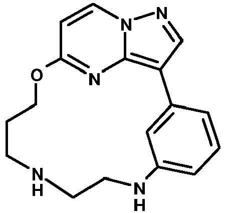 |
| Compound G2, Example G2 | Compound G3, Example G3 |
| 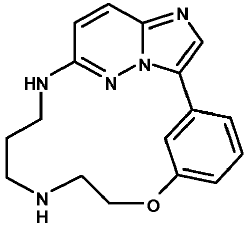 | 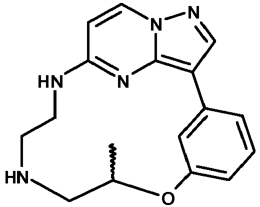 |
| Compound G4, Example G4 | Compound G5, Example G5 |
| 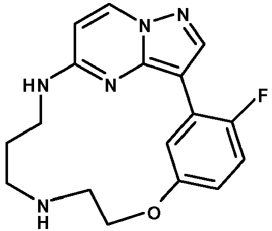 | 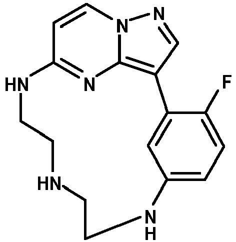 |
| Compound G6, Example G6 | Compound G7, Example G7 |
| 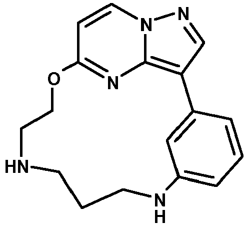 | 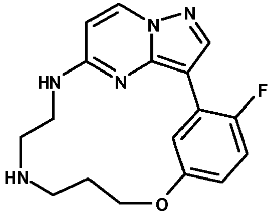 |
| Compound G8, Example G8 | Compound G10, Example G10 |
| 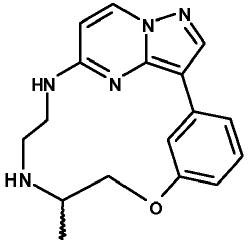 | 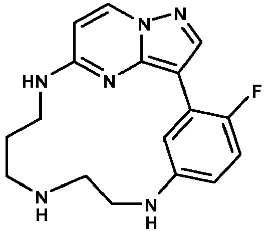 |
| Compound G11, Example G11 | Compound G12, Example G12 |
| 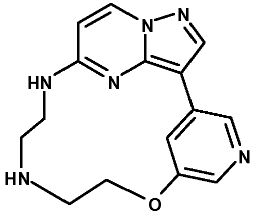 | 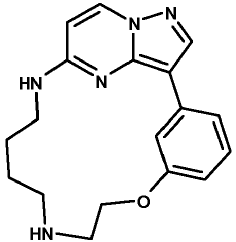 |
| Compound G13, Example G13 | Compound G14, Example G14 |
| 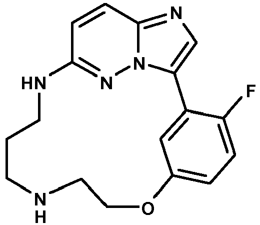 | 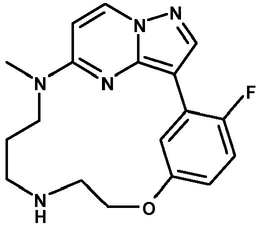 |
| Compound G15, Example G15 | Compound G16, Example G16 |
| 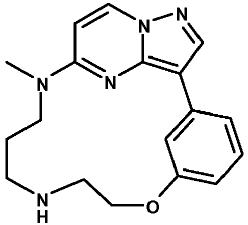 | 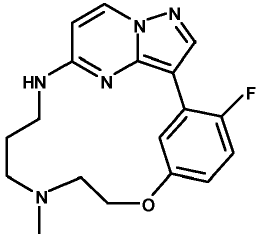 |
| Compound G17, Example G17 | Compound G18, Example G18 |
| 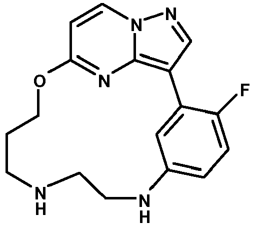 | 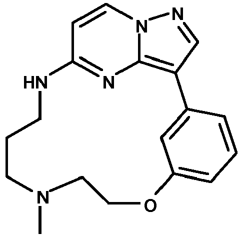 |
| Compound G19, Example G19 | Compound G20, Example G20 |
| 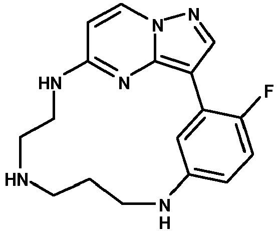 | 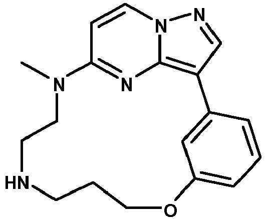 |
| Compound G21, Example G21 | Compound G22, Example G22 |
| 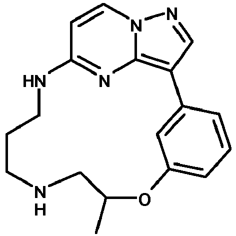 | 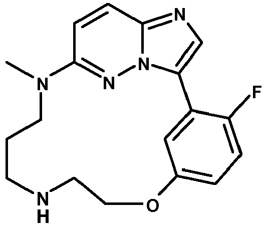 |
| Compound G23, Example G23 | Compound G24, Example G24 |
| 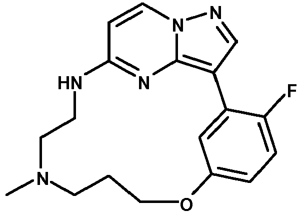 | 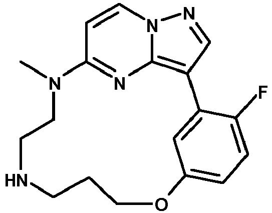 |
| Compound G25, Example G25 | Compound G26, Example G26 |
| 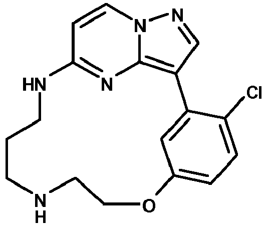 | 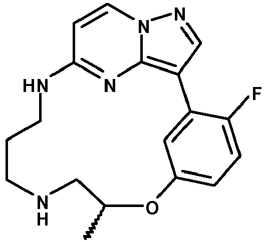 |
| Compound G27, Example G27 | Compound G28, Example G28 |
| 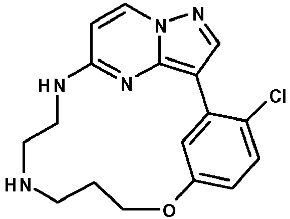 | 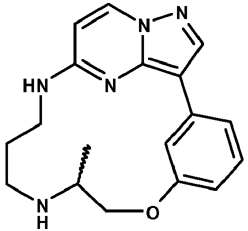 |
| Compound G30, Example G30 | Compound G31, Example G31 |
| 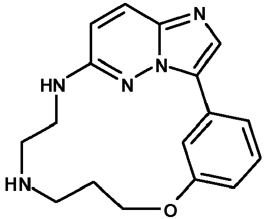 | 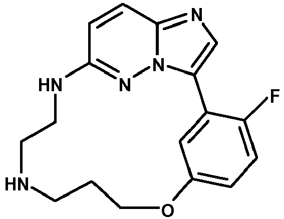 |
| Compound G32, Example G32 | Compound G33, Example G33 |
| 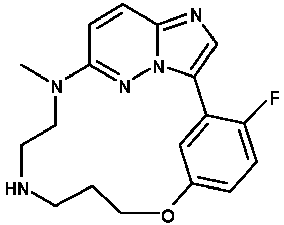 | 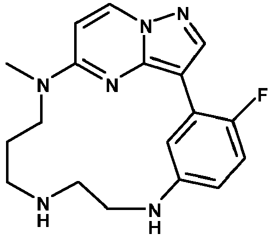 |
| Compound G34, Example G34 | Compound G35, Example G35 |
| 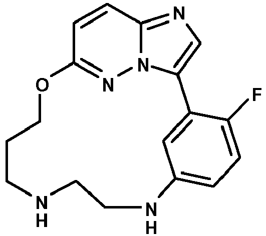 | 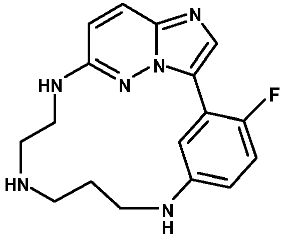 |
| Compound G36, Example G36 | Compound G37, Example G37 |
| 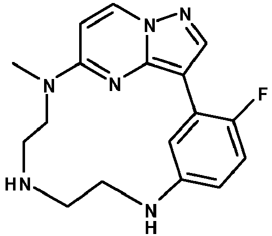 | 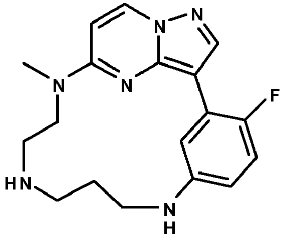 |
| Compound G38, Example G38 | Compound G39, Example G39 |
| 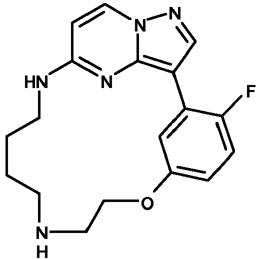 | 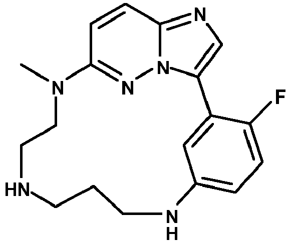 |
| Compound G40, Example G40 | Compound G41, Example G41 |
| 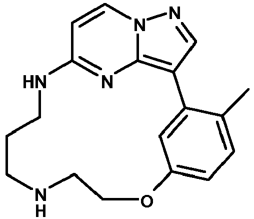 | 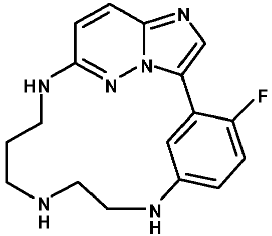 |
| Compound G42, Example G42 | Compound G43, Example G43 |
| 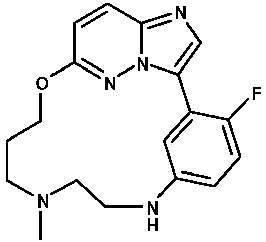 | 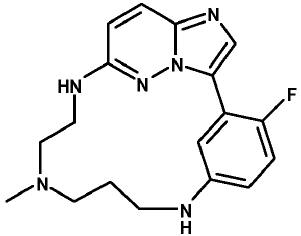 |
| Compound G44, Example G44 | Compound G45, Example G45 |
| 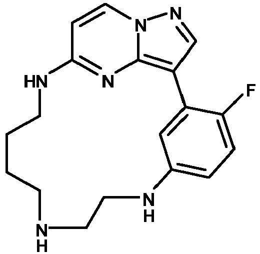 | 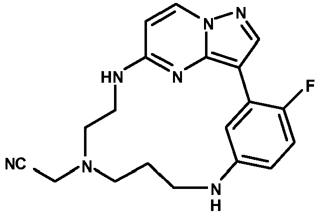 |
| Compound G46, Example G46 | Compound G47, Example G47 |
| 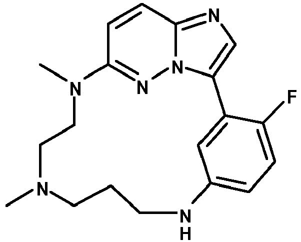 | 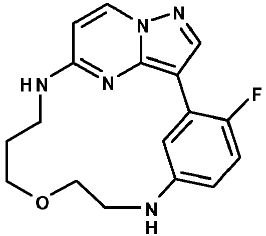 |
| Compound G48, Example G48 | Compound G49, Example G49 |
| 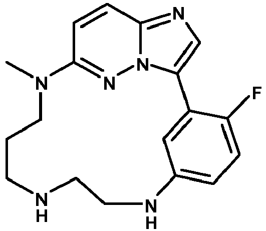 | |
| Compound G50, Example G50 | |

### Compound identification

### Melting points

For the melting point determination of the compounds of the present invention, the following method was used.

### Melting point method

For a number of compounds, melting points (m.p.) were determined in open capillary tubes on a Mettler FP62 apparatus. Melting points were measured with a temperature ranging from 50°C to 300°C , using a gradient of 10 °C/minute. The melting point value was read from a digital display and was not corrected.

**Table 2: Melting points**

| **COMPOUND NUMBER** | **MELTING POINT (°C)** |
|---|---|
| A33 | > 300 |
| C1 | > 300 |
| C2 | > 300 |
| D1 | > 300 |
| D3 | > 300 |
| D4 | 296,8 |
| D5 | 146,9 |
| G1 | 280,0 |
| G2 | > 300 |
| G3 | 280 |
| G4 | > 300 |
| G5 | 215 |
| G6 | 197,6 |
| G7 | > 300 |
| G8 | 276,7 |
| G10 | > 300 |
| G11 | 245,9 |
| G12 | > 300 |
| G13 | 283,3 |
| G14 | > 300 |
| G15 | ND* |
| G16 | > 300 |
| G17 | 297,5 |
| G18 | > 300 |
| G19 | 279,1 |
| G20 | ND* |
| G21 | > 300 |
| G22 | 286,4 |
| G23 | > 300 |
| G24 | > 300 |
| G25 | 168,8 |
| G26 | > 300 |
| G27 | > 300 |
| G28 | > 300 |
| G30 | > 300 |
| G31 | ND* |
| G32 | > 300 |
| G33 | > 300 |
| G34 | > 300 |
| G35 | 284,8 |
| G36 | > 300 |
| G37 | > 300 |
| G38 | 286,8 |
| G39 | > 300 |
| G40 | > 300 |
| G41 | 263,8 |
| G42 | 296,8 |
| G43 | > 300 |
| G44 | 242,3 |
| G45 | 289,1 |
| G46 | 270,4 |
| G47 | 262,6 |
| G48 | 289,4 |
| G49 | ND* |
| G50 | ND* |

| | |
|---|---|
| * Not determined | |

### LCMS

For LCMS-characterization of the compounds of the present invention, the following method was used.

### General procedure LCMS

All analyses were performed using an Agilent 6110 series LC/MSD quadrupole coupled to an Agilent 1290 series liquid chromatography (LC) system consisting of a binary pump with degasser, autosampler, thermostated column compartment and diode array detector. The mass spectrometer (MS) was operated with an atmospheric pressure electro-spray 103onization (API-ES) source in positive ion mode. The capillary voltage was set to 3000 V, the fragmentor voltage to 70 V and the quadrupole temperature was maintained at 100°C. The drying gas flow and temperature values were 12.0 L/min and 350°C respectively. Nitrogen was used as the nebulizer gas, at a pressure of 35 psig. Data acquisition was performed with Agilent Chemstation software.

### LCMS method 1

In addition to the general procedure LCMS1: Analyses were carried out on a Phenomenex Kinetex C18 column (50 mm long x 2.1 mm i.d.; 1.7 µm particles) at 60°C, with a flow rate of 1.5 mL/min. A gradient elution was performed from 90% (water + 0.1% formic acid) / 10% Acetonitrile to 10% (water + 0.1% formic acid) / 90% acetonitrile in 1.50 minutes, then the final mobile phase composition was held for an additional 0.40 min. The standard injection volume was 2 µL. Acquisition ranges were set to 254 nm for the UV-PDA detector and 80-800 m/z for the MS detector.

### LCMS method 2

In addition to the general procedure LCMS1: Analyses were carried out on a YMC pack ODS-AQ C18 column (50 mm long x 4.6 mm i.d.; 3 µm particles) at 35°C, with a flow rate of 2.6 mL/min. A gradient elution was performed from 95% (water + 0.1% formic acid) / 5% Acetonitrile to 5% (water + 0.1 % formic acid) / 95% Acetonitrile in 4.80 minutes, then the final mobile phase composition was held for an additional 1.00 min. The standard injection volume was 2 µL. Acquisition ranges were set to 190-400nm for the UV-PDA detector and 100-1400 m/z for the MS detector.

**Table 3: LCMS data**

| **COMPOUND NUMBER** | **MASS (MH)⁺ PEAK** | **RETENTION TIME (min)** | **LCMS METHOD** |
|---|---|---|---|
| A33 | 310,2 | 1,753 | 2 |
| C1 | 314,1 | 1,971 | 2 |
| C2 | 314,2 | 1,906 | 2 |
| D1 | 295,2 | 1,000 | 2 |
| D3 | 309,2 | 1,600 | 2 |
| D4 | 309,2 | 1,782 | 2 |
| D5 | 338,2 | 2,030 | 2 |
| G1 | 323,2 | 0,416 | 2 |
| G2 | 310,2 | 1,971 | 2 |
| G3 | 310,2 | 1,940 | 2 |
| G4 | 310,2 | 1,154 | 2 |
| G5 | 310,2 | 1,990 | 2 |
| G6 | 327,2 | 2,023 | 2 |
| G7 | 313,2 | 1,927 | 2 |
| G8 | 310,2 | 1,708 | 2 |
| G10 | 328,2 | 2,122 | 2 |
| G11 | 310,2 | 2,045 | 2 |
| G12 | 326,2 | 1,360 | 2 |
| G13 | 311,2 | 1,690 | 2 |
| G14 | 324,2 | 1,790 | 2 |
| G15 | 328,1 | 1,180 | 2 |
| G16 | 342,2 | 1,907 | 2 |
| G17 | 324,2 | 1,113 | 2 |
| G18 | 342,2 | 1,853 | 2 |
| G19 | 328,2 | 1,860 | 2 |
| G20 | 324,2 | 1,787 | 2 |
| G21 | 327,2 | 1,587 | 2 |
| G22 | 324,1 | 1,859 | 2 |
| G23 | 324,2 | 1,827 | 2 |
| G24 | 342,2 | 1,240 | 2 |
| G25 | 342,2 | 1,980 | 2 |
| G26 | 342,2 | 1,973 | 2 |
| G27 | 344,1 | 1,907 | 2 |
| G28 | 342,2 | 1,940 | 2 |
| G30 | 344 | 2,020 | 2 |
| G31 | 324,1 | 1,887 | 2 |
| G32 | 310,2 | 1,167 | 2 |
| G33 | 328,2 | 1,273 | 2 |
| G34 | 342,2 | 1,313 | 2 |
| G35 | 341,2 | 1,913 | 2 |
| G36 | 328,2 | 1,100 | 2 |
| G37 | 327 | 1,100 | 2 |
| G38 | 327 | 1,853 | 2 |
| G39 | 341,2 | 1,700 | 2 |
| G40 | 342,1 | 1,917 | 2 |
| G41 | 341,1 | 0,359 | 2 |
| G42 | 324,1 | 1,855 | 2 |
| G43 | 327,2 | 2,768 | 2 |
| G44 | 342,2 | 1,360 | 2 |
| G45 | 341,2 | 1,120 | 2 |
| G46 | 341,2 | 1,773 | 2 |
| G47 | 366,2 | 2,330 | 2 |
| G48 | 355,3 | 1,151 | 2 |
| G49 | 328,1 | 2,004 | 2 |
| G50 | 341,1 | 1,180 | 2 |

### Kinase Activity Assay

The inhibition of LRRK2 kinase was assessed using LRRK2 recombinant protein in an *in vitro* peptide-based kinase assay.

### Protocol

A radiometric protein kinase assay (³³PanQinase^{®} Activity Assay) is used for measuring the kinase activity. All assays are performed in 96-well FlashPlates™ from Perkin Elmer in a 50 µl reaction volume. The reaction cocktail is 106ipette in 4 steps in the following order:
10 µl of non-radioactive ATP solution (in H2O)
25 µl of assay buffer/ [y-³³P]-ATP mixture
5 µl of test sample in 10% DMSO
10 µl of enzyme/substrate mixture

The assay for LRRK2 contains 70 mM HEPES-NaOH pH 7.5, 3 mM MgCl₂, 3 mM MnCl₂, 3 µM Na-orthovanadate, 1.2 mM DTT, 50 µg/ml PEG20000, ATP (0.3 µM), [γ-³³P]-ATP (approx. 4 x 1005 cpm per well), protein kinase LRRK2 (7,3 nM) and substrate (GSK3(14-27), 1,0 µg/50 µl). The kinase is obtained from Invitrogen Corporation.

The reaction cocktails were incubated at 30° C for 60 minutes. The reaction was stopped with 50 µl of 2 % (v/v) H₃PO₄, plates were aspirated and washed two times with 200 µl 0.9 % (w/v) NaCl. Incorporation of ³³Pi (counting of "cpm") was determined with a microplate scintillation counter.

### Compounds

The compounds are dissolved to 10 mM in DMSO. Where needed, solutions are sonicated in a bath sonicator.

Table 2 provides the pIC₅₀ values of the compounds according to the invention, obtained using the above mentioned kinase assay.

**Table 2**

| **Compound N°** | **IC₅₀ for LRRK2** |
|---|---|
| A33 | +++ |
| C1 | +++ |
| C2 | +++ |
| D1 | +++ |
| D3 | +++ |
| D4 | +++ |
| D5 | +++ |
| G1 | +++ |
| G2 | +++ |
| G3 | +++ |
| G4 | +++ |
| G5 | +++ |
| G6 | +++ |
| G7 | +++ |
| G8 | +++ |
| G10 | +++ |
| G11 | +++ |
| G12 | +++ |
| G13 | +++ |
| G14 | +++ |
| G15 | +++ |
| G16 | +++ |
| G17 | +++ |
| G18 | +++ |
| G19 | +++ |
| G20 | +++ |
| G21 | +++ |
| G22 | +++ |
| G23 | +++ |
| G24 | +++ |
| G25 | ++ |
| G26 | +++ |
| G27 | +++ |
| G28 | +++ |
| G30 | ++ |
| G31 | +++ |
| G32 | +++ |
| G33 | +++ |
| G34 | +++ |
| G35 | +++ |
| G36 | +++ |
| G37 | +++ |
| G38 | +++ |
| G39 | +++ |
| G40 | +++ |
| G41 | +++ |
| G42 | ++ |
| G43 | +++ |
| G44 | +++ |
| G45 | +++ |
| G46 | +++ |
| G47 | +++ |
| G48 | ++ |
| G49 | +++ |
| G50 | +++ |

| | |
|---|---|
| + indicates an IC50 > 1□M, ++ indicates an IC50 of between 100 nM and 1□M, and +++ indicates an IC50 < 100nM | |

## Claims

1. A compound of Formula I or a stereoisomer, tautomer, racemic, salt, hydrate, N-oxide form, or solvate thereof, 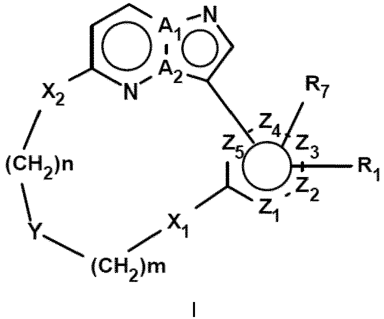 Wherein
A₁ and A₂ are selected from C and N; wherein when A₁ is C, then A₂ is N; and wherein when A₂ is C, then A₁ is N;
R₁ and R₇ are each independently selected from -H, -halo, -OH, -C₁₋₆alkyl, -O-C₁₋₆alkyl, -S-C₁₋₆alkyl, -NR₉R₁₀, -(C=O)-R₄, -SO₂-R₄, -CN, -NR₉-SO₂-R₄, -C₃₋₆cycloalkyl, and -Het₆; wherein each of said -C₁₋₆alkyl is optionally and independently substituted with from 1 to 3 substituents selected from -halo, -OH, -NR₁₁R₁₂, -O-C₁₋₆alkyl, and -S-C₁₋₆alkyl;
R₂ is selected from -H, -halo, -OH, -C₁₋₆alkyl, -O-C₁₋₆alkyl, -S-C₁₋₆alkyl, -(C=O)-C₁₋₆alkyl, -(C=O)-O-C₁₋₆alkyl, -(C=O)-NR₂₇R₂₈, -Het₃, -(C=O)-Het₃, -SO₂-C₁₋₆alkyl, and -C₃₋₆cycloalkyl; wherein each of said -C₁₋₆alkyl is optionally and independently substituted with from 1 to 3 substituents selected from -halo, -OH, -O-C₁₋₆alkyl, -S-C₁₋₆alkyl, -Het₃, -Ar₂, and -NR₁₃R₁₄;
R₃ is selected from -H, -halo, -OH, -C₁₋₆alkyl, -O-C₁₋₆alkyl, -S-C₁₋₆alkyl, -(C=O)-C₁₋₆alkyl, -(C=O)-O-C₁₋₆alkyl, -Het₂, -C₃₋₆cycloalkyl -(C=O)-Het₂, -(C=O)-NR₂₉R₃₀, and -SO₂-C₁₋₆alkyl; wherein each of said -C₁₋₆alkyl is optionally and independently substituted with from 1 to 3 substituents selected from -halo, -OH, -O-C₁₋₆alkyl, -S-C₁₋₆alkyl, -NR₁₅R₁₆, -Het₂, and -Ar₃;
R₄ is independently selected from -halo, -OH, -C₁₋₆alkyl, -O-C₁₋₆alkyl, -S-C₁₋₆alkyl, -NR₁₇R₁₈, and -Het₄;
R₅ is selected from -H, -C₁₋₆alkyl, -C₃₋₆cycloalkyl; wherein each of said C₁₋₆alkyl or -C₃₋₆cycloalkyl is optionally and independently substituted with from 1 to 3 substituents selected from -halo, -OH, -OC₁₋₆alkyl, -SC₁₋₆alkyl, -Het₅, -CN and -NR₃₁R₃₂;
R₆ is selected from -H, -OH, -halo, -C₁₋₆alkyl, -O-C₁₋₆alkyl, -S-C₁₋₆alkyl, -NR₃₃R₃₄, and -Het₈;
R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀,R₃₁, R₃₂, R₃₃, R₃₄, R₃₇ and R₃₈ are each independently selected from -H, =O, -C₁₋₆alkyl, and -Het₁; wherein each of said -C₁₋₆alkyl is optionally and independently substituted with from 1 to 3 substituents selected from -halo, -OH, -O-C₁₋₆alkyl, -S-C₁₋₆alkyl, -NR₃₅R₃₆, -Het₇, and -Ar₄;
R₃₅ and R₃₆ are each independently selected from -H, =O, and -C₁₋₆alkyl; wherein each of said -C₁₋₆alkyl is optionally and independently substituted with from 1 to 3 substituents selected from -halo, -OH, -O-C₁₋₆alkyl, and -S-C₁₋₆alkyl;
X₁ is selected from -C₁₋₆alkyl-, -O-C₁₋₆alkyl-, -S-C₁₋₆alkyl-, -(C=O)-, -NR₃-(C=O)-, -C₁₋₆alkyl-NR₃-, -NR₃-, -(C=O)-, -NR₃-(C=O)-NR₃₇-, -NR₃-C₁₋₆alkyl-, -NR₃-SO₂-, -NR₃(C=O)-C₁₋₆alkyl-, -(C=O)-NR₃-C₁₋₆alkyl-, -O-C₁₋₆alkyl-O-C₁₋₆alkyl- and -C₁₋₆alkyl-NR₃-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl, and -NR₂₃R₂₄
X₂ is selected from -C₁₋₆alkyl-, -O-C₁₋₆alkyl-, -S-C₁₋₆alkyl-, -(C=O)-, -NR₂-(C=O)-, -C₁₋₆alkyl-NR₂-, -NR₂-, -(C=O)-, -NR₂-(C=O)-NR₃₈-, -NR₂-C₁₋₆alkyl-, -NR₂-SO₂-, -NR₂-(C=O)-C₁₋₆alkyl-, -(C=O)-NR₂-C₁₋₆alkyl-, -O-C₁₋₆alkyl-O-C₁₋₆alkyl- and -C₁₋₆alkyl-NR₂-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl and -NR₂₅R₂₆;
Y is selected from a direct bond, -CHR₆-, -O-, -S-, and -NR₅-;
Ar₂, Ar₃, and Ar₄ are each independently a 5- or 6-membered aromatic heterocycle optionally comprising 1 or 2 heteroatoms selected from O, N and S; wherein each of said Ar₂, Ar₃, and Ar₄ is optionally and independently substituted with from 1 to 3 substituents selected from -NR₁₉R₂₀, -C₁₋₆alkyl, -O-C₁₋₆alkyl, and -S-C₁₋₆alkyl;
Het₁, Het₂, Het₃, Het₄, Het₅, Het₆, Het₇ and Het₈ are each independently a 5- or 6-membered monocyclic heterocycle having from 1 to 3 heteroatoms selected from O, N and S, wherein each of said Het₁, Het₂, Het₃, Het₄, Het₅, Het₆, Het₇ and Het₈ is optionally substituted with from 1 to 3 substituents selected from -C₁₋₆alkyl, -O-C₁₋₆alkyl, -S-C₁₋₆alkyl, and -NR₂₁R₂₂; wherein each of said -C₁₋₆alkyl is optionally and independently substituted with from 1 to 3 -halo;
Z₁, Z₂, Z₃, Z₄ and Z₅ are each independently selected from C and N;
m and n are each independently 0, 1, 2, 3, or 4.
for use in the prevention and/or treatment of a LRRK2-kinase associated disease.

2. A compound of Formula I or a stereoisomer, tautomer, racemic, salt, hydrate, N-oxide form, or solvate thereof, according to claim 1, wherein
A₁ is C and A₂ is N
R₁ and R₇ are each independently selected from -H, -halo, -OH, -C₁₋₆alkyl, -O-C₁₋₆alkyl, -S-C₁₋₆alkyl, -NR₉R₁₀, -(C=O)-R₄, -SO₂-R₄, -CN, -NR₉-SO₂-R₄, -C₃₋₆cycloalkyl, and -Het₆; wherein each of said -C₁₋₆alkyl is optionally and independently substituted with from 1 to 3 substituents selected from -halo, -OH, -NR₁₁R₁₂, -O-C₁₋₆alkyl, and -S-C₁₋₆alkyl;
R₂ is selected from -H, -halo, -OH, -C₁₋₆alkyl, -O-C₁₋₆alkyl, -S-C₁₋₆alkyl, -(C=O)-C₁₋₆alkyl, -(C=O)-O-C₁₋₆alkyl, -(C=O)-NR₂₇R₂₈, -Het₃, -(C=O)-Het₃, -SO₂-C₁₋₆alkyl, and -C₃₋₆cycloalkyl; wherein each of said -C₁₋₆alkyl is optionally and independently substituted with from 1 to 3 substituents selected from -halo, -OH, -O-C₁₋₆alkyl, -S-C₁₋₆alkyl, -Het₃, -Ar₂, and -NR₁₃R₁₄;
R₃ is selected from -H, -halo, -OH, -C₁₋₆alkyl, -O-C₁₋₆alkyl, -S-C₁₋₆alkyl, -(C=O)-C₁₋₆alkyl, -(C=O)-O-C₁₋₆alkyl, -Het₂, -C₃₋₆cycloalkyl -(C=O)-Het₂, -(C=O)-NR₂₉R₃₀, and -SO₂-C₁₋₆alkyl; wherein each of said -C₁₋₆alkyl is optionally and independently substituted with from 1 to 3 substituents selected from -halo, -OH, -O-C₁₋₆alkyl, -S-C₁₋₆alkyl, -NR₁₅R₁₆, -Het₂, and -Ar₃;
R₄ is independently selected from -halo, -OH, -C₁₋₆alkyl, -O-C₁₋₆alkyl, -S-C₁₋₆alkyl, -NR₁₇R₁₈, and -Het₄;
R₅ is selected from -H, -C₁₋₆alkyl, -C₃₋₆cycloalkyl; wherein each of said C₁₋₆alkyl or -C₃₋₆cycloalkyl is optionally and independently substituted with from 1 to 3 substituents selected from -halo, -OH, -OC₁₋₆alkyl, -SC₁₋₆alkyl, -Het₅, -CN and -NR₃₁R₃₂;
R₆ is selected from -H, -OH, -halo, -C₁₋₆alkyl, -O-C₁₋₆alkyl, -S-C₁₋₆alkyl, -NR₃₃R₃₄, and -Het₈;
R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀,R₃₁, R₃₂, R₃₃, R₃₄, R₃₇ and R₃₈ are each independently selected from -H, =O, -C₁₋₆alkyl, and -Het₁; wherein each of said -C₁₋₆alkyl is optionally and independently substituted with from 1 to 3 substituents selected from -halo, -OH, -O-C₁₋₆alkyl, -S-C₁₋₆alkyl, -NR₃₅R₃₆, -Het₇, and -Ar₄;
R₃₅ and R₃₆ are each independently selected from -H, =O and -C₁₋₆alkyl; wherein each of said -C₁₋₆alkyl is optionally and independently substituted with from 1 to 3 substituents selected from -halo, -OH, -O-C₁₋₆alkyl, and -S-C₁₋₆alkyl;
X₁ is selected from -C₁₋₆alkyl-, -O-C₁₋₆alkyl-, -S-C₁₋₆alkyl-, -(C=O)-, -NR₃-(C=O)-, -C₁₋₆alkyl-NR₃-, -NR₃-, -(C=O)-, -NR₃-(C=O)-NR₃₇-, -NR₃-C₁₋₆alkyl-, -NR₃-SO₂-, -NR₃-(C=O)-C₁₋₆alkyl-, -(C=O)-NR₃-C₁₋₆alkyl-, -O-C₁₋₆alkyl-O-C₁₋₆alkyl- and -C₁₋₆alkyl-NR₃-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl, and -NR₂₃R₂₄
X₂ is selected from -C₁₋₆alkyl-, -O-C₁₋₆alkyl-, -S-C₁₋₆alkyl-, -(C=O)-, -NR₂-(C=O)-, -C₁₋₆alkyl-NR₂-, -NR₂-, -(C=O)-, -NR₂-(C=O)-NR₃₈-, -NR₂-C₁₋₆alkyl-, -NR₂-SO₂-, -NR₂-(C=O)-C₁₋₆alkyl-, -(C=O)-NR₂-C₁₋₆alkyl-, -O-C₁₋₆alkyl-O-C₁₋₆alkyl- and -C₁₋₆alkyl-NR₂-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl and -NR₂₅R₂₆;
Y is selected from a direct bond, -CHR₆-, -O-, -S-, and -NR₅-;
Ar₂, Ar₃, and Ar₄ are each independently a 5- or 6-membered aromatic heterocycle optionally comprising 1 or 2 heteroatoms selected from O, N and S; wherein each of said Ar₂, Ar₃, and Ar₄ is optionally and independently substituted with from 1 to 3 substituents selected from -NR₁₉R₂₀, -C₁₋₆alkyl, -O-C₁₋₆alkyl, and -S-C₁₋₆alkyl;
Het₁, Het₂, Het₃, Het₄, Het₅, Het₆, Het₇ and Het₈ are each independently a 5-or 6-membered monocyclic heterocycle having from 1 to 3 heteroatoms selected from O, N and S, wherein each of said Het₁, Het₂, Het₃, Het₄, Het₅, Het₆, Het₇ and Het₈ is optionally substituted with from 1 to 3 substituents selected from -C₁₋₆alkyl, -O-C₁₋₆alkyl, -S-C₁₋₆alkyl, and -NR₂₁R₂₂; wherein each of said -C₁₋₆alkyl is optionally and independently substituted with from 1 to 3 -halo;
Z₁, Z₂, Z₃, Z₄ and Z₅ are each independently selected from C and N;
m and n are each independently 0, 1, 2, 3, or 4.
for use in the prevention and/or treatment of a LRRK2-kinase associated disease.

3. A compound of Formula I or a stereoisomer, tautomer, racemic, salt, hydrate, N-oxide form, or solvate thereof, according to claim 1, wherein
A₁ is N and A₂ is C
A₁ and A₂ are selected from C and N; wherein when A₁ is C, then A₂ is N; and wherein when A₂ is C, then A₁ is N;
R₁ and R₇ are each independently selected from -H, -halo, -OH, -C₁₋₆alkyl, -O-C₁₋₆alkyl, -S-C₁₋₆alkyl, -NR₉R₁₀, -(C=O)-R₄, -SO₂-R₄, -CN, -NR₉-SO₂-R₄, -C₃₋₆cycloalkyl, and -Het₆; wherein each of said -C₁₋₆alkyl is optionally and independently substituted with from 1 to 3 substituents selected from -halo, -OH, -NR₁₁R₁₂, -O-C₁₋₆alkyl, and -S-C₁₋₆alkyl;
R₂ is selected from -H, -halo, -OH, -C₁₋₆alkyl, -O-C₁₋₆alkyl, -S-C₁₋₆alkyl, -(C=O)-C₁₋₆alkyl, -(C=O)-O-C₁₋₆alkyl, -(C=O)-NR₂₇R₂₈, -Het₃, -(C=O)-Het₃, -SO₂-C₁₋₆alkyl, and -C₃₋₆cycloalkyl; wherein each of said -C₁₋₆alkyl is optionally and independently substituted with from 1 to 3 substituents selected from -halo, -OH, -O-C₁₋₆alkyl, -S-C₁₋₆alkyl, -Het₃, -Ar₂, and -NR₁₃R₁₄;
R₃ is selected from -H, -halo, -OH, -C₁₋₆alkyl, -O-C₁₋₆alkyl, -S-C₁₋₆alkyl, -(C=O)-C₁₋₆alkyl, -(C=O)-O-C₁₋₆alkyl, -Het₂, -C₃₋₆cycloalkyl -(C=O)-Het₂, -(C=O)-NR₂₉R₃₀, and -SO₂-C₁₋₆alkyl; wherein each of said -C₁₋₆alkyl is optionally and independently substituted with from 1 to 3 substituents selected from -halo, -OH, -O-C₁₋₆alkyl, -S-C₁₋₆alkyl, -NR₁₅R₁₆, -Het₂, and -Ar₃;
R₄ is independently selected from -halo, -OH, -C₁₋₆alkyl, -O-C₁₋₆alkyl, -S-C₁₋₆alkyl, -NR₁₇R₁₈, and -Het₄;
R₅ is selected from -H, -C₁₋₆alkyl, -C₃₋₆cycloalkyl; wherein each of said C₁₋₆alkyl or -C₃₋₆cycloalkyl is optionally and independently substituted with from 1 to 3 substituents selected from -halo, -OH, -OC₁₋₆alkyl, -SC₁₋₆alkyl, -Het₅, -CN and -NR₃₁R₃₂;
R₆ is selected from -H, -OH, -halo, -C₁₋₆alkyl, -O-C₁₋₆alkyl, -S-C₁₋₆alkyl, -NR₃₃R₃₄, and -Het₈;
R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀,R₃₁, R₃₂, R₃₃, R₃₄, R₃₇ and R₃₈ are each independently selected from -H, =O, -C₁₋₆alkyl, and -Het₁; wherein each of said -C₁₋₆alkyl is optionally and independently substituted with from 1 to 3 substituents selected from -halo, -OH, -O-C₁₋₆alkyl, -S-C₁₋₆alkyl, -NR₃₅R₃₆, -Het₇, and -Ar₄;
R₃₅ and R₃₆ are each independently selected from -H, =O and -C₁₋₆alkyl; wherein each of said -C₁₋₆alkyl is optionally and independently substituted with from 1 to 3 substituents selected from -halo, -OH, -O-C₁₋₆alkyl, and -S-C₁₋₆alkyl;
X₁ is selected from -C₁₋₆alkyl-, -O-C₁₋₆alkyl-, -S-C₁₋₆alkyl-, -(C=O)-, -NR₃-(C=O)-, -C₁₋₆alkyl-NR₃-, -NR₃-, -(C=O)-, -NR₃-(C=O)-NR₃₇-, -NR₃-C₁₋₆alkyl-, -NR₃-SO₂-, -NR₃-(C=O)-C₁₋₆alkyl-, -(C=O)-NR₃-C₁₋₆alkyl-, -O-C₁₋₆alkyl-O-C₁₋₆alkyl- and -C₁₋₆alkyl-NR₃-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl, and -NR₂₃R₂₄
X₂ is selected from -C₁₋₆alkyl-, -O-C₁₋₆alkyl-, -S-C₁₋₆alkyl-, -(C=O)-, -NR₂-(C=O)-, -C₁₋₆alkyl-NR₂-, -NR₂-, -(C=O)-, -NR₂-(C=O)-NR₃₈-, -NR₂-C₁₋₆alkyl-, -NR₂-SO₂-, -NR₂-(C=O)-C₁₋₆alkyl-, -(C=O)-NR₂-C₁₋₆alkyl-, -O-C₁₋₆alkyl-O-C₁₋₆alkyl- and -C₁₋₆alkyl-NR₂-C₁₋₆alkyl-; wherein each of said -C₁₋₆alkyl is optionally and independently substituted with from 1 to 3 substituents selected from -halo, -OH, -C₁₋₆alkyl, -O-C₁₋₆alkyl, -S-C₁₋₆alkyl, -phenyl and -NR₂₅R₂₆;
Y is selected from a direct bond, -CHR₆-, -O-, -S-, and -NR₅-;
Ar₂, Ar₃, and Ar₄ are each independently a 5- or 6-membered aromatic heterocycle optionally comprising 1 or 2 heteroatoms selected from O, N and S; wherein each of said Ar₂, Ar₃, and Ar₄ is optionally and independently substituted with from 1 to 3 substituents selected from -NR₁₉R₂₀, -C₁₋₆alkyl, -O-C₁₋₆alkyl, and -S-C₁₋₆alkyl;
Het₁, Het₂, Het₃, Het₄, Het₅, Het₆, Het₇ and Het₈ are each independently a 5-or 6-membered monocyclic heterocycle having from 1 to 3 heteroatoms selected from O, N and S, wherein each of said Het₁, Het₂, Het₃, Het₄, Het₅, Het₆, Het₇ and Het₈ is optionally substituted with from 1 to 3 substituents selected from -C₁₋₆alkyl, -O-C₁₋₆alkyl, -S-C₁₋₆alkyl, and -NR₂₁R₂₂; wherein each of said -C₁₋₆alkyl is optionally and independently substituted with from 1 to 3 -halo;
Z₁, Z₂, Z₃, Z₄ and Z₅ are each independently selected from C and N;
m and n are each independently 0, 1, 2, 3, or 4.
for use in the prevention and/or treatment of a LRRK2-kinase associated disease.

4. A compound of Formula I or a stereoisomer, tautomer, racemic, salt, hydrate, N-oxide form, or solvate thereof, according to claim 1, wherein
A₁ and A₂ are selected from C and N; wherein when A₁ is C, then A₂ is N; and wherein when A₂ is C, then A₁ is N;
R₁ and R₇ are each independently selected from -H, -halo and -C₁₋₆alkyl;
R₂ is selected from -H and -C₁₋₆alkyl;
R₃ is -H;
R₅ is selected from -H and -C₁₋₆alkyl; wherein said -C₁₋₆alkyl may be optionally substituted with-CN
X₁ is selected from -O-C₁₋₆alkyl- and -C₁₋₆alkyl-NR₃-; wherein each of said -C₁₋₆alkyl is optionally and independently substituted with from 1 to 3 substituents selected from -C₁₋₆alkyl;
X₂ is selected from -O-C₁₋₆alkyl- and -C₁₋₆alkyl-NR₂;
Y is selected from -O- and -NR₅-;
Z₁, Z₂, Z₃, Z₄ and Z₅ are each independently selected from C and N;
m and n are each independently 0, 1, 2, 3, or 4.
for use in the prevention and/or treatment of a LRRK2-kinase associated disease.

5. A compound of Formula I or a stereoisomer, tautomer, racemic, salt, hydrate, N-oxide form, or solvate thereof, according to claim 1, wherein
A₁ is N and A₂ is C
R₁ and R₇ are each independently selected from -H, -halo and -C₁₋₆alkyl;
R₂ is selected from -H and -C₁₋₆alkyl;
R₃ is -H;
R₅ is selected from -H and -C₁₋₆alkyl; wherein said -C₁₋₆alkyl may be optionally substituted with-CN
X₁ is selected from -O-C₁₋₆alkyl- and -C₁₋₆alkyl-NR₃-; wherein each of said -C₁₋₆alkyl is optionally and independently substituted with from 1 to 3 substituents selected from -C₁₋₆alkyl;
X₂ is selected from -O-C₁₋₆alkyl- and -C₁₋₆alkyl-NR₂;
Y is selected from -O- and -NR₅-;
Z₁, Z₂, Z₃, Z₄ and Z₅ are each independently selected from C and N;
m and n are each independently 0, 1, 2, 3, or 4.
for use in the prevention and/or treatment of a LRRK2-kinase associated disease.

6. A compound of Formula I or a stereoisomer, tautomer, racemic, salt, hydrate, N-oxide form, or solvate thereof, according to claim 1, wherein
A₁ is C and A₂ is N
R₁ and R₇ are each independently selected from -H, -halo and -C₁₋₆alkyl;
R₂ is selected from -H and -C₁₋₆alkyl;
R₃ is -H;
R₅ is selected from -H and -C₁₋₆alkyl; wherein said -C₁₋₆alkyl may be optionally substituted with-CN
X₁ is selected from -O-C₁₋₆alkyl- and -C₁₋₆alkyl-NR₃-; wherein each of said -C₁₋₆alkyl is optionally and independently substituted with from 1 to 3 substituents selected from -C₁₋₆alkyl;
X₂ is selected from -O-C₁₋₆alkyl- and -C₁₋₆alkyl-NR₂;
Y is selected from -O- and -NR₅-;
Z₁, Z₂, Z₃, Z₄ and Z₅ are each independently selected from C and N;
m and n are each independently 0, 1, 2, 3, or 4.
for use in the prevention and/or treatment of a LRRK2-kinase associated disease.

7. A compound as defined in any one of claims 1 to 6 for use in the diagnosis, prevention and/or treatment of a LRRK2-kinase associated disease; wherein said compound is selected from the list comprising:

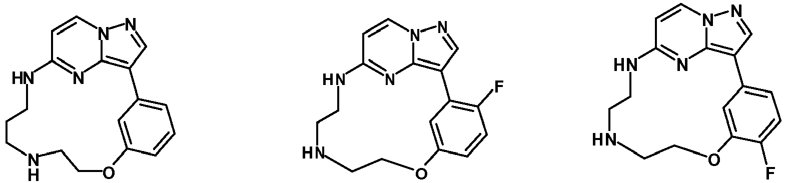

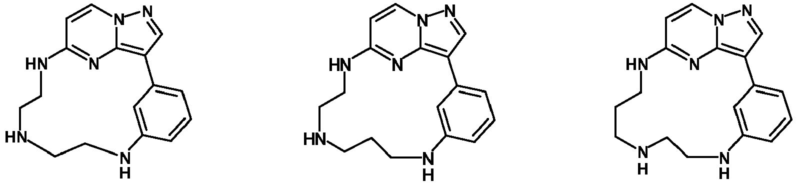

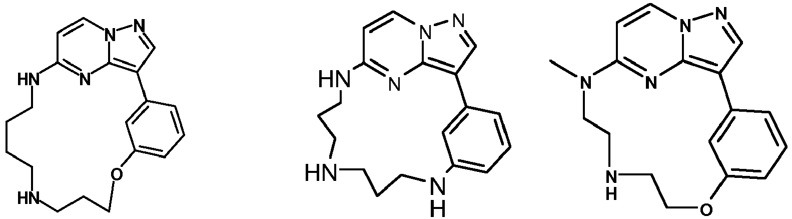

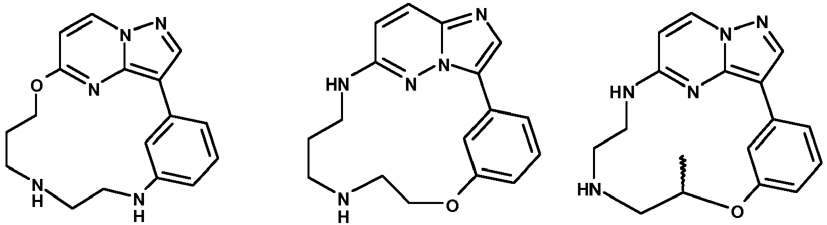

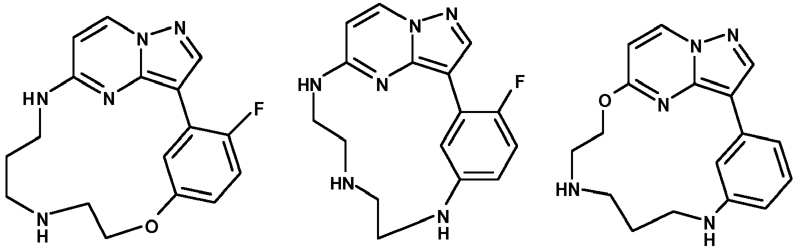

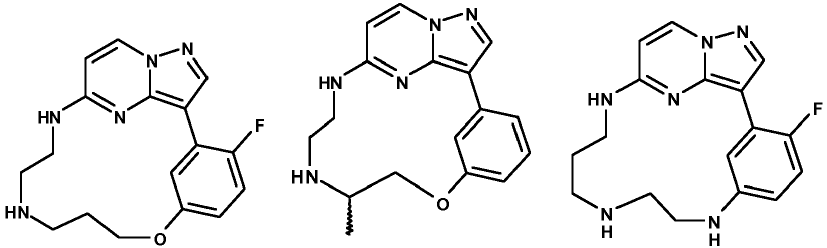

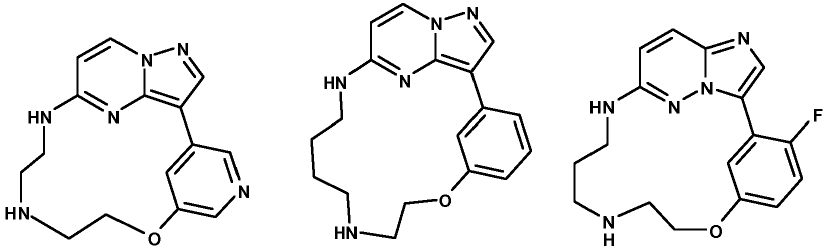

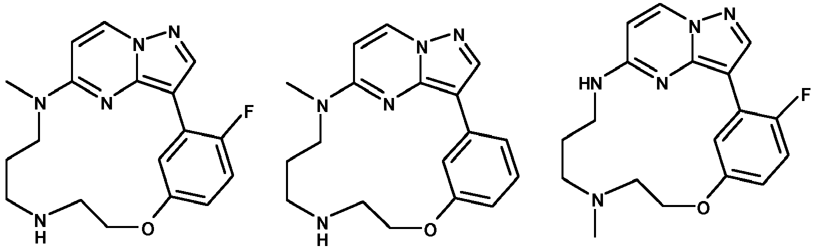

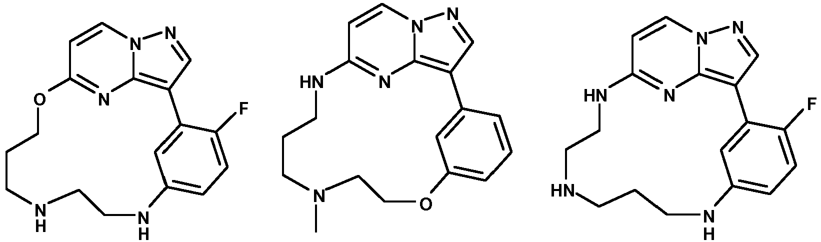

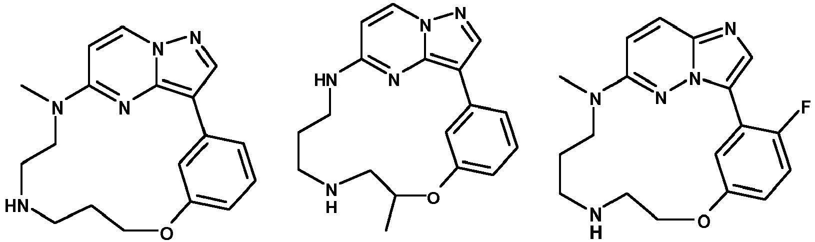

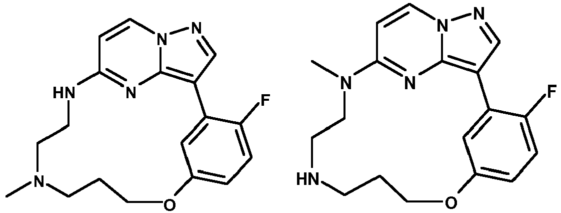

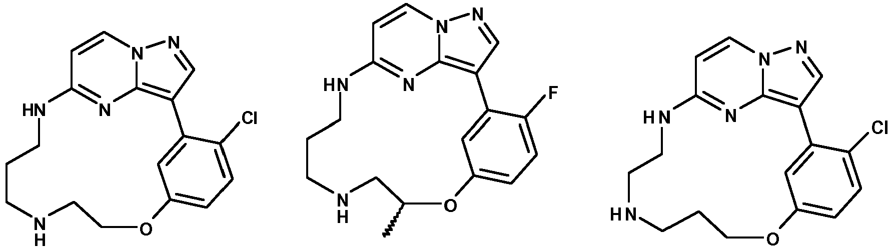

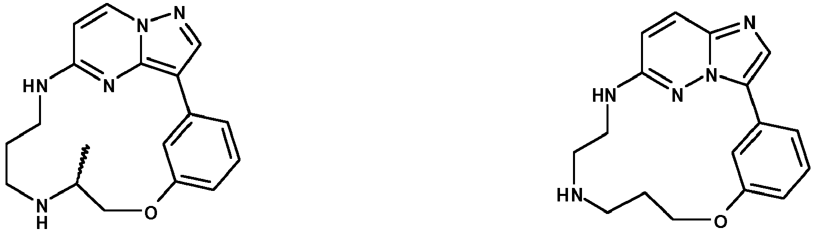

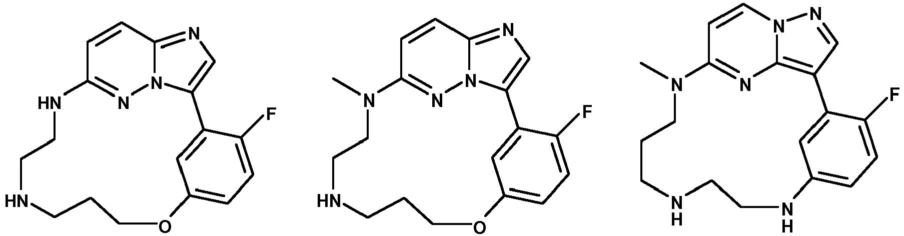

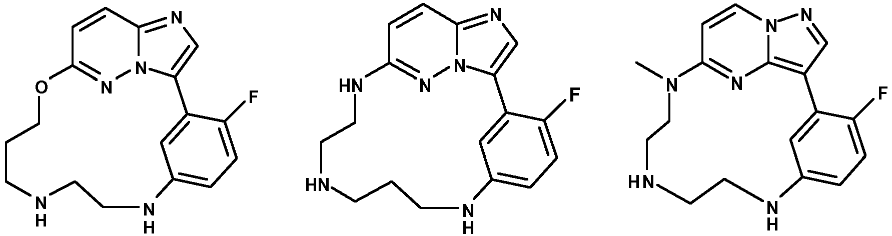

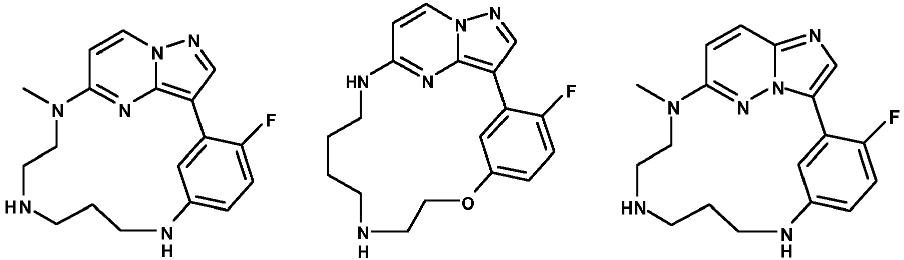

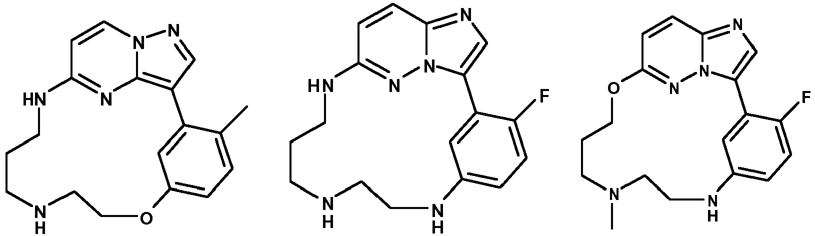

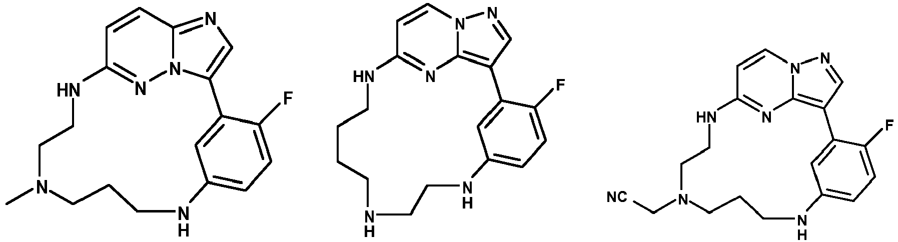

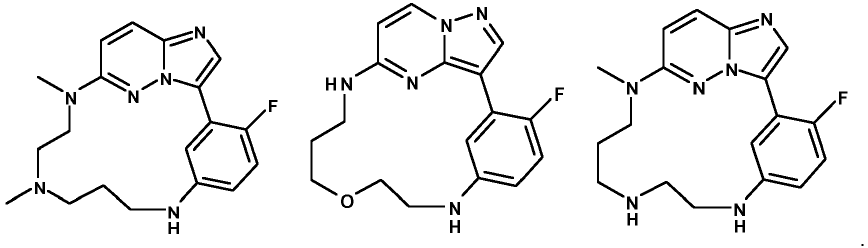

8. A compound as defined in any one of claims 1 to 7 for use in the diagnosis, prevention and/or treatment of a LRRK2-kinase associated disease; wherein the pyrazolopyrimidine or the imidazopyridazine moiety is linked to the aryl or heteroaryl moiety at position Z₄ or Z₅, in accordance with the numbering as provided in Formula I.

9. A compound as defined in any one of claims 1 to 7 for use in the diagnosis, prevention and/or treatment of a LRRK2-kinase associated disease; wherein R₁ is linked to the aryl or heteroaryl moiety at position Z₁, Z₂ or Z₃, in accordance with the numbering as provided in Formula I.

10. A compound as defined in any one of claims 1 to 7 for use in the diagnosis, prevention and/or treatment of a LRRK2-kinase associated disease; wherein the LRRK2-kinase associated disease is a neurological disorder, in particular selected from the list comprising Parkinson's disease or Alzheimer's disease.

11. A compound according to claim 1 of Formula (IIc) or a stereoisomer, tautomer, racemic, salt, hydrate, N-oxide form, or solvate thereof, 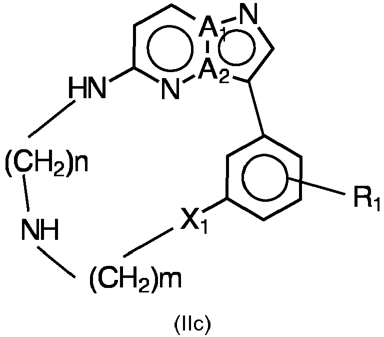 wherein
A₁ and A₂ are selected from C and N; wherein when A₁ is C, then A₂ is N; and wherein when A₂ is C, then A₁ is N;
R₁ is selected from the list comprising -H, -F, -CH₃, and -CN
X₁ is selected from the list comprising -NH- and -O-
m and n are each independently 1, 2, 3, or 4
for use in the prevention and/or treatment of a LRRK2-kinase associated disease.

12. A compound as defined in any one of claims 1 to 11 for use in the diagnosis, prevention and/or treatment of a LRRK2-kinase associated disease; wherein said compound is selected from the list comprising

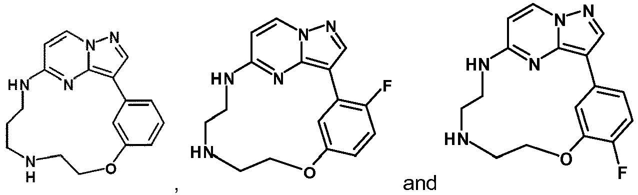

13. A pharmaceutical composition for use in the diagnosis, prevention and/or treatment of a LRRK2-kinase associated disease comprising a compound as defined in any one of claims 1 to 12.

14. A compound or a stereoisomer, tautomer, racemic, salt, hydrate, N-oxide form, or solvate thereof, according to the general formula (IIIc) 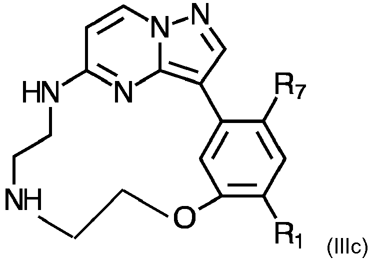 wherein
R₁ is -H and R₇ is -F; or
R₇ is -H and R₁ is -F.

15. A compound according to claim 14, wherein said compound is:

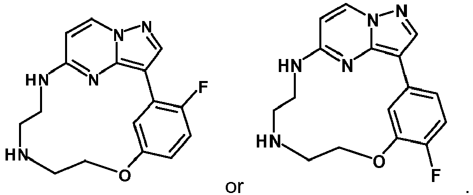

16. A compound selected from the list comprising:

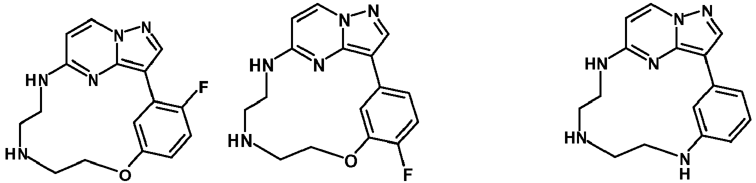

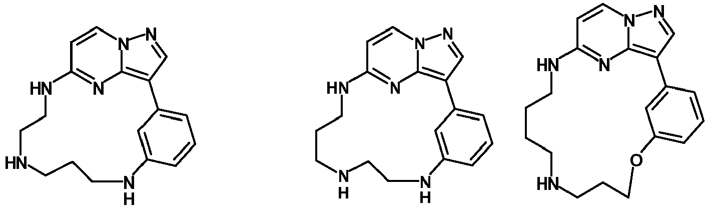

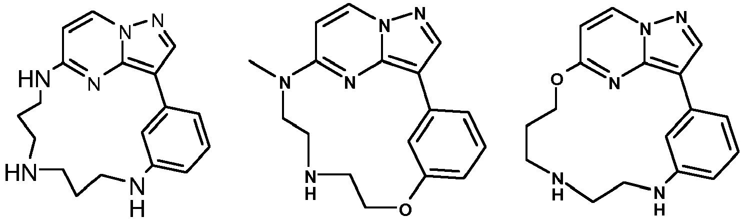

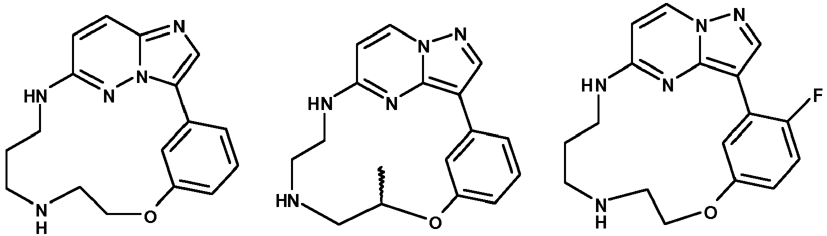

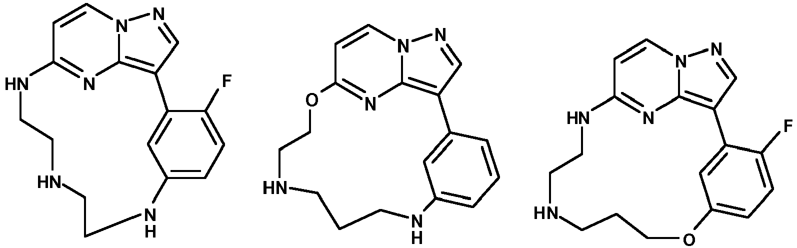

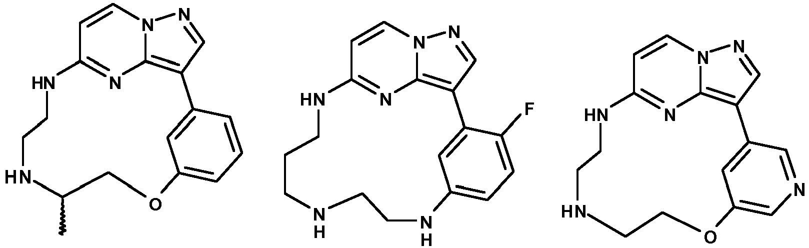

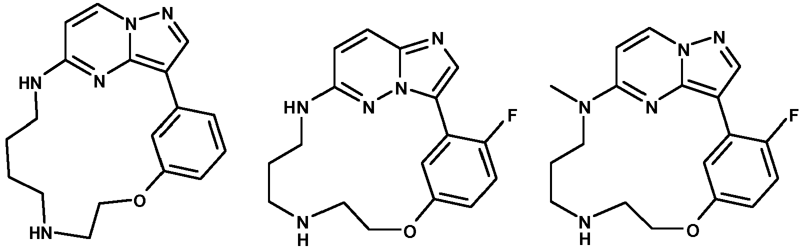

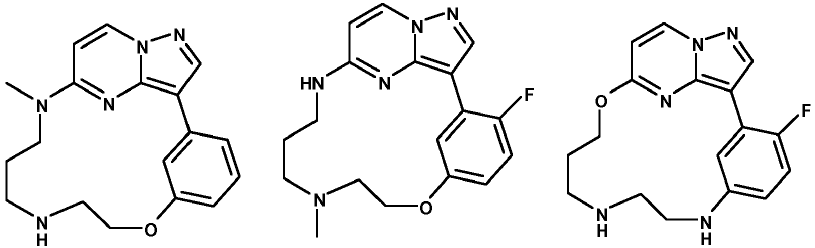

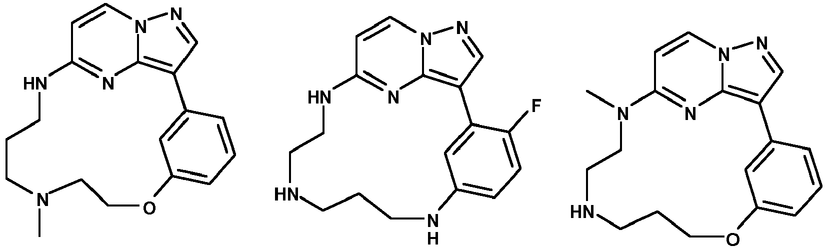

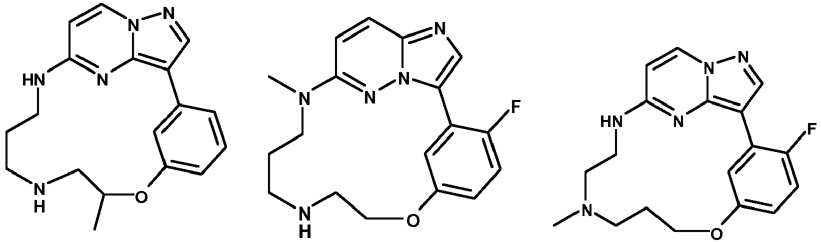

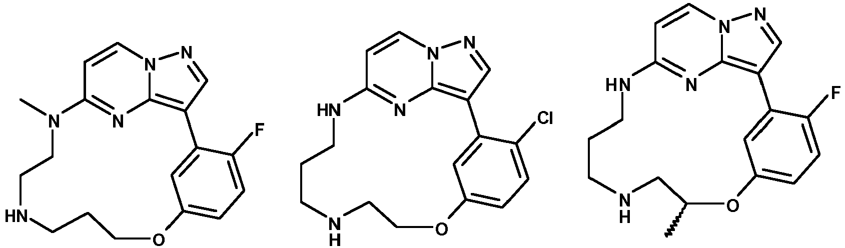

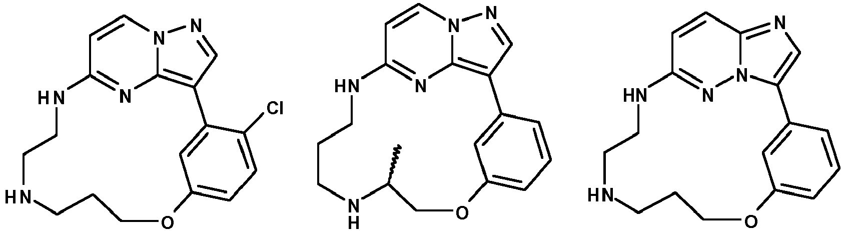

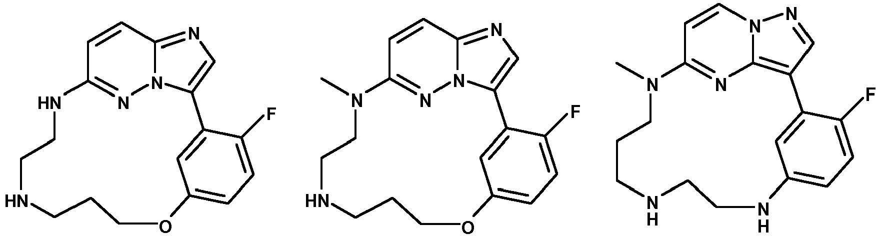

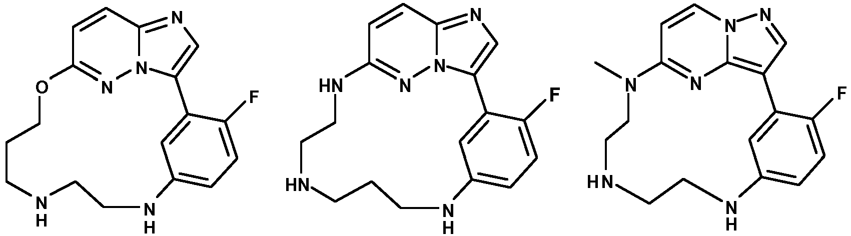

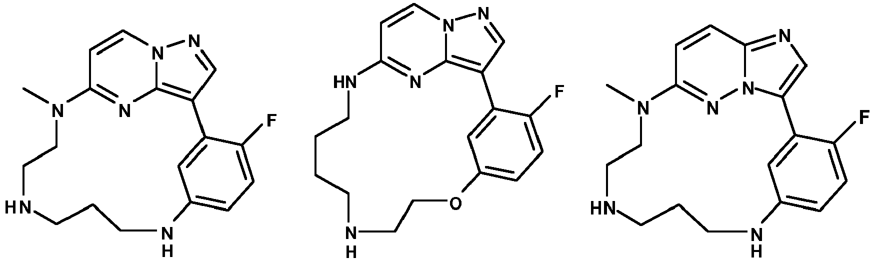

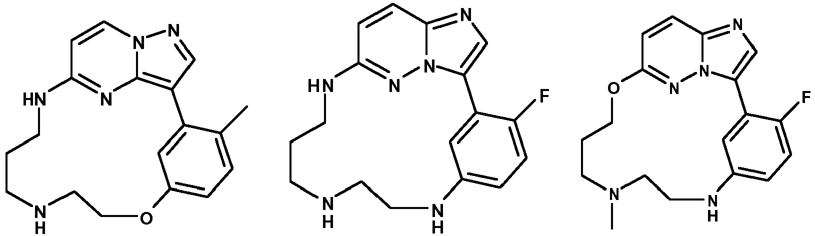

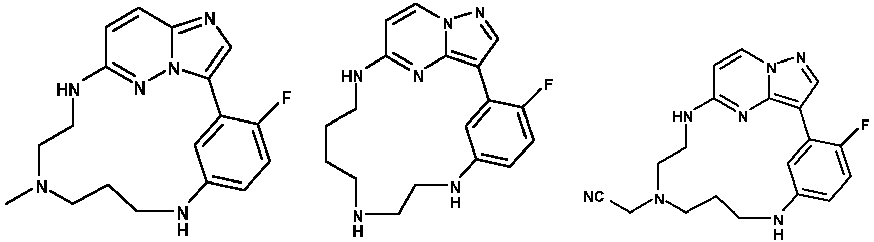

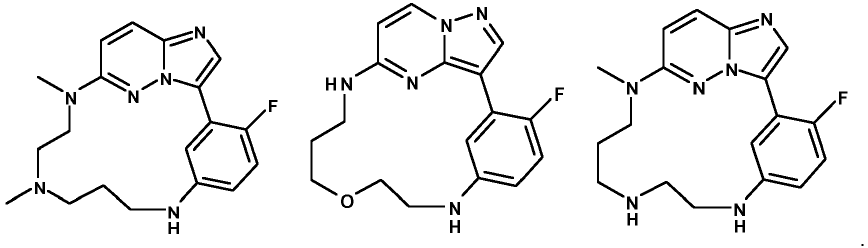

## Patentansprüche

1. Verbindung der Formel I oder ein Stereoisomer, ein Tautomer, eine racemische Mischung, ein Salz, ein Hydrat, eine N-Oxidform oder ein Solvat davon, 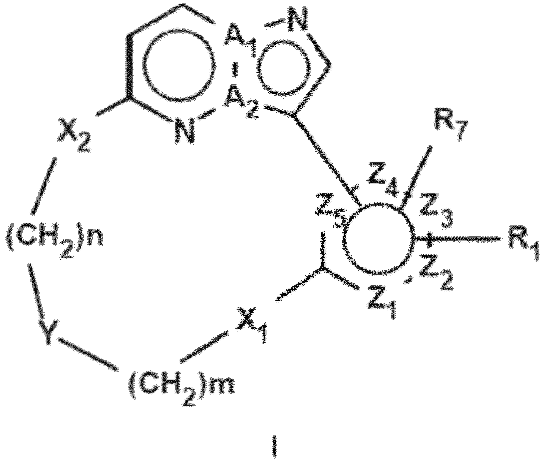 wobei
A₁ und A₂ aus C und N ausgewählt sind, wobei, wenn A₁ für C steht, A₂ für N steht, und wobei, wenn A₂ für C steht, A₁ für N steht,
R₁ und R₇ jeweils unabhängig voneinander aus -H, -Halogen, -OH, -C₁₋₆-Alkyl, -O-C₁₋₆-Alkyl, -S-C₁₋₆-Alkyl, -NR₉R₁₀, -(C=O)-R₄, -SO₂-R₄,-CN, -NR₉-SO₂-R₄, -C₃₋₆-Cycloalkyl und -Het₆ ausgewählt sind, wobei -C₁₋₆-Alkyl jeweils gegebenenfalls unabhängig durch 1 bis 3 aus-Halogen, -OH, -NR₁₁R₁₂, -O-C₁₋₆-Alkyl und -S-C₁₋₆-Alkyl ausgewählte Substituenten substituiert ist,
R₂ aus -H, -Halogen, -OH, -C₁₋₆-Alkyl, -O-C₁₋₆-Alkyl, -S-C₁₋₆-Alkyl, -(C=O)-C₁₋₆-Alkyl, -(C=O)-O-C₁₋₆-Alkyl, -(C=O)-NR₂R₂₈, -Het₃, -(C=O)-Het₃, -SO₂-C₁₋₆-Alkyl und -C₃₋₆-Cycloalkyl ausgewählt ist, wobei-C₁₋₆-Alkyl jeweils gegebenenfalls unabhängig durch 1 bis 3 aus -Halogen, -OH, -O-C₁₋₆-Alkyl, -S-C₁₋₆-Alkyl, -Het₃, -Ar₂ und -NR₁₃R₁₄ ausgewählte Substituenten substituiert ist,
R₃ aus -H, -Halogen, -OH, -C₁₋₆-Alkyl, -O-C₁₋₆-Alkyl, -S-C₁₋₆-Alkyl, -(C=O)-C₁₋₆-Alkyl, -(C=O)-O-C₁₋₆-Alkyl, -Het₂, -C₃₋₆-Cycloalkyl -(C=O)-Het₂,-(C=O)-NR₂₉R₃₀ und -SO₂-C₁₋₆-Alkyl ausgewählt ist, wobei -C₁₋₆-Alkyl jeweils gegebenenfalls unabhängig durch 1 bis 3 aus -Halogen, -OH, -O-C₁₋₆-Alkyl, -S-C₁₋₆-Alkyl, -NR₁₅R₁₆, -Het₂ und -Ar₃ ausgewählte Substituenten substituiert ist,
R₄ unabhängig aus -Halogen, -OH, -C₁₋₆-Alkyl, -O-C₁₋₆-Alkyl, -S-C₁₋₆-Alkyl, -NR₁₇R₁₈ und -Het₄ ausgewählt ist,
R₅ aus -H, -C₁₋₆-Alkyl, -C₃₋₆-Cycloalkyl ausgewählt ist, wobei C₁₋₆-Alkyl bzw. -C₃₋₆-Cycloalkyl jeweils gegebenenfalls unabhängig durch 1 bis 3 aus -Halogen, -OH, -O-C₁₋₆-Alkyl, -S-C₁₋₆-Alkyl, -Het₅, -CN und -NR₃₁R₃₂ ausgewählte Substituenten substituiert ist,
R₆ aus -H, -OH, -Halogen, -C₁₋₆-Alkyl, -O-C₁₋₆-Alkyl, -S-C₁₋₆-Alkyl, -NR₃₃R₃₄ und -Het₈ ausgewählt ist,
R₉, R₁₀, R₁₁, R₁₂, R₁₃, R₁₄, R₁₅, R₁₆, R₁₇, R₁₈, R₁₉, R₂₀,R₂₁, R₂₂, R₂₃, R₂₄, R₂₅, R₂₆, R₂₇, R₂₈, R₂₉, R₃₀, R₃₁,R₃₂, R₃₃, R₃₄, R₃₇ und R₃₈ jeweils unabhängig aus -H, =0, -C₁₋₆-Alkyl und -Het₁ ausgewählt sind, wobei-C₁₋₆-Alkyl jeweils gegebenenfalls unabhängig durch 1 bis 3 aus -Halogen, -OH, -O-C₁₋₆-Alkyl, -S-C₁₋₆-Alkyl, -NR₃₅R₃₆, -Het₇ und -Ar₄ ausgewählte Substituenten substituiert ist,
R₃₅ und R₃₆ jeweils unabhängig aus -H, =0 und -C₁₋₆-Alkyl ausgewählt sind, wobei -C₁₋₆-Alkyl jeweils gegebenenfalls unabhängig durch 1 bis 3 aus -Halogen, -OH, -O-C₁₋₆-Alkyl und -S-C₁₋₆-Alkyl ausgewählte Substituenten substituiert ist,
X₁ aus -C₁₋₆-Alkyl-, -O-C₁₋₆-Alkyl-, -S-C₁₋₆-Alkyl-, -(C=O)- -NR₃-(C=O)-, -C₁₋₆-Alkyl-NR₃-, -NR₃-, -(C=O)-, -NR₃-(C=O)-NR₃₇-, -NR₃-C₁₋₆-Alkyl-, -NR₃-SO₂-, -NR₃-(C=O)-C₁₋₆-Alkyl-, -(C=O)-NR₃-C₁₋₆-Alkyl-, -O-C₁₋₆-Alkyl-O-C₁₋₆-alkyl- und -C₁₋₆-Alkyl-NR₃-C₁₋₆-alkyl- ausgewählt ist, wobei -C₁₋₆-Alkyl jeweils gegebenenfalls unabhängig durch 1 bis 3 aus -Halogen, -OH, -C₁₋₆-Alkyl, -O-C₁₋₆-Alkyl, -S-C₁₋₆-Alkyl, -Phenyl und -NR₂₃R₂₄ ausgewählte Substituenten substituiert ist,
X₂ aus -C₁₋₆-Alkyl-, -O-C₁₋₆-Alkyl-, -S-C₁₋₆-Alkyl-, -(C=O)-, -NR₂-(C=O)-, -C₁₋₆-Alkyl-NR₂-, -NR₂-, -(C=O)-, -NR₂- (C=0) -NR₃₈-, -NR₂-C₁₋₆-Alkyl-, -NR₂-SO₂-, -NR₂-(C=O)-C₁₋₆-Alkyl-, -(C=O)-NR₂-C₁₋₆-Alkyl-, -O-C₁₋₆-Alkyl-O-C₁₋₆-alkyl- und -C₁₋₆-Alkyl-NR₂-C₁₋₆-alkyl- ausgewählt ist, wobei -C₁₋₆-Alkyl jeweils gegebenenfalls unabhängig durch 1 bis 3 aus -Halogen, -OH, -C₁₋₆-Alkyl, -O-C₁₋₆-Alkyl, -S-C₁₋₆-Alkyl, -Phenyl und -NR₂₅R₂₆ ausgewählte Substituenten substituiert ist,
Y aus einer direkten Bindung, -CHR₆-, -O-, -S-und -NR₅- ausgewählt ist,
Ar₂, Ar₃ und Ar₄ jeweils unabhängig voneinander für einen 5- oder 6-gliedrigen aromatischen Heterocyclus stehen, welcher gegebenenfalls 1 oder 2 aus 0, N und S ausgewählte Heteroatome umfasst, wobei Ar₂, Ar₃ und Ar₄ jeweils gegebenenfalls unabhängig durch 1 bis 3 aus -NR₁₉R₂₀, -C₁₋₆-Alkyl,-O-C₁₋₆-Alkyl und -S-C₁₋₆-Alkyl ausgewählte Substituenten substituiert sind,
Het₁, Het₂, Het₃, Het₄, Het₅, Het₆, Het₇ und Het₈ jeweils unabhängig voneinander für einen 5- oder 6-gliedrigen monocyclischen Heterocyclus mit 1 bis 3 aus 0, N und S ausgewählten Heteroatomen stehen, wobei Het₁, Het₂, Het₃, Het₄, Het₅, Het₆, Het₇ und Het₈ jeweils gegebenenfalls durch 1 bis 3 aus -C₁₋₆-Alkyl, -O-C₁₋₆-Alkyl, -S-C₁₋₆-Alkyl und -NR₂₁R₂₂ ausgewählte Substituenten substituiert sind, wobei -C₁₋₆-Alkyl jeweils gegebenenfalls unabhängig durch 1 bis 3 -Halogen substituiert ist,
Z₁, Z₂, Z₃, Z₄ und Z₅ jeweils unabhängig voneinander aus C und N ausgewählt sind,
m und n jeweils unabhängig voneinander für 0, 1, 2, 3 oder 4 stehen,
zur Verwendung bei der Prävention und/oder Behandlung einer mit LRRK2-Kinase assoziierten Krankheit.

2. Verbindung der Formel I oder ein Stereoisomer, ein Tautomer, eine racemische Mischung, ein Salz, ein Hydrat, eine N-Oxidform oder ein Solvat davon gemäß Anspruch 1, wobei
A₁ für C steht und A₂ für N steht,
R₁ und R₇ jeweils unabhängig voneinander aus -H, -Halogen, -OH, -C₁₋₆-Alkyl, -O-C₁₋₆-Alkyl, -S-C₁₋₆-Alkyl, -NR₉R₁₀, -(C=O)-R₄, -SO₂-R₄,-CN, -NR₉-SO₂-R₄, -C₃₋₆-Cycloalkyl und -Het₆ ausgewählt sind, wobei -C₁₋₆-Alkyl jeweils gegebenenfalls unabhängig durch 1 bis 3 aus-Halogen, -OH, -NR₁₁R₁₂, -O-C₁₋₆-Alkyl und -S-C₁₋₆-Alkyl ausgewählte Substituenten substituiert ist,
R₂ aus -H, -Halogen, -OH, -C₁₋₆-Alkyl, -O-C₁₋₆-Alkyl, -S-C₁₋₆-Alkyl, - (C=O) -C₁₋₆-Alkyl, - (C=O) -O-C₁₋₆-Alkyl, -(C=O)-NR₂₇R₂₈, -Het₃, - (C=O) -Het₃, -SO₂-C₁-₆-Alkyl und -C₃₋₆-Cycloalkyl ausgewählt ist, wobei-C₁₋₆-Alkyl jeweils gegebenenfalls unabhängig durch 1 bis 3 aus -Halogen, -OH, -O-C₁₋₆-Alkyl, -S-C₁₋₆-Alkyl, -Het₃, -Ar₂ und -NR₁₃R₁₄ ausgewählte Substituenten substituiert ist,
R₃ aus -H, -Halogen, -OH, -C₁₋₆-Alkyl, -O-C₁₋₆-Alkyl, -S-C₁₋₆-Alkyl, -(C=O) -C₁₋₆-Alkyl, -(C=O)-O-C₁-₆-Alkyl, -Het₂, -C₃₋₆-Cycloalkyl -(C=O)-Het₂,-(C=O) -NR₂₉R₃₀ und -SO₂-C₁₋₆-Alkyl ausgewählt ist, wobei -C₁₋₆-Alkyl jeweils gegebenenfalls unabhängig durch 1 bis 3 aus -Halogen, -OH, -O-C₁₋₆-Alkyl, -S-C₁₋₆-Alkyl, -NR₁₅R₁₆, -Het₂ und -Ar₃ ausgewählte Substituenten substituiert ist,
R₄ unabhängig aus -Halogen, -OH, -C₁₋₆-Alkyl, -O-C₁₋₆-Alkyl, -S-C₁₋₆-Alkyl, -NR₁₇R₁₈ und -Het₄ ausgewählt ist,
R₅ aus -H, -C₁₋₆-Alkyl, -C₃₋₆-Cycloalkyl ausgewählt ist, wobei C₁₋₆-Alkyl bzw. -C₃₋₆-Cycloalkyl jeweils gegebenenfalls unabhängig durch 1 bis 3 aus -Halogen, -OH, -O-C₁₋₆-Alkyl, -S-C₁₋₆-Alkyl, -Het₅, -CN und -NR₃₁R₃₂ ausgewählte Substituenten substituiert ist,
R₆ aus -H, -OH, -Halogen, -C₁₋₆-Alkyl, -O-C₁₋₆-Alkyl, -S-C₁₋₆-Alkyl, -NR₃₃R₃₄ und -Het₈ ausgewählt ist,
R₉, R₁₀, R₁₁, R₁₂, R₁₃, R₁₄, R₁₅, R₁₆, R₁₇, R₁₈, R₁₉, R₂₀,R₂₁, R₂₂, R₂₃, R₂₄, R₂₅, R₂₆, R₂₇, R₂₈, R₂₉, R₃₀, R₃₁,R₃₂, R₃₃, R₃₄, R₃₇ und R₃₈ jeweils unabhängig aus -H, =0, -C₁₋₆-Alkyl und -Het₁ ausgewählt sind, wobei-C₁₋₆-Alkyl jeweils gegebenenfalls unabhängig durch 1 bis 3 aus -Halogen, -OH, -O-C₁₋₆-Alkyl, -S-C₁₋₆-Alkyl, -NR₃₅R₃₆, -Het₇ und -Ar₄ ausgewählte Substituenten substituiert ist,
R₃₅ und R₃₆ jeweils unabhängig aus -H, =0 und -C₁₋₆-Alkyl ausgewählt sind, wobei -C₁₋₆-Alkyl jeweils gegebenenfalls unabhängig durch 1 bis 3 aus -Halogen, -OH, -O-C₁₋₆-Alkyl und -S-C₁₋₆-Alkyl ausgewählte Substituenten substituiert ist,
X₁ aus -C₁₋₆-Alkyl-, -O-C₁₋₆-Alkyl-, -S-C₁₋₆-Alkyl-, -(C=O)- -NR₃-(C=O)-, -C₁₋₆-Alkyl-NR₃-, -NR₃-, -(C=O)-, -NR₃-(C=O)-NR₃₇-, -NR₃-C₁₋₆-Alkyl-, -NR₃-SO₂-, -NR₃-(C=O)-C₁₋₆-Alkyl-, -(C=O)-NR₃-C₁₋₆-Alkyl-, -O-C₁₋₆-Alkyl-O-C₁₋₆-alkyl- und -C₁₋₆-Alkyl-NR₃-C₁₋₆-alkyl- ausgewählt ist, wobei -C₁₋₆-Alkyl jeweils gegebenenfalls unabhängig durch 1 bis 3 aus -Halogen, -OH, -C₁₋₆-Alkyl, -O-C₁₋₆-Alkyl, -S-C₁₋₆-Alkyl, -Phenyl und -NR₂₃R₂₄ ausgewählte Substituenten substituiert ist,
X₂ aus -C₁₋₆-Alkyl-, -O-C₁₋₆-Alkyl-, -S-C₁₋₆-Alkyl-, -(C=O)-, -NR₂-(C=O)-, -C₁₋₆-Alkyl-NR₂-, -NR₂-, -(C=O)-, -NR₂- (C=0) -NR₃₈-, -NR₂-C₁₋₆-Alkyl-, -NR₂-SO₂-, -NR₂-(C=O)-C₁₋₆-Alkyl-, -(C=O)-NR₂-C₁₋₆-Alkyl-, -O-C₁₋₆-Alkyl-O-C₁₋₆-alkyl- und -C₁₋₆-Alkyl-NR₂-C₁₋₆-alkyl- ausgewählt ist, wobei -C₁₋₆-Alkyl jeweils gegebenenfalls unabhängig durch 1 bis 3 aus -Halogen, -OH, -C₁₋₆-Alkyl, -O-C₁₋₆-Alkyl, -S-C₁₋₆-Alkyl, -Phenyl und -NR₂₅R₂₆ ausgewählte Substituenten substituiert ist,
Y aus einer direkten Bindung, -CHR₆-, -O-, -S-und -NR₅- ausgewählt ist,
Ar₂, Ar₃ und Ar₄ jeweils unabhängig voneinander für einen 5- oder 6-gliedrigen aromatischen Heterocyclus stehen, welcher gegebenenfalls 1 oder 2 aus 0, N und S ausgewählte Heteroatome umfasst, wobei Ar₂, Ar₃ und Ar₄ jeweils gegebenenfalls unabhängig durch 1 bis 3 aus -NR₁₉R₂₀, -C₁₋₆-Alkyl,-O-C₁₋₆-Alkyl und -S-C₁₋₆-Alkyl ausgewählte Substituenten substituiert sind,
Het₁, Het₂, Het₃, Het₄, Het₅, Het₆, Het₇ und Het₈ jeweils unabhängig voneinander für einen 5- oder 6-gliedrigen monocyclischen Heterocyclus mit 1 bis 3 aus 0, N und S ausgewählten Heteroatomen stehen, wobei Het₁, Het₂, Het₃, Het₄, Het₅, Het₆, Het₇ und Het₈ jeweils gegebenenfalls durch 1 bis 3 aus -C₁₋₆-Alkyl, -O-C₁₋₆-Alkyl, -S-C₁₋₆-Alkyl und -NR₂₁R₂₂ ausgewählte Substituenten substituiert sind, wobei -C₁₋₆-Alkyl jeweils gegebenenfalls unabhängig durch 1 bis 3 -Halogen substituiert ist,
Z₁, Z₂, Z₃, Z₄ und Z₅ jeweils unabhängig voneinander aus C und N ausgewählt sind,
m und n jeweils unabhängig voneinander für 0, 1, 2, 3 oder 4 stehen,
zur Verwendung bei der Prävention und/oder Behandlung einer mit LRRK2-Kinase assoziierten Krankheit.

3. Verbindung der Formel I oder ein Stereoisomer, ein Tautomer, eine racemische Mischung, ein Salz, ein Hydrat, eine N-Oxidform oder ein Solvat davon nach Anspruch 1, wobei
A₁ für N steht und A₂ für C steht,
A₁ und A₂ aus C und N ausgewählt sind, wobei, wenn A₁ für C steht, A₂ für N steht, und wobei, wenn A₂ für C steht, A₁ für N steht,
R₁ und R₇ jeweils unabhängig voneinander aus -H, -Halogen, -OH, -C₁₋₆-Alkyl, -O-C₁₋₆-Alkyl, -S-C₁₋₆-Alkyl, -NR₉R₁₀, -(C=O)-R₄, -SO₂-R₄,-CN, -NR₉-SO₂-R₄, -C₃₋₆-Cycloalkyl und -Het₆ ausgewählt sind, wobei -C₁₋₆-Alkyl jeweils gegebenenfalls unabhängig durch 1 bis 3 aus-Halogen, -OH, -NR₁₁R₁₂, -O-C₁₋₆-Alkyl und -S-C₁₋₆-Alkyl ausgewählte Substituenten substituiert ist,
R₂ aus -H, -Halogen, -OH, -C₁₋₆-Alkyl, -O-C₁₋₆-Alkyl, -S-C₁₋₆-Alkyl, -(C=O)-C₁₋₆-Alkyl, -(C=O)-O-C₁-₆-Alkyl, -(C=O)-NR₂₇R₂₈, -Het₃, -(C=O)-Het₃, -SO₂-C₁-₆-Alkyl und -C₃₋₆-Cycloalkyl ausgewählt ist, wobei-C₁₋₆-Alkyl jeweils gegebenenfalls unabhängig durch 1 bis 3 aus -Halogen, -OH, -O-C₁₋₆-Alkyl, -S-C₁₋₆-Alkyl, -Het₃, -Ar₂ und -NR₁₃R₁₄ ausgewählte Substituenten substituiert ist,
R₃ aus -H, -Halogen, -OH, -C₁₋₆-Alkyl, -O-C₁₋₆-Alkyl, -S-C₁₋₆-Alkyl, -(C=O)-C₁₋₆-Alkyl, -(C=O)-O-C₁₋₆-Alkyl, -Het₂, -C₃₋₆-Cycloalkyl -(C=O)-Het₂,-(C=O)-NR₂₉R₃₀ und -SO₂-C₁₋₆-Alkyl ausgewählt ist, wobei -C₁₋₆-Alkyl jeweils gegebenenfalls unabhängig durch 1 bis 3 aus -Halogen, -OH, -O-C₁₋₆-Alkyl, -S-C₁₋₆-Alkyl, -NR₁₅R₁₆, -Het₂ und -Ar₃ ausgewählte Substituenten substituiert ist,
R₄ unabhängig aus -Halogen, -OH, -C₁₋₆-Alkyl, -O-C₁₋₆-Alkyl, -S-C₁₋₆-Alkyl, -NR₁₇R₁₈ und -Het₄ ausgewählt ist,
R₅ aus -H, -C₁₋₆-Alkyl, -C₃₋₆-Cycloalkyl ausgewählt ist, wobei C₁₋₆-Alkyl bzw. -C₃₋₆-Cycloalkyl jeweils gegebenenfalls unabhängig durch 1 bis 3 aus -Halogen, -OH, -O-C₁₋₆-Alkyl, -S-C₁₋₆-Alkyl, -Het₅, -CN und -NR₃₁R₃₂ ausgewählte Substituenten substituiert ist,
R₆ aus -H, -OH, -Halogen, -C₁₋₆-Alkyl, -O-C₁₋₆-Alkyl, -S-C₁₋₆-Alkyl, -NR₃₃R₃₄ und -Het₈ ausgewählt ist,
R₉, R₁₀, R₁₁, R₁₂, R₁₃, R₁₄, R₁₅, R₁₆, R₁₇, R₁₈, R₁₉, R₂₀,R₂₁, R₂₂, R₂₃, R₂₄, R₂₅, R₂₆, R₂₇, R₂₈, R₂₉, R₃₀, R₃₁,R₃₂, R₃₃, R₃₄, R₃₇ und R₃₈ jeweils unabhängig aus -H, =0, -C₁₋₆-Alkyl und -Het₁ ausgewählt sind, wobei-C₁₋₆-Alkyl jeweils gegebenenfalls unabhängig durch 1 bis 3 aus -Halogen, -OH, -O-C₁₋₆-Alkyl, -S-C₁₋₆-Alkyl, -NR₃₅R₃₆, -Het₇ und -Ar₄ ausgewählte Substituenten substituiert ist,
R₃₅ und R₃₆ jeweils unabhängig aus -H, =O und -C₁₋₆-Alkyl ausgewählt sind, wobei -C₁₋₆-Alkyl jeweils gegebenenfalls unabhängig durch 1 bis 3 aus -Halogen, -OH, -O-C₁₋₆-Alkyl und -S-C₁₋₆-Alkyl ausgewählte Substituenten substituiert ist,
X₁ aus -C₁₋₆-Alkyl-, -O-C₁₋₆-Alkyl-, -S-C₁₋₆-Alkyl-, -(C=O)- -NR₃-(C=O)-, -C₁₋₆-Alkyl-NR₃-, -NR₃-, -(C=O)-, -NR₃- (C=0) -NR₃₇-, -NR₃-C₁₋₆-Alkyl-, -NR₃-SO₂-, -NR₃- (C=O) -C₁₋₆-Alkyl-, -(C=O)-NR₃-C₁₋₆-Alkyl-, -O-C₁₋₆-Alkyl-O-C₁₋₆-alkyl- und -C₁₋₆-Alkyl-NR₃-C₁₋₆-alkyl- ausgewählt ist, wobei -C₁₋₆-Alkyl jeweils gegebenenfalls unabhängig durch 1 bis 3 aus -Halogen, -OH, -C₁₋₆-Alkyl, -O-C₁₋₆-Alkyl, -S-C₁₋₆-Alkyl, -Phenyl und -NR₂₃R₂₄ ausgewählte Substituenten substituiert ist,
X₂ aus -C₁₋₆-Alkyl-, -O-C₁₋₆-Alkyl-, -S-C₁₋₆-Alkyl-, -(C=O)-, -NR₂-(C=O)-, -C₁₋₆-Alkyl-NR₂-, -NR₂-, -(C=O)-, -NR₂- (C=0) -NR₃₈-, -NR₂-C₁₋₆-Alkyl-, -NR₂-SO₂-, -NR₂-(C=O)-C₁₋₆-Alkyl-, -(C=O)-NR₂-C₁₋₆-Alkyl-, -O-C₁₋₆-Alkyl-O-C₁₋₆-alkyl- und -C₁₋₆-Alkyl-NR₂-C₁₋₆-alkyl- ausgewählt ist, wobei -C₁₋₆-Alkyl jeweils gegebenenfalls unabhängig durch 1 bis 3 aus -Halogen, -OH, -C₁₋₆-Alkyl, -O-C₁₋₆-Alkyl, -S-C₁₋₆-Alkyl, -Phenyl und -NR₂₅R₂₆ ausgewählte Substituenten substituiert ist,
Y aus einer direkten Bindung, -CHR₆-, -O-, -S-und -NR₅- ausgewählt ist,
Ar₂, Ar₃ und Ar₄ jeweils unabhängig voneinander für einen 5- oder 6-gliedrigen aromatischen Heterocyclus stehen, welcher gegebenenfalls 1 oder 2 aus 0, N und S ausgewählte Heteroatome umfasst, wobei Ar₂, Ar₃ und Ar₄ jeweils gegebenenfalls unabhängig durch 1 bis 3 aus -NR₁₉R₂₀, -C₁₋₆-Alkyl,-O-C₁₋₆-Alkyl und -S-C₁₋₆-Alkyl ausgewählte Substituenten substituiert sind,
Het₁, Het₂, Het₃, Het₄, Het₅, Het₆, Het₇ und Het₈ jeweils unabhängig voneinander für einen 5- oder 6-gliedrigen monocyclischen Heterocyclus mit 1 bis 3 aus 0, N und S ausgewählten Heteroatomen stehen, wobei Het₁, Het₂, Het₃, Het₄, Het₅, Het₆, Het₇ und Het₈ jeweils gegebenenfalls durch 1 bis 3 aus -C₁₋₆-Alkyl, -O-C₁₋₆-Alkyl, -S-C₁₋₆-Alkyl und -NR₂₁R₂₂ ausgewählte Substituenten substituiert sind, wobei -C₁₋₆-Alkyl jeweils gegebenenfalls unabhängig durch 1 bis 3 -Halogen substituiert ist,
Z₁, Z₂, Z₃, Z₄ und Z₅ jeweils unabhängig voneinander aus C und N ausgewählt sind,
m und n jeweils unabhängig voneinander für 0, 1, 2, 3 oder 4 stehen,
zur Verwendung bei der Prävention und/oder Behandlung einer mit LRRK2-Kinase assoziierten Krankheit.

4. Verbindung der Formel I oder ein Stereoisomer, ein Tautomer, eine racemische Mischung, ein Salz, ein Hydrat, eine N-Oxidform oder ein Solvat davon nach Anspruch 1, wobei
A₁ und A₂ aus C und N ausgewählt sind, wobei, wenn A₁ für C steht, A₂ für N steht, und wobei, wenn A₂ für C steht, A₁ für N steht,
R₁ und R₇ jeweils unabhängig voneinander aus -H, -Halogen und -C₁₋₆-Alkyl ausgewählt sind,
R₂ aus -H und -C₁₋₆-Alkyl ausgewählt ist,
R₃ für -H steht,
R₅ aus -H und -C₁₋₆-Alkyl ausgewählt ist, wobei -C₁₋₆-Alkyl gegebenenfalls durch -CN substituiert sein kann,
X₁ aus -O-C₁₋₆-Alkyl- und -C₁₋₆-Alkyl-NR₃- ausgewählt ist, wobei -C₁₋₆-Alkyl jeweils gegebenenfalls unabhängig durch 1 bis 3 aus -C₁₋₆-Alkyl ausgewählte Substituenten substituiert ist,
X₂ aus -O-C₁₋₆-Alkyl- und -C₁₋₆-Alkyl-NR₂ ausgewählt ist,
Y aus -0- und -NR₅- ausgewählt ist,
Z₁, Z₂, Z₃, Z₄ und Z₅ jeweils unabhängig voneinander aus C und N ausgewählt sind,
m und n jeweils unabhängig voneinander für 0, 1, 2, 3 oder 4 stehen,
zur Verwendung bei der Prävention und/oder Behandlung einer mit LRRK2-Kinase assoziierten Krankheit.

5. Verbindung der Formel I oder ein Stereoisomer, ein Tautomer, eine racemische Mischung, ein Salz, ein Hydrat, eine N-Oxidform oder ein Solvat davon nach Anspruch 1, wobei
A₁ für N steht und A₂ für C steht,
R₁ und R₇ jeweils unabhängig voneinander aus -H, -Halogen und -C₁₋₆-Alkyl ausgewählt sind,
R₂ aus -H und -C₁₋₆-Alkyl ausgewählt ist,
R₃ für -H steht,
R₅ aus -H und -C₁₋₆-Alkyl ausgewählt ist, wobei -C₁₋₆-Alkyl gegebenenfalls durch -CN substituiert sein kann,
X₁ aus -O-C₁₋₆-Alkyl- und -C₁₋₆-Alkyl-NR₃- ausgewählt ist, wobei -C₁₋₆-Alkyl jeweils gegebenenfalls unabhängig durch 1 bis 3 aus -C₁₋₆-Alkyl ausgewählte Substituenten substituiert ist,
X₂ aus -O-C₁₋₆-Alkyl- und -C₁₋₆-Alkyl-NR₂ ausgewählt ist,
Y aus -0- und -NR₅- ausgewählt ist,
Z₁, Z₂, Z₃, Z₄ und Z₅ jeweils unabhängig voneinander aus C und N ausgewählt sind,
m und n jeweils unabhängig voneinander für 0, 1, 2, 3 oder 4 stehen,
zur Verwendung bei der Prävention und/oder Behandlung einer mit LRRK2-Kinase assoziierten Krankheit.

6. Verbindung der Formel I oder ein Stereoisomer, ein Tautomer, eine racemische Mischung, ein Salz, ein Hydrat, eine N-Oxidform oder ein Solvat davon nach Anspruch 1, wobei
A₁ für C steht und A₂ für N steht,
R₁ und R₇ jeweils unabhängig voneinander aus -H, -Halogen und -C₁₋₆-Alkyl ausgewählt sind,
R₂ aus -H und -C₁₋₆-Alkyl ausgewählt ist,
R₃ für -H steht,
R₅ aus -H und -C₁₋₆-Alkyl ausgewählt ist, wobei -C₁₋₆-Alkyl gegebenenfalls durch -CN substituiert sein kann,
X₁ aus -O-C₁₋₆-Alkyl- und -C₁₋₆-Alkyl-NR₃- ausgewählt ist, wobei -C₁₋₆-Alkyl jeweils gegebenenfalls unabhängig durch 1 bis 3 aus -C₁₋₆-Alkyl ausgewählte Substituenten substituiert ist,
X₂ aus -O-C₁₋₆-Alkyl- und -C₁₋₆-Alkyl-NR₂ ausgewählt ist,
Y aus -0- und -NR₅- ausgewählt ist,
Z₁, Z₂, Z₃, Z₄ und Z₅ jeweils unabhängig voneinander aus C und N ausgewählt sind,
m und n jeweils unabhängig voneinander für 0, 1, 2, 3 oder 4 stehen,
zur Verwendung bei der Prävention und/oder Behandlung einer mit LRRK2-Kinase assoziierten Krankheit.

7. Verbindung, definiert wie in einem der Ansprüche 1 bis 6, zur Verwendung bei der Diagnose, Prävention und/oder Behandlung einer mit LRRK2-Kinase assoziierten Krankheit, wobei die Verbindung ausgewählt ist aus der Liste umfassend:

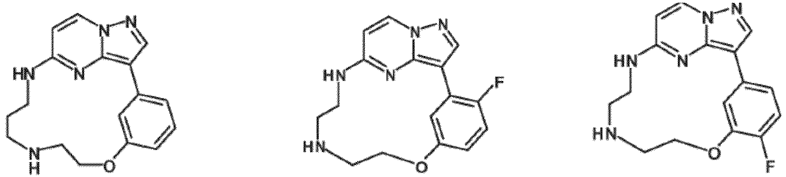

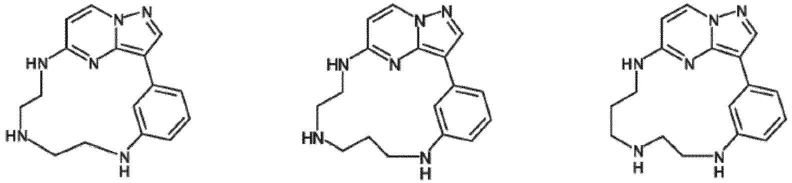

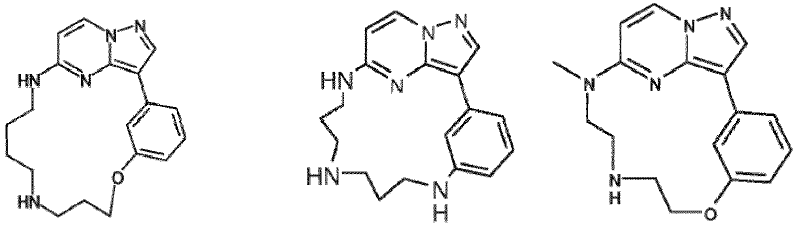

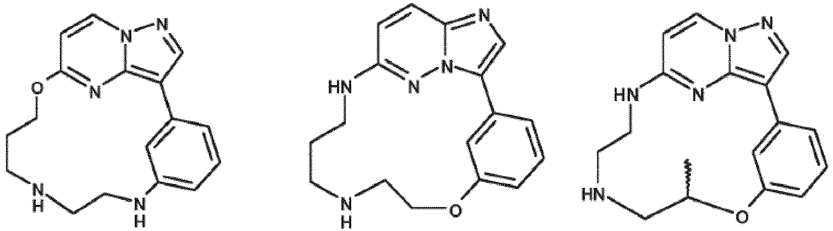

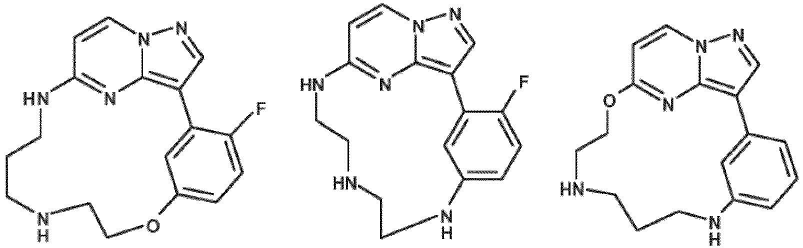

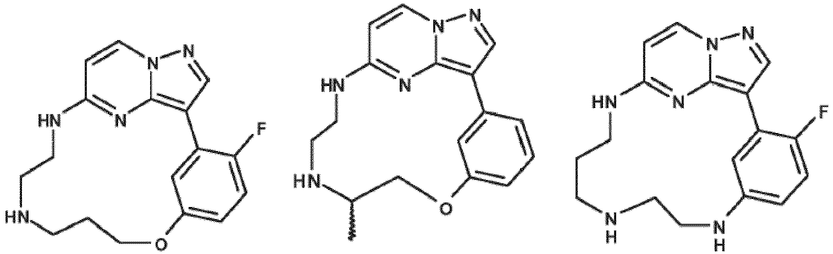

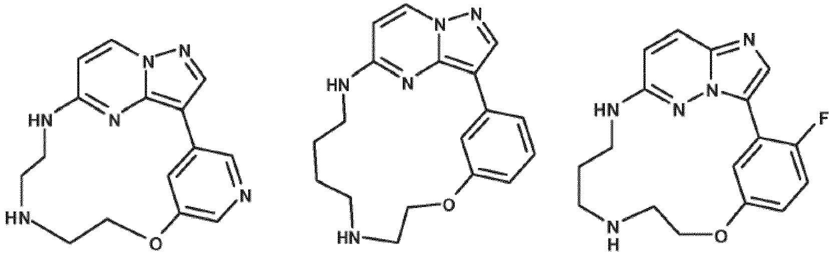

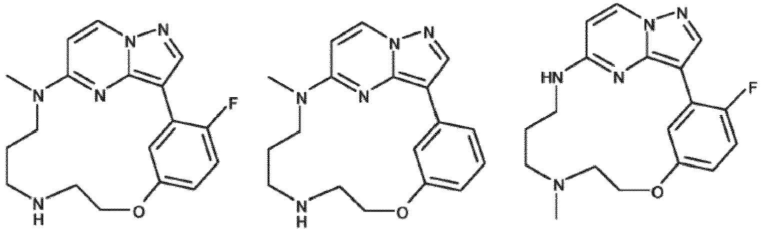

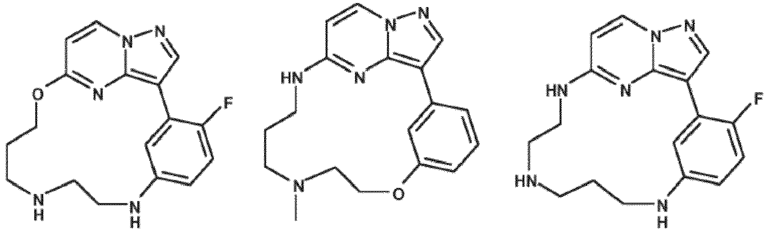

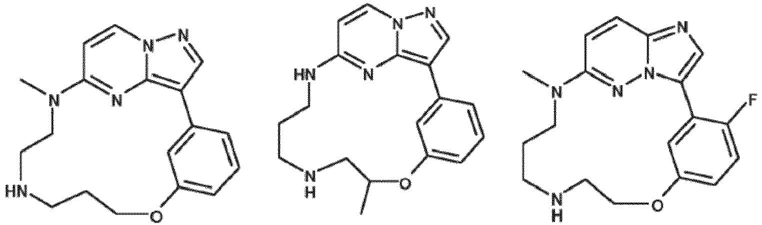

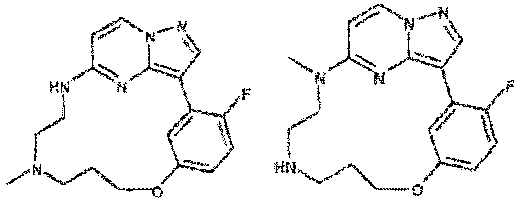

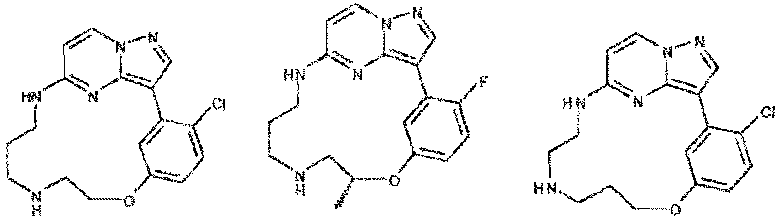

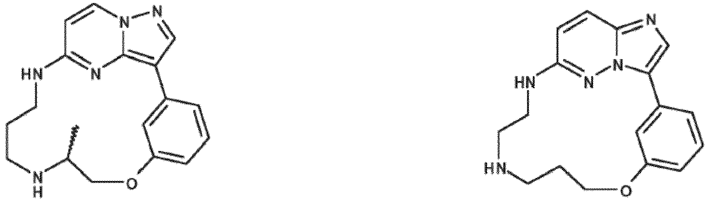

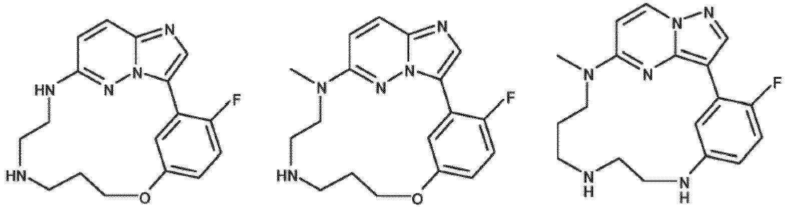

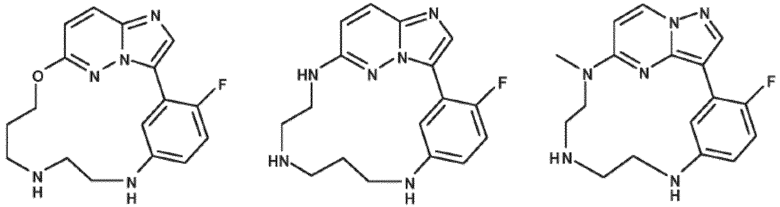

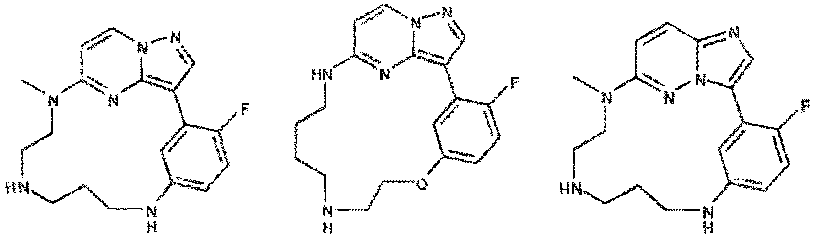

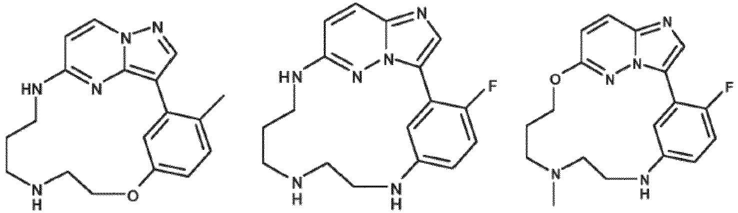

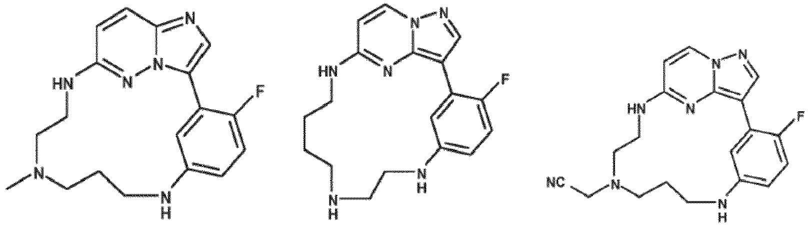

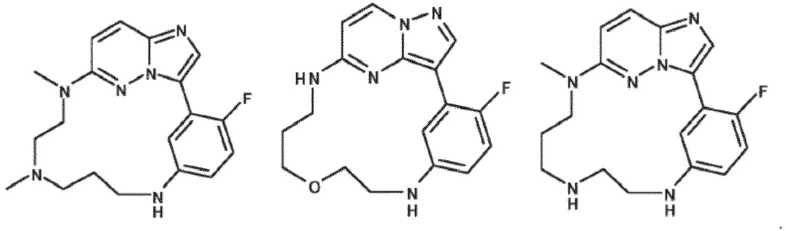

8. Verbindung, definiert wie in einem der Ansprüche 1 bis 7, zur Verwendung bei der Diagnose, Prävention und/oder Behandlung einer mit LRRK2-Kinase assoziierten Krankheit, wobei die Pyrazolopyrimidin- oder die Imidazopyridazingruppierung in Position Z₄ oder Z₅ gemäß der Nummerierung nach Formel I an die Aryl- bzw. Heteroarylgruppierung gebunden ist.

9. Verbindung, definiert wie in einem der Ansprüche 1 bis 7, zur Verwendung bei der Diagnose, Prävention und/oder Behandlung einer mit LRRK2-Kinase assoziierten Krankheit, wobei R₁ in Position Z₁, Z₂ oder Z₃ gemäß der Nummerierung nach Formel I an die Aryl- bzw. Heteroarylgruppierung gebunden ist.

10. Verbindung, definiert wie in einem der Ansprüche 1 bis 7, zur Verwendung bei der Diagnose, Prävention und/oder Behandlung einer mit LRRK2-Kinase assoziierten Krankheit, wobei es sich bei der mit LRRK2-Kinase assoziierten Krankheit um eine neurologische Erkrankung, insbesondere ausgewählt aus der Parkinson-Krankheit und Alzheimer-Krankheit umfassenden Liste, handelt.

11. Verbindung nach Anspruch 1 der Formel (IIc), oder ein Stereoisomer, ein Tautomer, eine racemische Mischung, ein Salz, ein Hydrat, eine N-Oxidform oder ein Solvat davon, 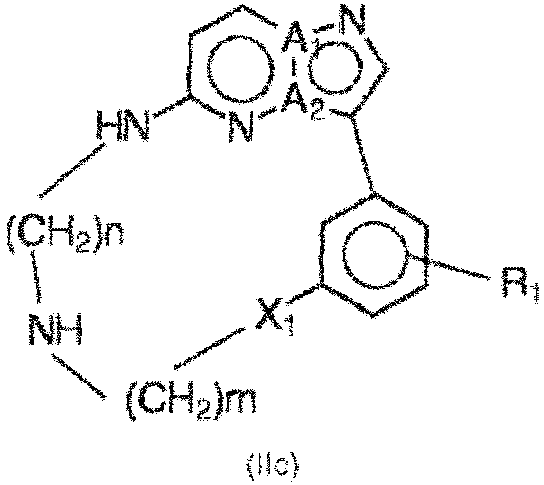 wobei
A₁ und A₂ aus C und N ausgewählt sind, wobei, wenn A₁ für C steht, A₂ für N steht, und wobei, wenn A₂ für C steht, A₁ für N steht,
R₁ aus der -H, -F, -CH₃ und -CN umfassenden Liste ausgewählt ist,
X₁ aus der -NH- und -0- umfassenden Liste ausgewählt ist,
m und n jeweils unabhängig voneinander für 1, 2, 3 oder 4 stehen,
zur Verwendung bei der Prävention und/oder Behandlung einer mit LRRK2-Kinase assoziierten Krankheit.

12. Verbindung, definiert wie in einem der Ansprüche 1 bis 11, zur Verwendung bei der Diagnose, Prävention und/oder Behandlung einer mit LRRK2-Kinase assoziierten Krankheit, wobei die Verbindung ausgewählt ist aus der Liste umfassend 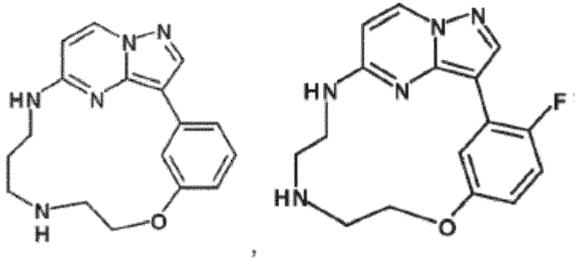 und

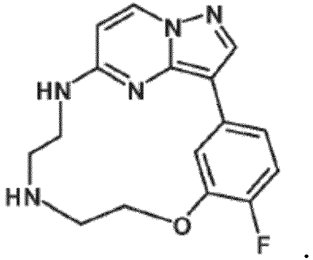

13. Pharmazeutische Zusammensetzung zur Verwendung bei der Diagnose, Prävention und/oder Behandlung einer mit LRRK2-Kinase assoziierten Krankheit, umfassend eine wie in einem der Ansprüche 1 bis 12 definierte Verbindung.

14. Verbindung oder ein Stereoisomer, ein Tautomer, eine racemische Mischung, ein Salz, ein Hydrat, eine N-Oxidform oder ein Solvat davon gemäß der allgemeinen Formel (IIIc) 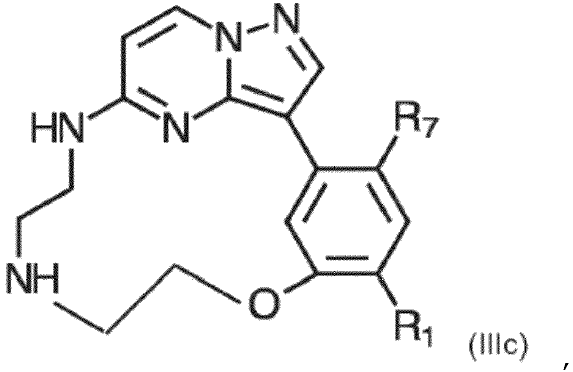 wobei
R₁ für -H steht und R₇ für -F steht oder
R₇ für -H steht und R₁ für -F steht.

15. Verbindung nach Anspruch 14, wobei es sich bei der Verbindung um: 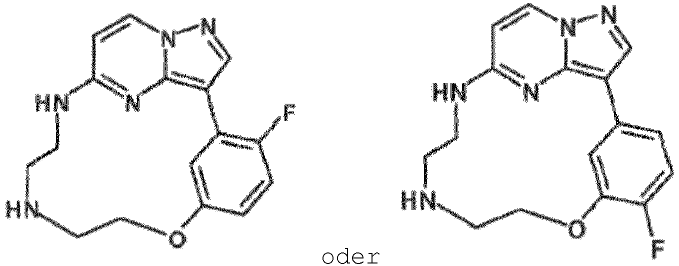 handelt.

16. Verbindung, ausgewählt aus der Liste umfassend:

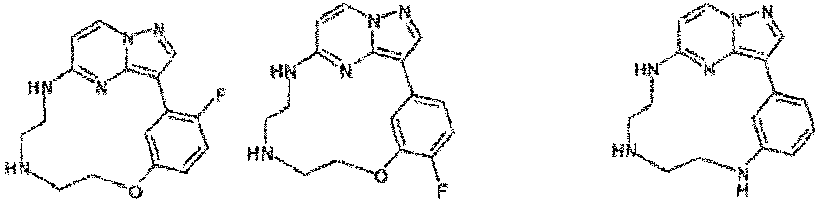

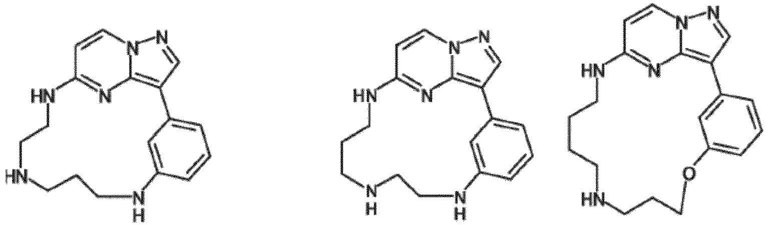

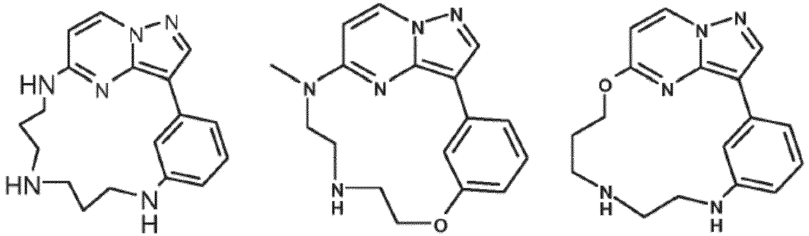

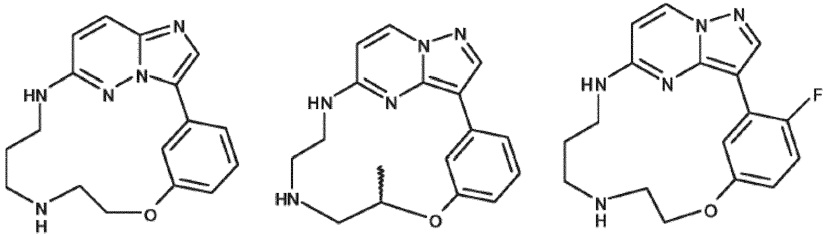

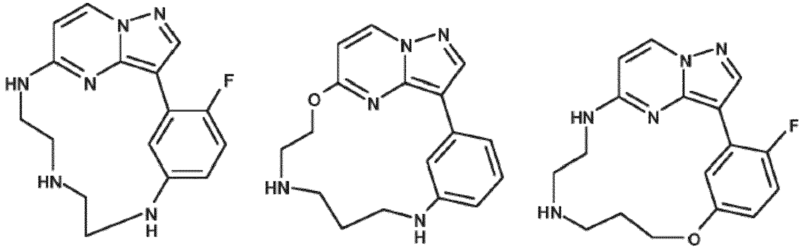

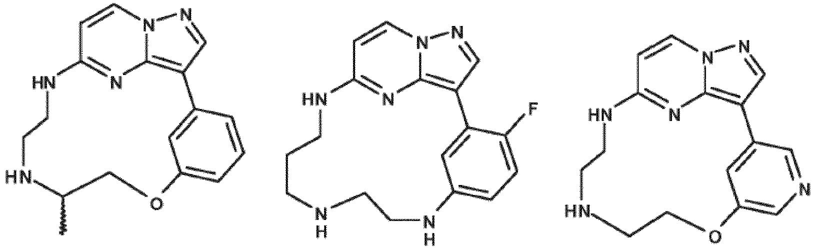

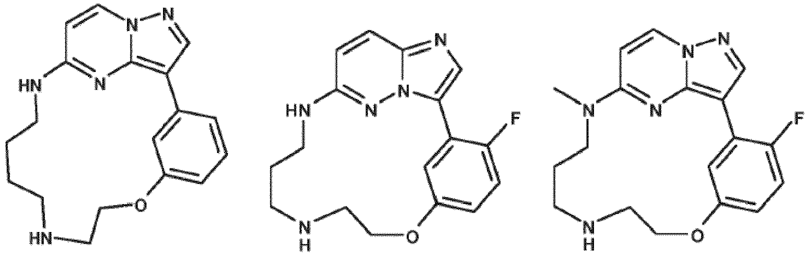

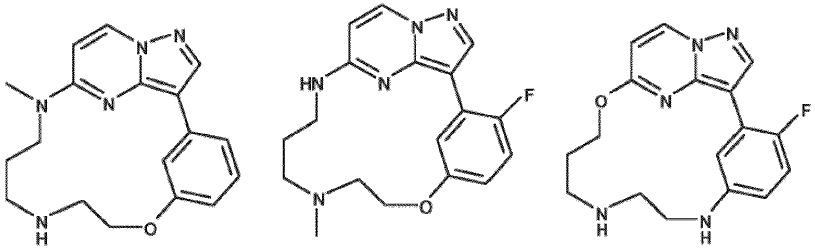

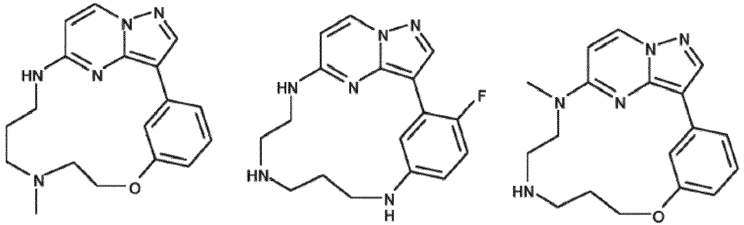

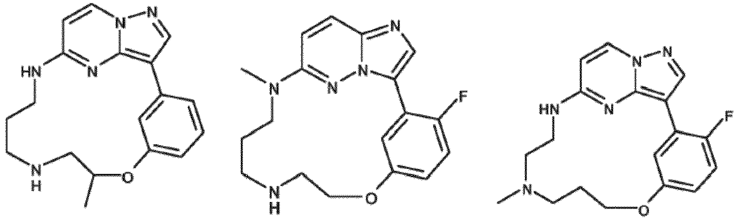

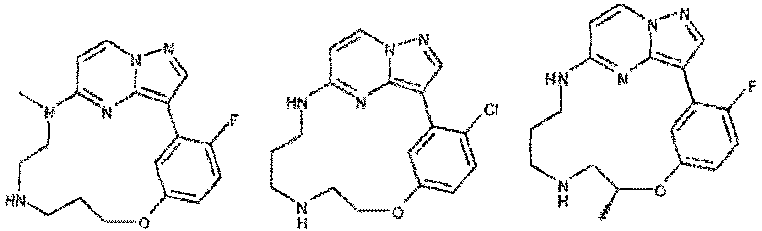

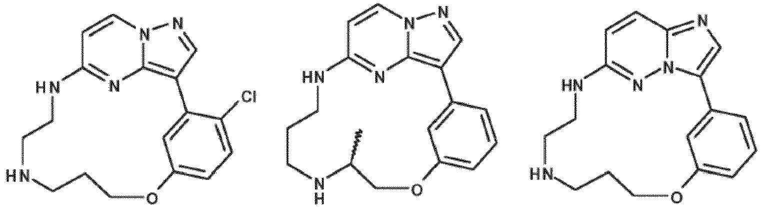

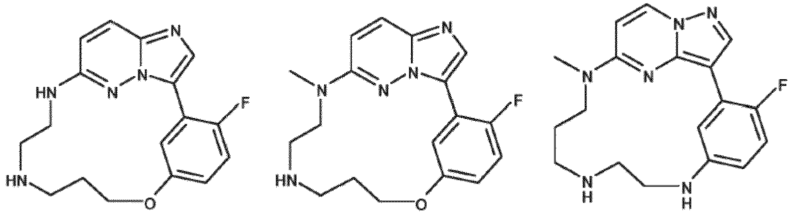

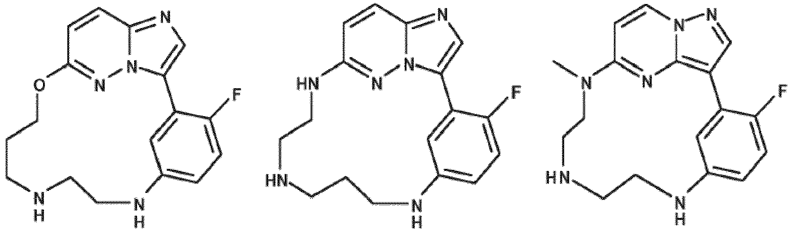

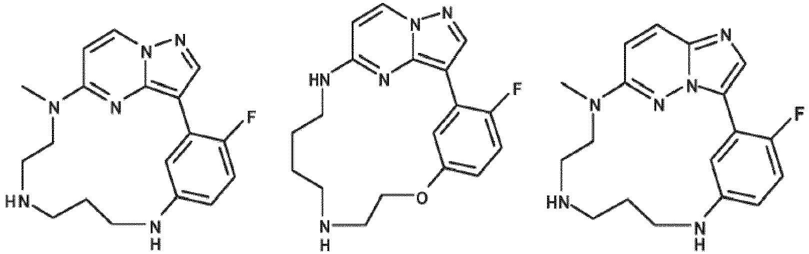

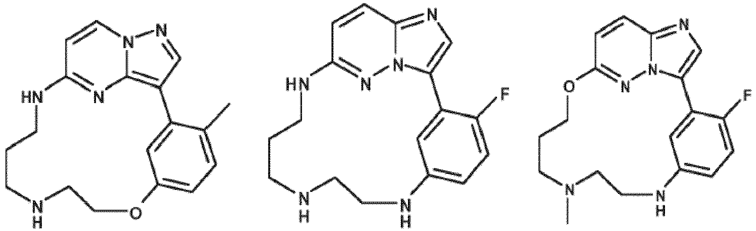

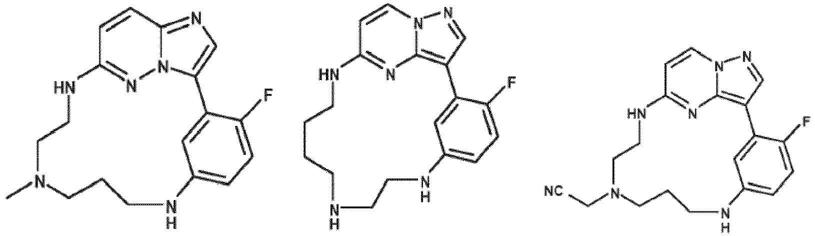

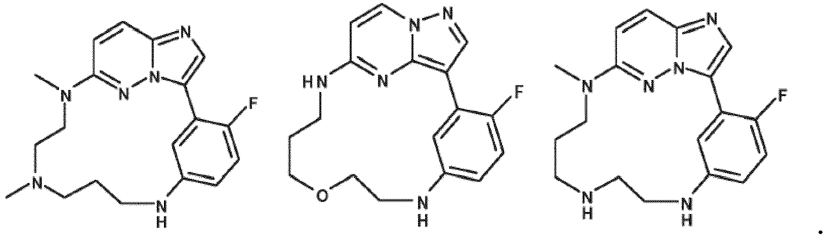

## Revendications

1. Composé de formule I ou stéréoisomère, tautomère, racémique, sel, hydrate, forme de N-oxyde, ou solvate de celui-ci, 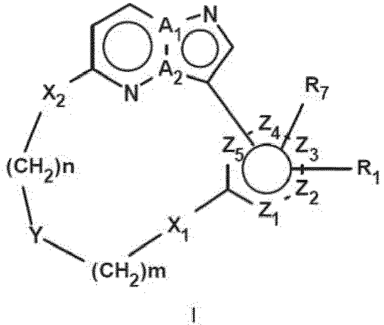 dans lequel
A₁ et A₂ sont choisis parmi C et N ; où, lorsque A₁ est C, alors A₂ est N ; et où, lorsque A₂ est C, alors A₁ est N ;
R₁ et R₇ sont chacun indépendamment choisis parmi -H, -halogéno, -OH, -(alkyle en C₁₋₆), -O-(alkyle en C₁₋₆), -S- (alkyle en C₁₋₆), -NR₉R₁₀, -(C=O)-R₄, -SO₂-R₄, -CN, -NR₉-SO₂-R₄, - (cycloalkyle en C₃₋₆), et -Het₆ ; où chacun desdits - (alkyle en C₁₋₆) est facultativement et indépendamment substitué par 1 à 3 substituants choisis parmi -halogéno, -OH, -NR₁₁R₁₂, -O-(alkyle en C₁₋₆), et -S- (alkyle en C₁₋₆) ;
R₂ est choisi parmi -H, -halogéno, -OH, -(alkyle en C₁₋₆), -O-(alkyle en C₁₋₆), -S- (alkyle en C₁₋₆), -(C=O)-(alkyle en C₁₋₆), -(C=O)-O-(alkyle en C₁₋₆), -(C=O)-NR₂₇R₂₈, -Het₃, - (C=O) -Het₃, -SO₂-(alkyle en C₁₋₆), et -(cycloalkyle en C₃₋₆) ; où chacun desdits - (alkyle en C₁₋₆) est facultativement et indépendamment substitué par 1 à 3 substituants choisis parmi -halogéno, -OH, -O-(alkyle en C₁₋₆), -S-(alkyle en C₁₋₆), -Het₃, -Ar₂, et -NR₁₃R₁₄ ;
R₃ est choisi parmi -H, -halogéno, -OH, -(alkyle en C₁₋₆), -O-(alkyle en C₁₋₆), -S- (alkyle en C₁₋₆), -(C=O)-(alkyle en C₁₋₆), -(C=O)-O-(alkyle en C₁₋₆), -Het₂, - (cycloalkyle en C₃₋₆) -(C=O)-Het₂, -(C=O)-NR₂₉R₃₀, et -SO₂-(alkyle en C₁₋₆) ; où chacun desdits - (alkyle en C₁₋₆) est facultativement et indépendamment substitué par 1 à 3 substituants choisis parmi -halogéno, -OH, -O-(alkyle en C₁₋₆), -S-(alkyle en C₁₋₆), -NR₁₅R₁₆, -Het₂, et -Ar₃ ;
R₄ est indépendamment choisi parmi -halogéno, -OH, -(alkyle en C₁₋₆), -O-(alkyle en C₁₋₆), -S- (alkyle en C₁₋₆), -NR₁₇R₁₈, et -Het₄ ;
R₅ est choisi parmi -H, - (alkyle en C₁₋₆), -(cycloalkyle en C₃₋₆) ; où chacun desdits alkyle en C₁₋₆ ou -(cycloalkyle en C₃₋₆) est facultativement et indépendamment substitué par 1 à 3 substituants choisis parmi -halogéno, -OH, -O(alkyle en C₁₋₆), -S (alkyle en C₁₋₆), -Het₅, -CN et -NR₃₁R₃₂ ;
R₆ est choisi parmi -H, -OH, -halogéno, -(alkyle en C₁₋₆), -O-(alkyle en C₁₋₆), -S-(alkyle en C₁₋₆), -NR₃₃R₃₄, et -Het₈ ;
R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₇ et R₃₈ sont chacun indépendamment choisis parmi -H, =0, - (alkyle en C₁₋₆), et -Het₁ ; où chacun desdits - (alkyle en C₁₋₆) est facultativement et indépendamment substitué par 1 à 3 substituants choisis parmi -halogéno, -OH, -O-(alkyle en C₁₋₆), -S-(alkyle en C₁₋₆), -NR₃₅R₃₆, -Het₇, et -Ar₄ ;
R₃₅ et R₃₆ sont chacun indépendamment choisis parmi -H, =0, et - (alkyle en C₁₋₆) ; où chacun desdits - (alkyle en C₁₋₆) est facultativement et indépendamment substitué par 1 à 3 substituants choisis parmi -halogéno, -OH, -O-(alkyle en C₁₋₆), et -S-(alkyle en C₁₋₆) ;
X₁ est choisi parmi - (alkyle en C₁₋₆)-, -O-(alkyle en C₁₋₆)-, -S-(alkyle en C₁₋₆)-, -(C=O)-, -NR₃-(C=O)-, - (alkyle en C₁₋₆)-NR₃-, -NR₃-, -(C=O)-, -NR₃-(C=O)-NR₃₇-, -NR₃-(alkyle en C₁₋₆)-, -NR₃-SO₂-, -NR₃-(C=O)-(alkyle en C₁₋₆)-, -(C=O) -NR₃-(alkyle en C₁-₆)-, -O-(alkyle en C₁₋₆)-O-(alkyle en C₁₋₆)- et -(alkyle en C₁₋₆)-NR₃- (alkyle en C₁₋₆)- ; où chacun desdits - (alkyle en C₁₋₆) est facultativement et indépendamment substitué par 1 à 3 substituants choisis parmi -halogéno, -OH, -(alkyle en C₁₋₆), -O-(alkyle en C₁₋₆), -S-(alkyle en C₁₋₆), -phényle, et -NR₂₃R₂₄
X₂ est choisi parmi -(alkyle en C₁₋₆)-, -O-(alkyle en C₁₋₆)-, -S-(alkyle en C₁₋₆)-, -(C=O)-, -NR₂-(C=O)-, -(alkyle en C₁₋₆)-NR₂-, -NR₂-, -(C=O)-, -NR₂-(C=O)-NR₃₈-, -NR₂-(alkyle en C₁₋₆)-, -NR₂-SO₂-, -NR₂-(C=O)-(alkyle en C₁₋₆)-, -(C=O)-NR₂-(alkyle en C₁₋₆)-, -O-(alkyle en C₁₋₆)-0- (alkyle en C₁₋₆)- et - (alkyle en C₃₋₆)-NR₂- (alkyle en C₁₋₆)- ; où chacun desdits - (alkyle en C₁₋₆) est facultativement et indépendamment substitué par 1 à 3 substituants choisis parmi -halogéno, -OH, -(alkyle en C₁₋₆), -O-(alkyle en C₁₋₆), -S- (alkyle en C₁₋₆), -phényle et -NR₂₅R₂₆ ;
Y est choisi parmi une liaison directe, -CHR₆-, -0-, -S-, et -NR₅- ;
Ar₂, Ar₃ et Ar₄ sont chacun indépendamment un hétérocycle aromatique de 5 ou 6 chaînons comprenant facultativement 1 ou 2 hétéroatomes choisis parmi 0, N et S ; où chacun desdits Ar₂, Ar₃, et Ar₄ est facultativement et indépendamment substitué par 1 à 3 substituants choisis parmi -NR₁₉R₂₀, -(alkyle en C₁₋₆), -O-(alkyle en C₁₋₆), et -S- (alkyle en C₁₋₆) ;
Het₁, Het₂, Het₃, Het₄, Het₅, Het₆, Het₇ et Het₈ sont chacun indépendamment un hétérocycle monocyclique de 5 ou 6 chaînons ayant de 1 à 3 hétéroatomes choisis parmi 0, N et S, où chacun desdits Het₁, Het₂, Het₃, Het₄, Het₅, Het₆, Het₇ et Het₈ est facultativement substitué par 1 à 3 substituants choisis parmi -(alkyle en C₁₋₆), -O-(alkyle en C₁₋₆), -S- (alkyle en C₁₋₆), et -NR₂₁R₂₂ ; où chacun desdits - (alkyle en C₁₋₆) est facultativement et indépendamment substitué par 1 à 3 halogéno ;
Z₁, Z₂, Z₃, Z₄ et Z₅ sont chacun indépendamment choisis parmi C et N ;
m et n sont chacun indépendamment 0, 1, 2, 3 ou 4,
pour utilisation dans la prévention et/ou le traitement d'une maladie liée à la kinase LRRK2.

2. Composé de formule I ou stéréoisomère, tautomère, racémique, sel, hydrate, forme de N-oxyde, ou solvate de celui-ci, selon la revendication 1, dans lequel
A₁ est C et A₂ est N
R₁ et R₇ sont chacun indépendamment choisis parmi -H, -halogéno, -OH, -(alkyle en C₁₋₆), -O-(alkyle en C₁₋₆), -S- (alkyle en C₁₋₆), -NR₉R₁₀, -(C=O)-R₄, -SO₂-R₄, -CN, -NR₉-SO₂-R₄, -(cycloalkyle en C₃₋₆), et -Het₆ ; où chacun desdits - (alkyle en C₁₋₆) est facultativement et indépendamment substitué par 1 à 3 substituants choisis parmi -halogéno, -OH, -NR₁₁R₁₂, -O-(alkyle en C₁₋₆), et -S- (alkyle en C₁₋₆) ;
R₂ est choisi parmi -H, -halogéno, -OH, -(alkyle en C₁₋₆), -O-(alkyle en C₁₋₆), -S- (alkyle en C₁-₆), -(C=O)-(alkyle en C₁₋₆), -(C=O)-O-(alkyle en C₁₋₆), -(C=O)-NR₂₇R₂₈, -Het₃, -(C=O)-Het₃, -SO₂-(alkyle en C₁₋₆), et -(cycloalkyle en C₃₋₆) ; où chacun desdits - (alkyle en C₁₋₆) est facultativement et indépendamment substitué par 1 à 3 substituants choisis parmi -halogéno, -OH, -O-(alkyle en C₁₋₆), -S-(alkyle en C₁₋₆), -Het₃, -Ar₂, et -NR₁₃R₁₄ ;
R₃ est choisi parmi -H, -halogéno, -OH, -(alkyle en C₁₋₆), -O-(alkyle en C₁₋₆), -S- (alkyle en C₁₋₆), -(C=O)-(alkyle en C₁₋₆), -(C=O)-O-(alkyle en C₁₋₆), -Het₂, -(cycloalkyle en C₃₋₆) -(C=O)-Het₂, -(C=O)-NR₂₉R₃₀, et -SO₂- (alkyle en C₁₋₆) ; où chacun desdits - (alkyle en C₁₋₆) est facultativement et indépendamment substitué par 1 à 3 substituants choisis parmi -halogéno, -OH, -O-(alkyle en C₁₋₆), -S-(alkyle en C₁₋₆), -NR₁₅R₁₆, -Het₂, et -Ar₃ ;
R₄ est indépendamment choisi parmi -halogéno, -OH, -(alkyle en C₁₋₆), -O-(alkyle en C₁₋₆), -S- (alkyle en C₁₋₆), -NR₁₇R₁₈, et -Het₄ ;
R₅ est choisi parmi -H, - (alkyle en C₁₋₆), -(cycloalkyle en C₃₋₆); où chacun desdits - (alkyle en C₁₋₆) ou -(cycloalkyle en C₃₋₆) est facultativement et indépendamment substitué par 1 à 3 substituants choisis parmi -halogéno, -OH, -O-(alkyle en C₁₋₆), -S-(alkyle en C₁₋₆), -Het₅, -CN et -NR₃₁R₃₂ ;
R₆ est choisi parmi -H, -OH, -halogéno, -(alkyle en C₁₋₆), -O-(alkyle en C₁₋₆), -S-(alkyle en C₁₋₆), -NR₃₃R₃₄, et -Het₈ ;
R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₇ et R₃₈ sont chacun indépendamment choisis parmi -H, =0, - (alkyle en C₁₋₆), et -Het₁ ; où chacun desdits - (alkyle en C₁₋₆) est facultativement et indépendamment substitué par 1 à 3 substituants choisis parmi -halogéno, -OH, -O-(alkyle en C₁₋₆), -S-(alkyle en C₁₋₆), -NR₃₅R₃₆, -Het₇, et -Ar₄ ;
R₃₅ et R₃₆ sont chacun indépendamment choisis parmi -H, =0, et - (alkyle en C₁₋₆) ; où chacun desdits - (alkyle en C₁₋₆) est facultativement et indépendamment substitué par 1 à 3 substituants choisis parmi -halogéno, -OH, -O-(alkyle en C₁₋₆), et -S-(alkyle en C₁₋₆) ;
X₁ est choisi parmi - (alkyle en C₁₋₆)-, -O-(alkyle en C₁₋₆)-, -S-(alkyle en C₁₋₆)-, -(C=O)-, -NR₃-(C=O)-, - (alkyle en C₁₋₆)-NR₃-, -NR₃-, -(C=O)-, -NR₃- (C=O) -NR₃₇-, -NR₃- (alkyle en C₁₋₆)-, -NR₃-SO₂-, -NR₃-(C=O)- (alkyle en C₁₋₆)-, -(C=O) -NR₃- (alkyle en C₁₋₆)-, -O-(alkyle en C₁₋₆)-0- (alkyle en C₁₋₆)- et - (alkyle en C₁₋₆) -NR₃- (alkyle en C₁₋₆)- ; où chacun desdits - (alkyle en C₁₋₆) est facultativement et indépendamment substitué par 1 à 3 substituants choisis parmi -halogéno, -OH, -(alkyle en C₁₋₆), -O-(alkyle en C₁₋₆), -S- (alkyle en C₁₋₆), -phényle, et -NR₂₃R₂₄
X₂ est choisi parmi - (alkyle en C₁₋₆)-, -O-(alkyle en C₁₋₆)-, -S-(alkyle en C₁₋₆)-, -(C=O)-, -NR₂-(C=O)-, - (alkyle en C₁₋₆)-NR₂-, -NR₂-, -(C=O)-, -NR₂-(C=O)-NR₃₈-, -NR₂-(alkyle en C₁₋₆)-, -NR₂-SO₂-, -NR₂-(C=O)- (alkyle en C₁₋₆)-, -(C=O)-NR₂-(alkyle en C₁₋₆)-, -O-(alkyle en C₁₋₆)-O-(alkyle en C₁₋₆)- et -(alkyle en C₁₋₆) -NR₂- (alkyle en C₁₋₆)-; où chacun desdits - (alkyle en C₁₋₆) est facultativement et indépendamment substitué par 1 à 3 substituants choisis parmi -halogéno, -OH, -(alkyle en C₁₋₆), -O- (alkyle en C₁₋₆), -S- (alkyle en C₁₋₆), -phényle et -NR₂₅R₂₆ ;
Y est choisi parmi une liaison directe, -CHR₆-, -0-, -S-, et -NR₅- ;
Ar₂, Ar₃, et Ar₄ sont chacun indépendamment un hétérocycle aromatique de 5 ou 6 chaînons comprenant facultativement 1 ou 2 hétéroatomes choisis parmi 0, N et S ; où chacun desdits Ar₂, Ar₃, et Ar₄ est facultativement et indépendamment substitué par 1 à 3 substituants choisis parmi -NR₁₉R₂₀, - (alkyle en C₁₋₆), -O- (alkyle en C₁₋₆), et -S- (alkyle en C₁₋₆) ;
Het₁, Het₂, Het₃, Het₄, Het₅, Het₆, Het₇ et Het₈ sont chacun indépendamment un hétérocycle monocyclique de 5 ou 6 chaînons ayant de 1 à 3 hétéroatomes choisis parmi 0, N et S, où chacun desdits Het₁, Het₂, Het₃, Het₄, Het₅, Het₆, Het₇ et Het₈ est facultativement substitué par 1 à 3 substituants choisis parmi -(alkyle en C₁₋₆), -O- (alkyle en C₁₋₆), -S- (alkyle en C₁₋₆), et -NR₂₁R₂₂ ; où chacun desdits - (alkyle en C₁₋₆) est facultativement et indépendamment substitué par 1 à 3 halogéno ;
Z₁, Z₂, Z₃, Z₄ et Z₅ sont chacun indépendamment choisis parmi C et N ;
m et n sont chacun indépendamment 0,1, 2, 3 ou 4,
pour utilisation dans la prévention et/ou traitement d'une maladie liée à la kinase LRRK2.

3. Composé de formule I ou stéréoisomère, tautomère, racémique, sel, hydrate, forme de N-oxyde, ou solvate de celui-ci, selon la revendication 1, dans lequel
A₁ est N et A₂ est C
A₁ et A₂ sont choisis parmi C et N ; où, lorsque A₁ est C, alors A₂ est N ; et où, lorsque A₂ est C, alors A₁ est N ;
R₁ et R₇ sont chacun indépendamment choisis parmi -H, -halogéno, -OH, - (alkyle en C₁₋₆), -O- (alkyle en C₁₋₆), -S- (alkyle en C₁₋₆), -NR₉R₁₀, -(C=O)-R₄, -SO₂-R₄, -CN, -NR₉-SO₂-R₄, - cycloalkyle en C₃₋₆), et -Het₆ ; où chacun desdits - (alkyle en C₁₋₆) est facultativement et indépendamment substitué par 1 à 3 substituants choisis parmi -halogéno, -OH, -.NR₁₁R₁₂, -O- (alkyle en C₁₋₆), et -S- (alkyle en C₁₋₆) ;
R₂ est choisi parmi -H, -halogéno, -OH, -(alkyle en C₁₋₆), -O- (alkyle en C₁₋₆), -S- (alkyle en C₁₋₆), -(C=O)-(alkyle en C₁₋₆), -(C=O)-O-(alkyle en C₁₋₆), -(C=O)-NR₂₇R₂₈, -Het₃, - (C=O) -Het₃, -SO₂- (alkyle en C₁₋₆), et -(cycloalkyle en C₃₋₆) ; où chacun desdits - (alkyle en C₁₋₆) est facultativement et indépendamment substitué par 1 à 3 substituants choisis parmi -halogéno, -OH, -O- (alkyle en C₁₋₆), -S-(alkyle en C₁₋₆), -Het₃, -Ar₂, et -NR₁₃R₁₄ ;
R₃ est choisi parmi -H, -halogéno, -OH, -(alkyle en C₁₋₆), -O- (alkyle en C₁₋₆), -S- (alkyle en C₁₋₆), -(C=O)-(alkyle en C₁₋₆), -(C=O)-O-(alkyle en C₁₋₆), -Het₂, - (cycloalkyle en C₃₋₆) -(C=O)-Het₂, - (C=O)-NR₂₉R₃₀, et -SO₂- (alkyle en C₁₋₆) ; où chacun desdits - (alkyle en C₁₋₆) est facultativement et indépendamment substitué par 1 à 3 substituants choisis parmi -halogéno, -OH, -O-(alkyle en C₁₋₆), -S-(alkyle en C₁₋₆) , -NR₁₅R₁₆, -Het₂, et -Ar₃ ;
R₄ est indépendamment choisi parmi -halogéno, -OH, - (alkyle en C₁₋₆), -O-(alkyle en C₁₋₆), -S- (alkyle en C₁₋₆), -NR₁₇R₁₈, et -Het₄ ;
R₅ est choisi parmi -H, - (alkyle en C₁₋₆), -(cycloalkyle en C₃₋₆) ; où chacun desdits alkyle en C₁₋₆ ou - cycloalkyle en C₃₋₆) est facultativement et indépendamment substitué par 1 à 3 substituants choisis parmi -halogéno, -OH, -O(alkyle en C₁₋₆), -S (alkyle en C₁₋₆), -Het₅, -CN et -NR₃₁R₃₂ ;
R₆ est choisi parmi -H, -OH, -halogéno, -(alkyle en C₁₋₆), -O-(alkyle en C₁₋₆), -S-(alkyle en C₁₋₆), -NR₃₃R₃₄, et -Het₈ ;
R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₇ et R₃₈ sont chacun indépendamment choisis parmi -H, =0, - (alkyle en C₁₋₆) , et -Het₁ ; où chacun desdits - (alkyle en C₁₋₆) est facultativement et indépendamment substitué par 1 à 3 substituants choisis parmi -halogéno, -OH, -O-(alkyle en C₁₋₆) , -S-(alkyle en C₁₋₆) , -NR₃₅R₃₆, -Het₇, et -Ar₄ ;
R₃₅ et R₃₆ sont chacun indépendamment choisis parmi -H, =0, et - (alkyle en C₁₋₆) ; où chacun desdits - (alkyle en C₁₋₆) est facultativement et indépendamment substitué par 1 à 3 substituants choisis parmi -halogéno, -OH, -O-(alkyle en C₁₋₆), et -S-(alkyle en C₁₋₆) ;
X₁ est choisi parmi - (alkyle en C₁₋₆)-, -O-(alkyle en C₁₋₆)-, -S-(alkyle en C₁₋₆)-, -(C=O)-, -NR₃-(C=O)-, - (alkyle en C₁₋₆) -NR₃-, -NR₃-, - (C=O) -, -NR₃- (C=O) -NR₃₇-, -NR₃-(alkyle en C₁₋₆)-, -NR₃-SO₂-, -NR₃-(C=O) - (alkyle en C₁₋₆)-, -(C=O) -NR₃- (alkyle en C₁₋₆)-, -O-(alkyle en C₁₋₆)-0- (alkyle en C₁₋₆)- et - (alkyle en C₁₋₆)-NR₃- (alkyle en C₁₋₆)-; où chacun desdits - (alkyle en C₁₋₆) est facultativement et indépendamment substitué par 1 à 3 substituants choisis parmi -halogéno, -OH, -(alkyle en C₁₋₆) , -O-(alkyle en C₁₋₆) , -S- (alkyle en C₁₋₆), -phényle, et -NR₂₃R₂₄
X₂ est choisi parmi - (alkyle en C₁₋₆)-, -O-(alkyle en C₁₋₆)-, -S-(alkyle en C₁₋₆)-, -(C=O)-, -NR₂-(C=O)-, - (alkyle en C₁₋₆)-NR₂-, -NR₂-, -(C=O)-, -NR₂-(C=O)-NR₃₈-, -NR₂-(alkyle en C₁₋₆)-, -NR₂-SO₂-, -NR₂-(C=O)- (alkyle en C₁₋₆)-, -(C=O)-NR₂-(alkyle en C₁₋₆)-, -O-(alkyle en C₁₋₆)-O-C (alkyle en C₁₋₆)- et - (alkyle en C₁₋₆)-NR₂- (alkyle en C₁₋₆)-; où chacun desdits - (alkyle en C₁₋₆) est facultativement et indépendamment substitué par 1 à 3 substituants choisis parmi -halogéno, -OH, -(alkyle en C₁₋₆), -O-(alkyle en C₁₋₆), -S- (alkyle en C₁₋₆), -phényle et-NR₂₅R₂₆ ;
Y est choisi parmi une liaison directe, -CHR₆-, -0-, -S-, et -NR₅- ;
Ar₂, Ar₃, et Ar₄ sont chacun indépendamment un hétérocycle aromatique de 5 ou 6 chaînons comprenant facultativement 1 ou 2 hétéroatomes choisis parmi 0, N et S ; où chacun desdits Ar₂, Ar₃, et Ar₄ est facultativement et indépendamment substitué par 1 à 3 substituants choisis parmi -NR₁₉R₂₀, - (alkyle en C₁₋₆), -O-(alkyle en C₁₋₆), et -S- (alkyle en C₁₋₆) ;
Het₁, Het₂, Het₃, Het₄, Het₅, Het₆, Het₇ et Het₈ sont chacun indépendamment un hétérocycle monocyclique de 5 ou 6 chaînons ayant de 1 à 3 hétéroatomes choisis parmi O, N et S, où chacun desdits Het₁, Het₂, Het₃, Het₄, Het₅, Het₆, Het₇ et Het₈ est facultativement substitué par 1 à 3 substituants choisis parmi -(alkyle en C₁₋₆), -O-(alkyle en C₁₋₆), -S- (alkyle en C₁₋₆), et -NR₂₁R₂₂ ; où chacun desdits - (alkyle en C₁₋₆) est facultativement et indépendamment substitué par 1 à 3 halogéno ;
Z₁, Z₂, Z₃, Z₄ et Z₅ sont chacun indépendamment choisis parmi C et N ;
m et n sont chacun indépendamment 0, 1, 2, 3 ou 4,
pour utilisation dans la prévention et/ou le traitement d'une maladie liée à la kinase LRRK2.

4. Composé de formule I ou stéréoisomère, tautomère, racémique, sel, hydrate, forme de N-oxyde, ou solvate de celui-ci, selon la revendication 1, dans lequel
A₁ et A₂ sont choisis parmi C et N ; où, lorsque A₁ est C, alors A₂ est N ; et où, lorsque A₂ est C, alors A₁ est N ;
R₁ et R₇ sont chacun indépendamment choisis parmi -H, -halogéno et - (alkyle en C₁₋₆) ;
R₂ est choisi parmi -H et - (alkyle en C₁₋₆) ;
R₃ est -H ;
R₅ est choisi parmi -H et - (alkyle en C₁₋₆) ; où ledit -(alkyle en C₁₋₆) peut être facultativement substitué par -CN
X₁ est choisi parmi -O-(alkyle en C₁₋₆)- et - (alkyle en C₁₋₆)-NR₃- ; où chacun desdits - (alkyle en C₁₋₆) est facultativement et indépendamment substitué par 1 à 3 substituants choisis parmi - (alkyle en C₁₋₆) ;
X₂ est choisi parmi -O-(alkyle en C₁₋₆)- et - (alkyle en C₁₋₆)-NR₂ ;
Y est choisi parmi -0- et -NR₅- ;
Z₁, Z₂, Z₃, Z₄ et Z₅ sont chacun indépendamment choisis parmi C et N ;
m et n sont chacun indépendamment 0, 1, 2, 3 ou 4,
pour utilisation dans la prévention et/ou le traitement d'une maladie liée à la kinase LRRK2.

5. Composé de formule I ou stéréoisomère, tautomère, racémique, sel, hydrate, forme de N-oxyde, ou solvate de celui-ci, selon la revendication 1, dans lequel
A₁ est N et A₂ est C
R₁ et R₇ sont chacun indépendamment choisis parmi -H, -halogéno et - (alkyle en C₁₋₆) ;
R₂ est choisi parmi -H et - (alkyle en C₁₋₆) ;
R₃ est -H ;
R₅ est choisi parmi -H et - (alkyle en C₁₋₆) ; où ledit -(alkyle en C₁₋₆) peut être facultativement substitué par -CN
X₁ est choisi parmi -O-(alkyle en C₁₋₆)- et - (alkyle en C₁₋₆)-NR₃- ; où chacun desdits - (alkyle en C₁₋₆) est facultativement et indépendamment substitué par 1 à 3 substituants choisis parmi - (alkyle en C₁₋₆) ;
X₂ est choisi parmi -O-(alkyle en C₁₋₆)- et - (alkyle en C₁₋₆)-NR₂ ;
Y est choisi parmi -0- et -NR₅- ;
Z₁, Z₂, Z₃, Z₄ et Z₅ sont chacun indépendamment choisis parmi C et N ;
m et n sont chacun indépendamment 0, 1, 2, 3 ou 4,
pour utilisation dans la prévention et/ou le traitement d'une maladie liée à la kinase LRRK2.

6. Composé de formule I ou stéréoisomère, tautomère, racémique, sel, hydrate, forme de N-oxyde, ou solvate de celui-ci, selon la revendication 1, dans lequel
A₁ est C et A₂ est N
R₁ et R₇ sont chacun indépendamment choisis parmi -H, -halogéno et - (alkyle en C₁₋₆) ;
R₂ est choisi parmi -H et - (alkyle en C₁₋₆) ;
R₃ est -H ;
R₅ est choisi parmi -H et - (alkyle en C₁₋₆) ; où ledit -(alkyle en C₁₋₆) peut être facultativement substitué par - CN
X₁ est choisi parmi -O-(alkyle en C₁₋₆)- et - (alkyle en C₁₋₆)-NR₃- ; où chacun desdits - (alkyle en C₁₋₆) est facultativement et indépendamment substitué par 1 à 3 substituants choisis parmi - (alkyle en C₁₋₆) ;
X₂ est choisi parmi -O-(alkyle en C₁₋₆)- et - (alkyle en C₁₋₆)-NR₂ ;
Y est choisi parmi -O- et -NR₅- ;
Z₁, Z₂, Z₃, Z₄ et Z₅ sont chacun indépendamment choisis parmi C et N ; m et n
sont chacun indépendamment 0, 1,2, 3, ou 4,
pour utilisation dans la prévention et/ou le traitement d'une maladie liée à LRRK2.

7. Composé tel que défini dans l'une quelconque des revendications 1 à 6 pour utilisation dans le diagnostic, la prévention et/ou le traitement d'une maladie liée à la kinase LRRK2 ; ledit composé étant choisi dans la liste comprenant :

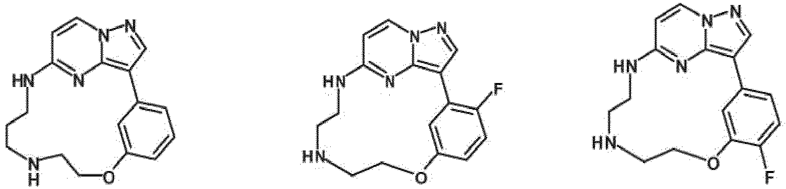

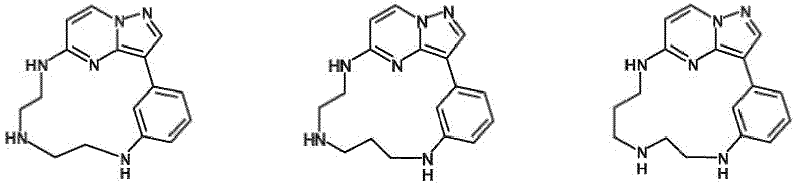

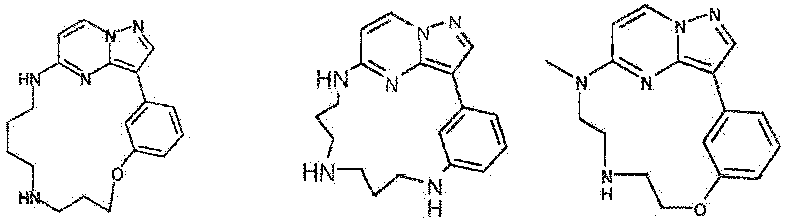

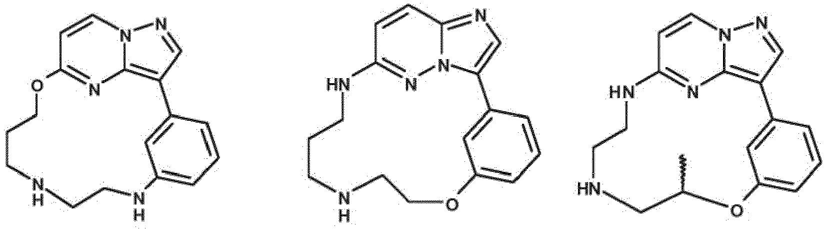

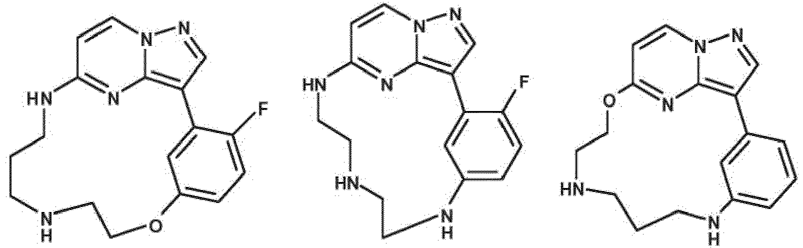

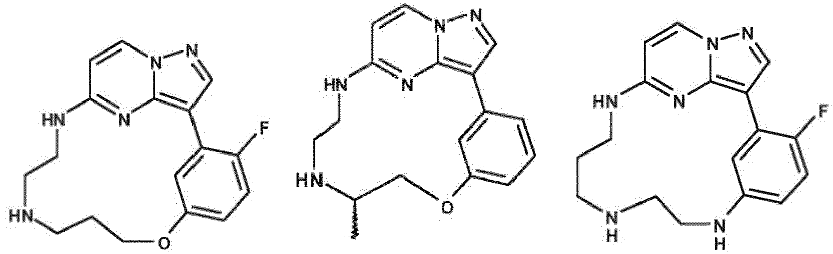

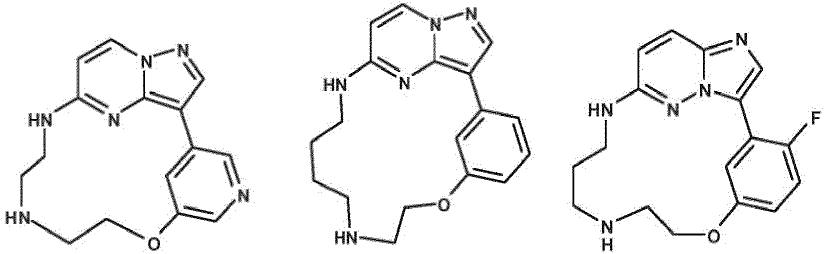

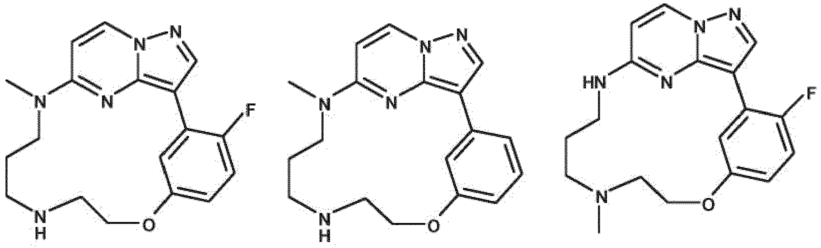

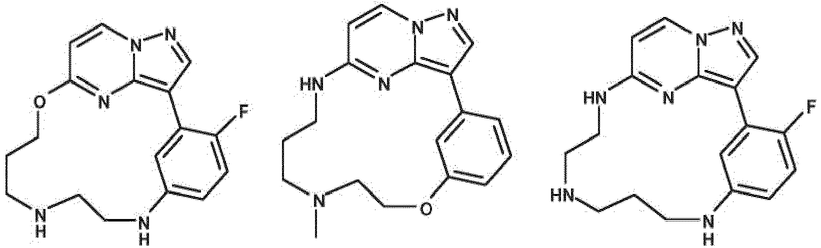

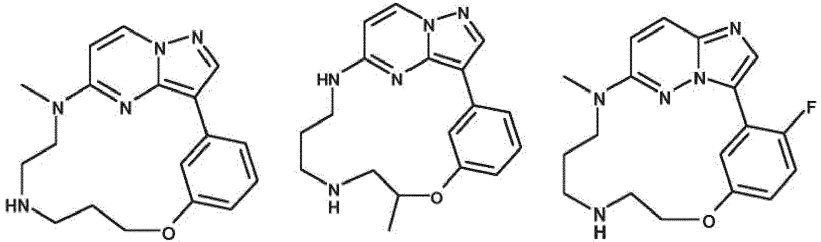

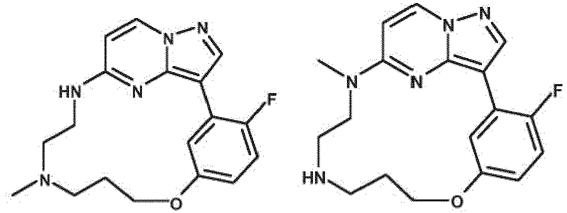

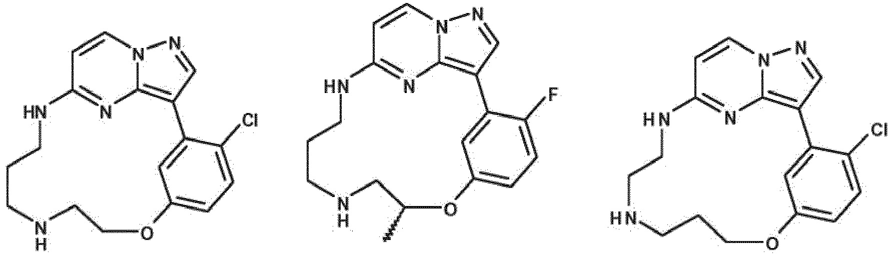

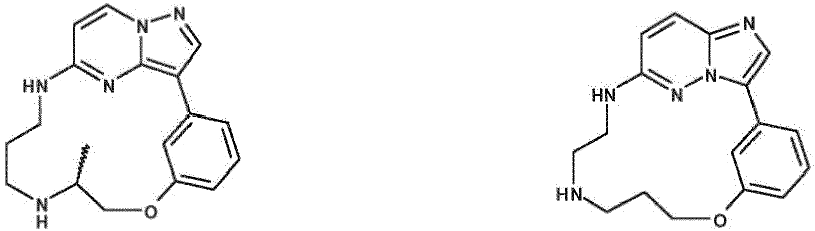

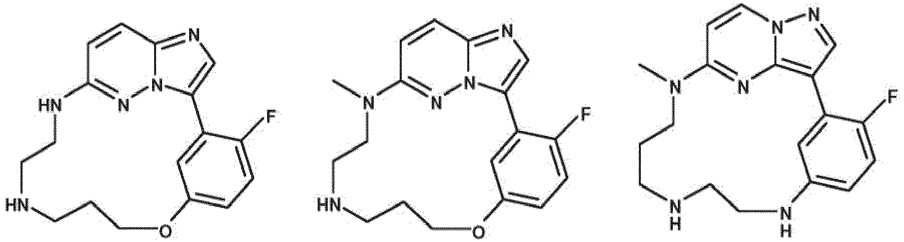

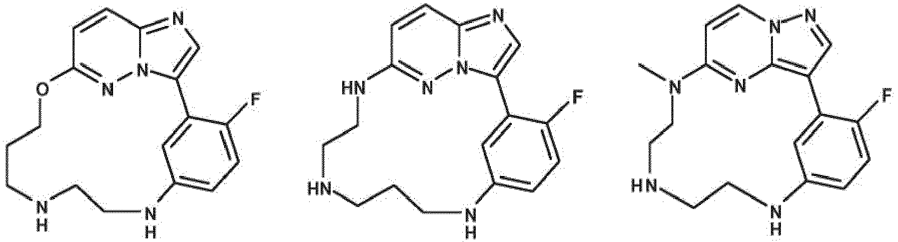

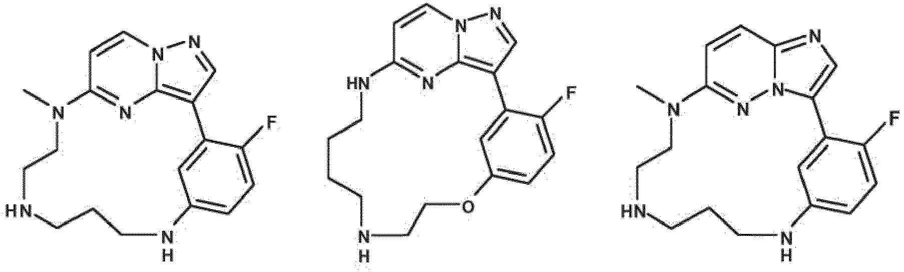

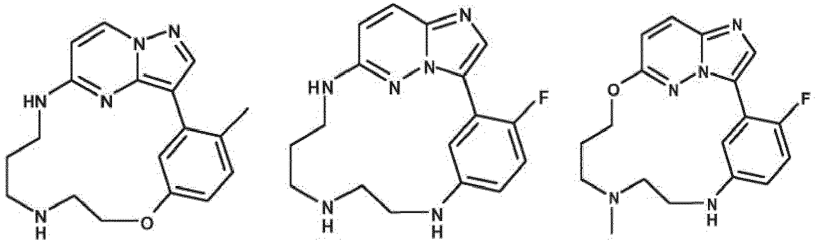

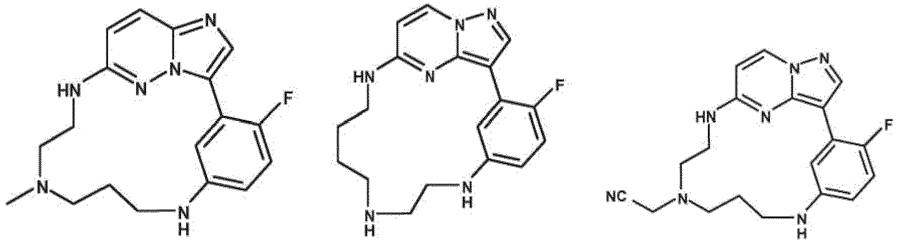

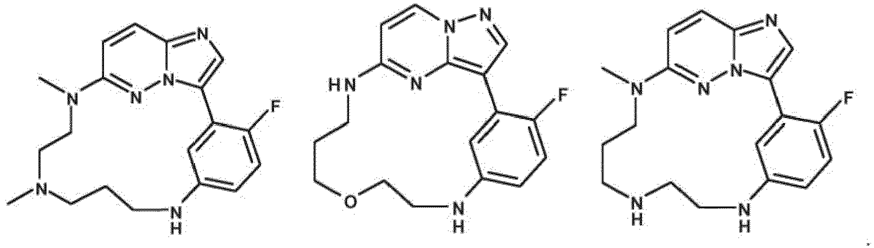

8. Composé tel que défini dans l'une quelconque des revendications 1 à 7 pour utilisation dans le diagnostic, la prévention et/ou le traitement d'une maladie liée à la kinase LRRK2 ; dans lequel le fragment pyrazolopyrimidine ou imidazopyridazine est lié au fragment aryle ou hétéroaryle à la position Z₄ ou Z₅, conformément à la numérotation décrite dans la formule I.

9. Composé tel que défini dans l'une quelconque des revendications 1 à 7 pour utilisation dans le diagnostic, la prévention et/ou le traitement d'une maladie liée à la kinase LRRK2 ; dans lequel R₁ est lié au fragment aryle ou hétéroaryle à la position Z₁, Z₂ ou Z₃, conformément à la numérotation décrite dans la formule I.

10. Composé tel que défini dans l'une quelconque des revendications 1 à 7 pour utilisation dans le diagnostic, la prévention et/ou le traitement d'une maladie liée à LRRK2 ; la maladie liée à la kinase LRRK2 étant un trouble neurologique, en particulier choisie dans la liste comprenant la maladie de Parkinson ou la maladie d'Alzheimer.

11. Composé selon la revendication 1 de formule (IIc) ou stéréoisomère, tautomère, racémique, sel, hydrate, forme de N-oxyde, ou solvate de celui-ci, 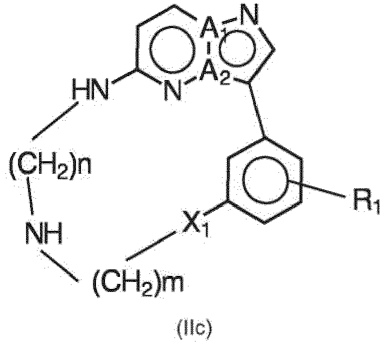 dans lequel
A₁ et A₂ sont choisis parmi C et N ; où, lorsque A₁ est C, alors A₂ est N ; et où, lorsque A₂ est C, alors A₁ est N ;
R₁ est choisi dans la liste comprenant -H, -F, -CH₃, et -CN X₁ est choisi dans la liste comprenant -NH-et -O-
m et n sont chacun indépendamment 1, 2, 3 ou 4
pour utilisation dans la prévention et/ou traitement d'une maladie liée à la kinase LRRK2.

12. Composé tel que défini dans l'une quelconque des revendications 1 à 11 pour utilisation dans le diagnostic, la prévention et/ou le traitement d'une maladie liée à la kinase LRRK2 ; ledit composé étant choisi dans la liste comprenant

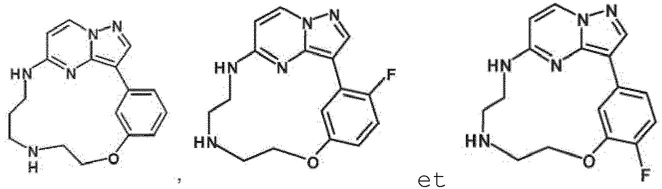

13. Composition pharmaceutique pour utilisation dans le diagnostic, la prévention et/ou le traitement d'une maladie liée à la kinase LRRK2 comprenant un composé tel que défini dans l'une quelconque des revendications 1 à 12.

14. Composé ou stéréoisomère, tautomère, racémique, sel, hydrate, forme de N-oxyde, ou solvate de celui-ci, selon la formule générale (IIIc) 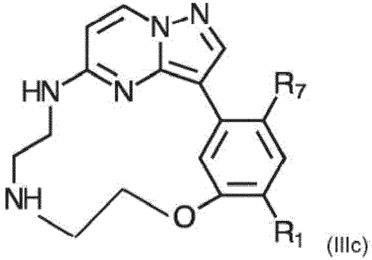 dans lequel
R₁ est -H et R₇ est -F ; ou
R₇ est -H et R₁ est -F.

15. Composé selon la revendication 14, ledit composé étant :

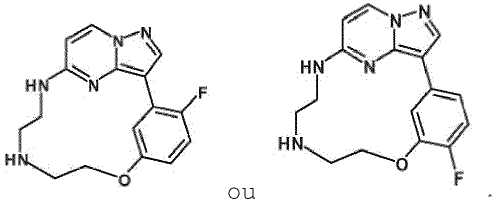

16. Composé choisi dans la liste comprenant :
